# EUROPEAN PATENT SPECIFICATION

(11) **EP 2 675 287 B1**
(45) Date of publication and mention of the grant of the patent: **03.08.2016**
(21) Application number: 12728303.4
(22) Date of filing: 19.01.2012
(51) Int. Cl.: A23K 10/18, A23K 20/189, A23K 50/30, A23K 50/75, A23K 50/60, A61K 38/46, A61K 45/06, A61K 35/741, A61K 31/745, C12N 1/20, C12N 9/16, C12R 1/125

(54) **FEED ADDITIVE COMPOSITION**
FUTTERZUSATZZUSAMMENSETZUNG
COMPOSITION D'ADDITIF ALIMENTAIRE

(30) Priority: 18.02.2011 GB 201102865; 18.02.2011 GB 201102857
(43) Date of publication of application: 25.12.2013
(73) Proprietor: DuPont Nutrition Biosciences ApS, 1411 Copenhagen K (DK)
(72) Inventor: MILLÁN, Luis Fernando Romero, Swindon, Wiltshire SN3 1PG (GB); BERNARD, Luke, Marlborough, Wiltshire SN8 1HN (GB); PLUMSTEAD, Peter, Marlborough, Wiltshire SN8 1AA (GB)
(74) Representative: D Young & Co LLP
(86) International application number: PCT/GB2012/050122
(87) International publication number: WO 2012/110776

(56) References cited:
- WO-A1-2004/085638
- WO-A1-2006/037328
- WO-A1-2007/112739
- WO-A2-2006/038128
- US-A1- 2007 202 088
- DATABASE WPI Week 200853 Thomson Scientific, London, GB; AN 2008-J20352 XP002675948, -& CN 101 181 016 A (JINAN HEMIHUA FEED CO LTD) 21 May 2008 (2008-05-21)
- RAVINDRAN V ET AL: "Response of broiler chickens to microbial phytase supplementation as influenced by dietary phytic acid and non-phytate phosphorous levels. II. Effects on apparent metabolisable energy, nutrient digestibility and nutrient retention", BRITISH POULTRY SCIENCE, vol. 41, 1 January 2000 (2000-01-01), pages 193-200, XP008151769, ISSN: 0007-1668

## Description

### FIELD OF INVENTION

The present invention relates to methods for improving feed compositions using a direct fed microbial in combination with a phytase derivable (preferably derived) from *Citrobacter* spp., particularly to a phytase derivable (preferably derived) from *Citrobacter braakii,* and to a feed additive composition comprising a direct fed microbial in combination with a phytase derived from *Citrobacter* spp., particularly to a phytase derivable (preferably derived) from *Citrobacter braakii.* The present invention further relates to uses and kits.

### BACKGROUND OF THE INVENTION

Supplemental enzymes are used as additives to animal feed, particularly poultry and swine feeds, as a means to improve nutrient utilization and production performance characteristics.

Enzyme blends are available to improve the nutritional value of diets containing cereal grain, soybean meal, animal protein meals, or high fibre food by-products. The concept of direct fed microbials (DFMs) involves the feeding of beneficial microbes to animals, such as broiler chickens when they are under periods of stress (disease, ration changes, environmental or production challenges). Probiotics is another term for this category of feed additives.

Probiotics or DFMs have been shown to improve animal performance in controlled studies. DFMs including direct fed bacteria and/or yeast-based products.

Although combinations of DFMs with some enzymes have been contemplated, the interaction between DFMs and exogenous enzymes in animal feed has never been fully understood.

Database WPI Week 200853 Thomson Scientific, London, GB, AN 2008-J20352 XP002675948 & CN101181016A discloses a layer chicken feed for improving laying performance which comprises *Bacillus subtilis* and phytase.

Ravindran V et al. (British Poultry Science (2000) 41: 193-200) discloses a study of the response of broiler chickens to microbial phytase supplementation and found that it increased apparent metabolisable energy, ileal digestibilities of phosphorus, nitrogen and amino acids and the retention of dry matter, phosphorus and nitrogen.

US 2007/202088 discloses an isolated *Bacillus* strain LSSAO1 which can be administered as a direct-fed microbial. The strain may be fed to a bird in order to increase low G+C, gram positive bacteria in the gastrointestinal flora of the bird, or to inhibit a pathogen such as *E. coli, Salmonella* or *Clostridium.* These benefits may enhance feed conversion in poultry.

The present invention relates to novel specific combinations which surprisingly significantly improve production performance characteristics in animals.

### SUMMARY OF INVENTION

A seminal finding of the present invention is that a DFM in combination with a phytase derivable (preferably derived) from *Citrobacter* spp. (particularly from *Citrobacter braakil*) has significant beneficial effects on the performance of an animal.

In particular, a seminal finding of the present invention is that a DFM in combination with a phytase derivable (preferably derived) from *Citrobacter* spp. (particularly from *Citrobacter braakii*) has significant beneficial effects on the performance of an animal, including improving one or more of the following: feed conversion ratio (FCR), nitrogen retention, survival, carcass yield, growth rate, weight gain, feed efficiency and animals resistance to necrotic enteritis.

Another surprising effect of the present invention is that it can reduce nutrient excretion in manure (e.g. reduce nitrogen and phosphorus) content of a subject's manure.

In one aspect, the present invention provides a feed additive composition comprising (or consisting essentially of or consisting of) a direct fed microbial (DFM) in combination with a phytase derivable (preferably derived) from *Citrobacter* spp. (particularly from *Citrobacter braakii*)*,* wherein the phytase comprises a polypeptide comprising an amino acid sequence which has at least 99.1% identity with amino acids 23-433 of SEQ ID No 1 or 2, and wherein the direct fed microbial comprises a bacterium from one or more of the following species: *Bacillus subtilis, Lactobacillus farciminus, Lactobacillus rhamnosus* and combinations thereof.

In another aspect, the present invention provides a method for improving nitrogen retention, or for avoiding the negative effects of necrotic enteritis or for improving feed conversion ratio (FCR) or for improving weight gain in a subject or for improving feed efficiency in a subject or for reducing nutrient excretion in manure which method comprising administering to a subject a direct fed microbial (DFM) in combination with a phytase derivable (preferably derived) from *Citrobacter* spp, (particularly from *Citrobacter braakii*), wherein the phytase comprises a polypeptide comprising an amino acid sequence which has at least 99.1% identity with amino acids 23-433 of SEQ ID No 1 or 2, wherein the direct fed microbial comprises a bacterium from one or more of the following species: *Bacillus subtilis, Lactobacillus farciminus, Lactobacillus rhamnosus* and combinations thereof, and wherein the subject is poultry.

A yet further aspect of the present invention is use of a direct fed microbial (DFM) in combination with a phytase derivable (preferably derived) from *Citrobacter* spp. (particularly from *Citrobacter braakii*) for improving nitrogen retention or for avoiding the negative effects of necrotic enteritis or for improving feed conversion ratio (FCR) or for improving weight gain in a subject or for improving feed efficiency in a subject or for reducing nutrient excretion in manure, wherein the phytase comprises a polypeptide comprising an amino acid sequence which has at least 99.1% identity with amino acids 23-433 of SEQ ID No 1 or 2, wherein the direct fed microbial comprises a bacterium from one or more of the following species: *Bacillus subtilis, Lactobacillus farciminus, Lactobacillus rhamnosus* and combinations thereof, and wherein the subject is poultry.

In a further aspect of the present invention there is provided a kit comprising a direct fed microbial (DFM), a phytase derivable (preferably derived) from *Citrobacter* spp. (particularly from *Citrobacter braakii*)*,* optionally at least one vitamin, optionally at least one mineral, and instructions for administration, wherein the phytase comprises a polypeptide comprising an amino acid sequence which has at least 99.1% identity with amino acids 23-433 of SEQ ID No 1 or 2, wherein the direct fed microbial comprises a bacterium from one or more of the following species: *Bacillus subtilis, Lactobacillus farciminus, Lactobacillus rhamnosus* and combinations thereof.

In another aspect the present invention provides a method of preparing a feed additive composition, comprising admixing a direct fed microbial (DFM) with a phytase derivable (preferably derived) from *Citrobacter* spp. (particularly from *Citrobacter braakii*) and (optionally) packaging, wherein the phytase comprises a polypeptide comprising an amino acid sequence which has at least 99.1% identity with amino acids 23-433 of SEQ ID No 1 or 2, wherein the direct fed microbial comprises a bacterium from one or more of the following species: *Bacillus subtilis, Lactobacillus farciminus, Lactobacillus rhamnosus* and combinations thereof.

In a yet further aspect the present invention provides a feed or feedstuff comprising a feed additive composition according to the present invention.

The invention also provides a premix comprising a feed additive composition comprising (or consisting essentially of or consisting of) a direct fed microbial (DFM) in combination with a phytase derivable (preferably derived) from *Citrobacter* spp. (particularly from *Citrobacter braakii*)*,* and at least one mineral and/or at least one vitamin, wherein the phytase comprises a polypeptide comprising an amino acid sequence which has at least 99.1% identity with amino acids 23-433 of SEQ ID No 1 or 2 and wherein the direct fed microbial comprises a bacterium from one or more of the following species: *Bacillus subtilis, Lactobacillus farciminus, Lactobacillus rhamnosus* and combinations thereof.

In another aspect, the present invention provides a method of preparing a feedstuff comprising admixing a feed component with a feed additive composition according to the present invention.

In a further aspect, the present invention relates to a feed additive composition according to the present invention for preventing and/or treating coccidiosis and/or necrotic enteritis in poultry.

### BRIEF DESCRIPTION OF THE DRAWINGS

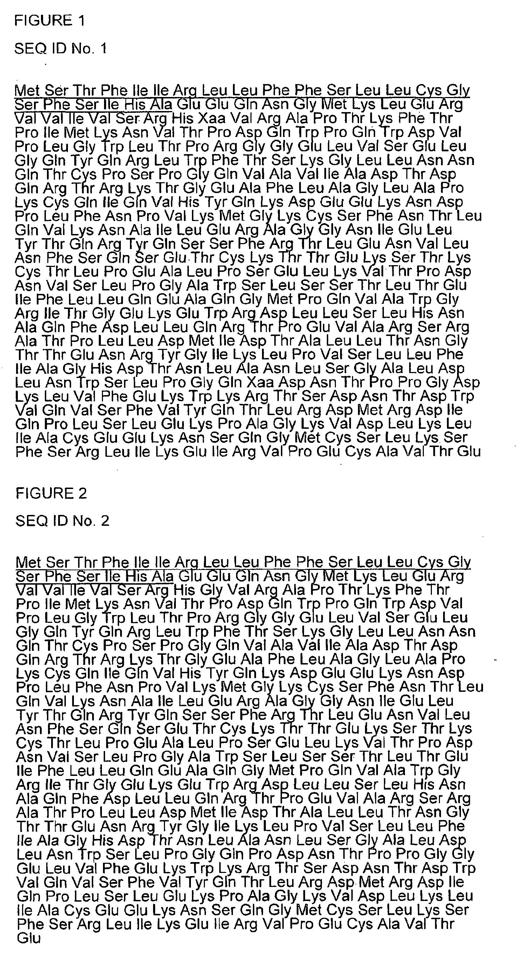

**Figure 1** shows a sequence (SEQ ID No. 1) for a polypeptide having phytase (6-phytase) activity from *Citrobacter braakii* ATCC 51113 - the first Xaa in the sequence stands for Gly and the second Xaa in the sequence stands for Pro. The first 22 amino acids (underlined) are a signal peptide which is cleaved in the mature protein. The mature protein therefore starts at amino acid 23 of this amino acid sequence. Therefore the mature protein is from 23-433. This enzyme is sold commercially by DSM/Novozymes as Ronozyme HiPhos™.
**Figure 2** shows a sequence (SEQ ID No. 2) for a polypeptide having phytase activity from *Citrobacter braakii -* the first Xaa in the sequence stands for Gly and the second Xaa in the sequence stands for Pro. The first 22 amino acids (underlined) are a signal peptide which is cleaved in the mature protein. The mature protein therefore starts at amino acid 23 of this amino acid sequence. Therefore the mature protein is from 23-433.

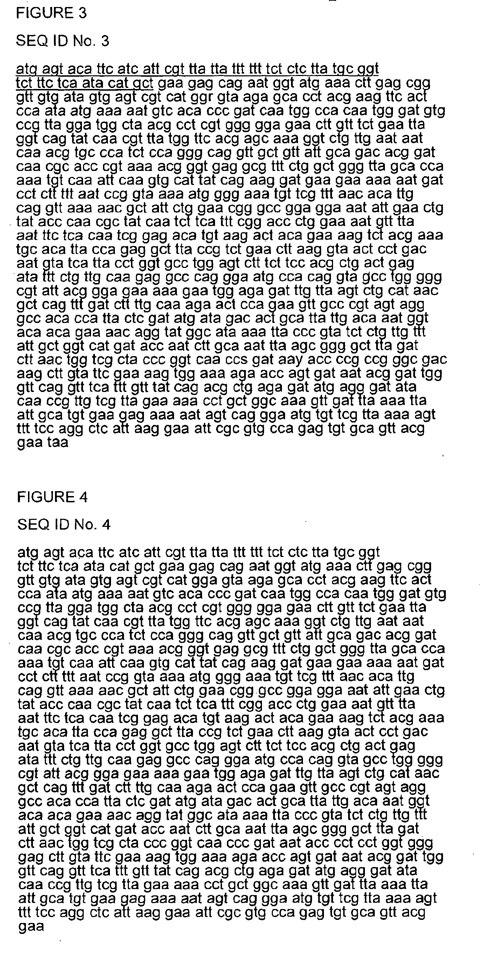

**Figure 3** shows a sequence (SEQ ID No. 3) for a nucleotide sequence which encodes a polypeptide having phytase (6-phytase) activity. Nucleotides 67 to 1299 of SEQ ID No. 3 encode a polypeptide having phytase activity (namely the polypeptide shown in SEQ ID No. 1) (where present r means g or a; y means t/u or c; and s means g or c).
**Figure 4** shows a sequence (SEQ ID No. 4) for a nucleotide sequence which encodes a polypeptide having phytase activity. Nucleotides 67 to 1299 of SEQ ID No. 4 encode a polypeptide having phytase activity (namely the polypeptide shown in SEQ ID No. 2) (where present r means g or a; y means t/u or c; and s means g or c).

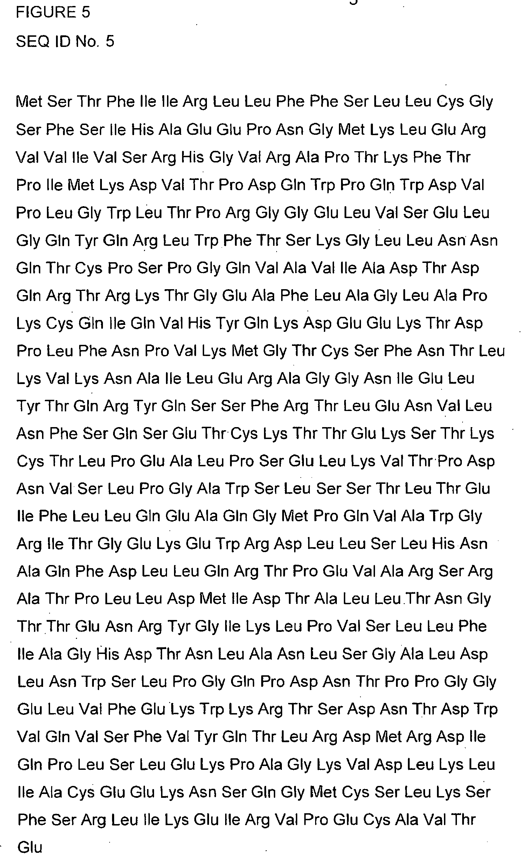

**Figure 5** shows an amino acid sequence (SEQ ID No. 5) for a polypeptide having phytase (6-phytase) activity from *Citrobacter freundii.*

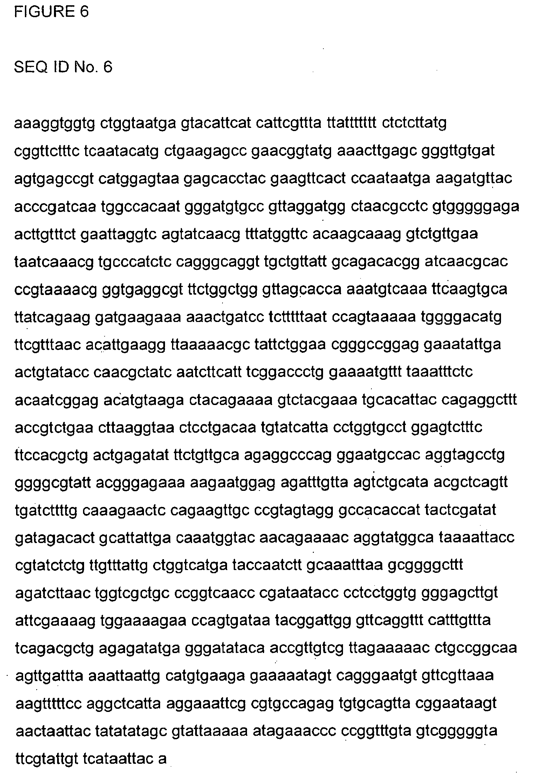

**Figure 6** shows a nucleotide sequence (SEQ ID No. 6) which encodes a polypeptide having phytase activity (namely the polypeptide shown in SEQ ID No. 5).

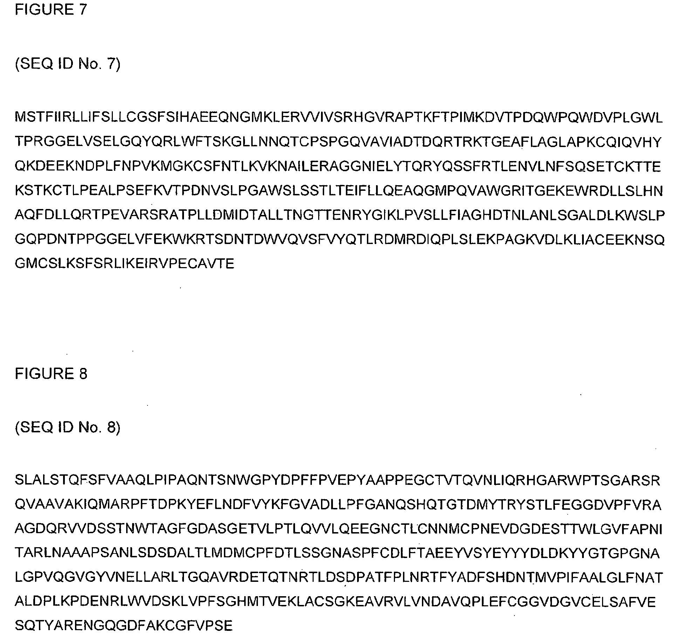

**Figure 7** shows an amino acid sequence for a polypeptide having phytase (6-phytase) activity from *Citrobacter braakii* YH-15 (SEQ I D No. 7).
**Figure 8** shows the amino acid sequence (SEQ ID No. 8) for a polypeptide having phytase (6-phytase) from *Peniphora lycii expressed in Aspergillus oryzae - and* as sold by DSM & Novozymes as Ronozyme P™.

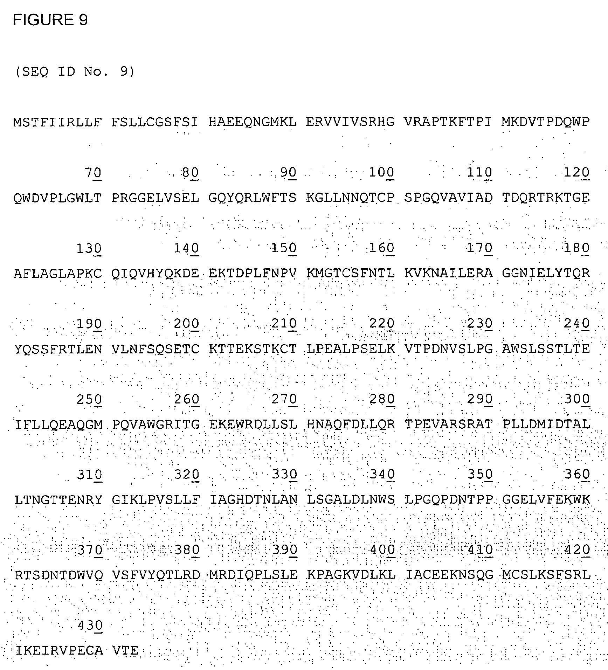

**Figure 9** shows a sequence (SEQ ID No. 9) for a polypeptide having phytase activity from *Citrobacter freundii* (UniProtKB/TrEMBLaccession no. Q676V7).

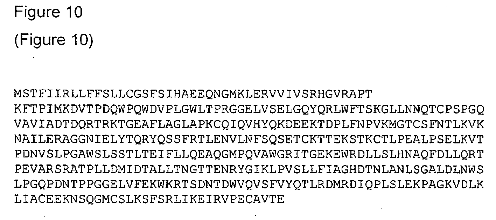

**Figure 10** shows a sequence (SEQ ID No. 10) for a polypeptide having phytase activity from *Citrobacter freundii* (EBI Accession No. EM-PRO:AY390262).

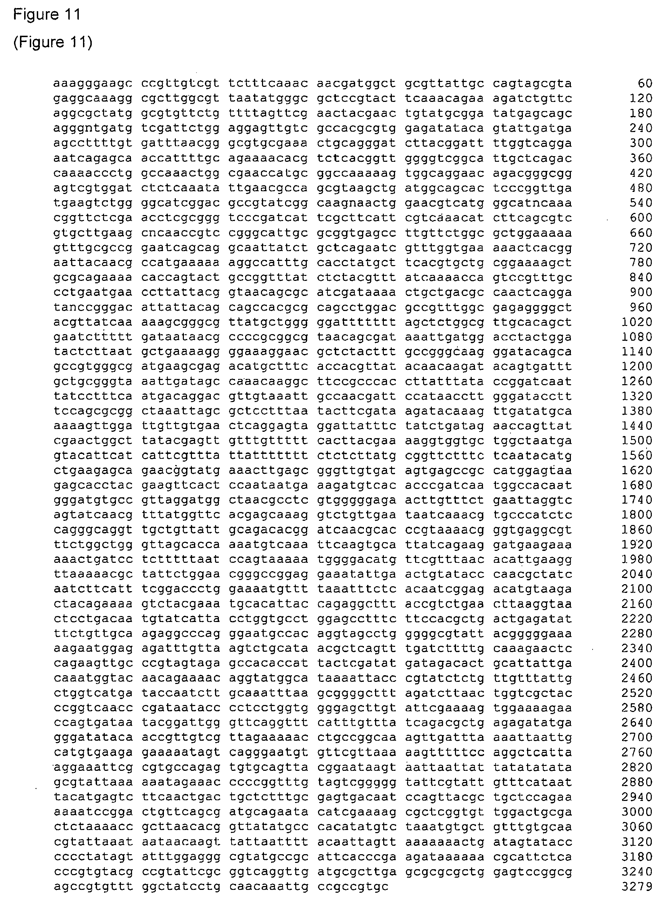

**Figure 11** shows a nucleotide sequence (SEQ ID No. 11) which encodes a polypeptide having phytase activity (namely the polypeptide shown in SEQ ID No. 10).

### DETAILED DESCRIPTION OF THE INVENTION

Preferably the enzyme(s) used in the present invention is/are exogenous to the DFM. In other words the enzyme(s) is/are preferably added to or admixed with the DFM.

Unless defined otherwise, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 20 ED., John Wiley and Sons, New York (1994), and Hale & Marham, THE HARPER COLLINS DICTIONARY OF BIOLOGY, Harper Perennial, NY (1991) provide one of skill with a general dictionary of many of the terms used in this disclosure.

Numeric ranges are inclusive of the numbers defining the range. Unless otherwise indicated, any nucleic acid sequences are written left to right in 5' to 3' orientation; amino acid sequences are written left to right in amino to carboxy orientation, respectively.

The headings provided herein are not limitations of the various aspects or embodiments of this disclosure which can be had by reference to the specification as a whole. Accordingly, the terms defined immediately below are more fully defined by reference to the specification as a whole.

Amino acids are referred to herein using the name of the amino acid, the three letter abbreviation or the single letter abbreviation.

The term "protein", as used herein, includes proteins, polypeptides, and peptides.

As used herein, the term "amino acid sequence" is synonymous with the term "polypeptide" and/or the term "protein". In some instances, the term "amino acid sequence" is synonymous with the term "peptide". In some instances, the term "amino acid sequence" is synonymous with the term "enzyme".

The terms "protein" and "polypeptide" are used interchangeably herein. In the present disclosure and claims, the conventional one-letter and three-letter codes for amino acid residues may be used. The 3-letter code for amino acids as defined in conformity with the IUPACIUB Joint Commission on Biochemical Nomenclature (JCBN). It is also understood that a polypeptide may be coded for by more than one nucleotide sequence due to the degeneracy of the genetic code.

Other definitions of terms may appear throughout the specification. It is also to be understood that the terminology used herein is for the purpose of describing particular embodiments only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise. Thus, for example, reference to "an enzyme" includes a plurality of such candidate agents and reference to "the feed" includes reference to one or more feeds and equivalents thereof known to those skilled in the art, and so forth.

The publications discussed herein are provided solely for their disclosure prior to the filing date of the present application. Nothing herein is to be construed as an admission that such publications constitute prior art to the claims appended hereto.

The enzymes for use in the present invention can be produced either by solid or submerged culture, including batch, fed-batch and continuous-flow processes. Culturing is accomplished in a growth medium comprising an aqueous mineral salts medium, organic growth factors, the carbon and energy source material, molecular oxygen, and, of course, a starting inoculum of one or more particular microorganism species to be employed.

### Direct Fed Microbial (DFM)

The term "microbial" herein is used interchangeably with "microorganism".

The term "DFM" as used here in means direct fed microbial.

Preferably the DFM comprises a viable microorganism. Preferably the DFM comprises a viable bacterium.

The term "viable microorganism" means a microorganism which is metabolically active or able to differentiate.

In one embodiment the DFM may be a spore forming bacterium and hence the term DFM may be comprised of or contain spores, e.g. bacterial spores. Therefore in one embodiment the term "viable microorganism" as used herein may include microbial spores, such as endospores or conidia.

In another embodiment the DFM in the feed additive composition according to the present invention is not comprised of or does not contain microbial spores, e.g. endospores or conidia.

The microorganism may be a naturally occurring microorganism or it may be a transformed microorganism. The microorganism may also be a combination of suitable microorganisms.

Preferably the DFM according to the present invention is a probiotic microorganism.

Preferably the DFM is a direct fed bacterium.

Preferably the DFM is a combination comprising two or more bacteria, e.g. three or more or four or more.

Preferably the bacterium or bacteria is or are isolated.

In one embodiment the DFM may be selected from the *Bacillus* spp *Bacillus subtilis.*

In one embodiment the DFM may be a combination comprising two or more *Bacillus subtilis* strains.

In one embodiment the DFM may be a combination of two or more of the *Bacillus subtilis* strains 3A-P4 (PTA-6506); 15A-P4 (PTA-6507); 22C-P1 (PTA-6508); 2084 (NRRL B-500130); LSSA01 (NRRL-B-50104); BS27 (NRRL B-50105); BS 18 (NRRL B-50633); and BS 278 (NRRL B-50634).

Strains 3A-P4 (PTA-6506), 15A-P4 (PTA-6507) and 22C-P1 (PTA-6508) are publically available from American Type Culture Collection (ATCC).

Strains 2084 (NRRL B-500130); LSSA01 (NRRL-B-50104); BS27 (NRRL B-50105) are publically available from the Agricultural Research Service Culture Collection (NRRL). Strain *Bacillus subtilis* LSSA01 is sometimes referred to as *B. subtilis* 8.

These strains are taught in US 7, 754, 469 B2.

*Bacillus subtilis* BS 18 and *Bacillus subtilis* BS 278 were deposited by Andy Madisen of W227 N752 Westmound Dr. Waukesha, WI 53186, USA or Danisco USA Inc. of W227 N752 Westmound Dr. Waukesha, WI 53186, USA under the Budapest Treaty at the Agricultural Research Service Culture Collection (NRRL) at 1815 North University Street, Peoria, Illinois 61604, United States of America, under deposit numbers NRRL B-50633 and NRRL B-50634, respectively on 9 January 2012.

Andy Madisen of W227 N752 Westmound Dr. Waukesha, WI 53186, USA and Danisco USA Inc. of W227 N752 Westmound Dr. Waukesha, WI 53186, USA authorise Danisco A/S of Langebrogade 1, PO Box 17, DK-1001, Copenhagen K, Denmark to refer to these deposited biological materials in this patent application and have given unreserved and irrevocable consent to the deposited material being made available to the public.

In some embodiments the DFM may be a combination comprising the *Bacillus subtilis* strains as detailed in the table below:

| *B. subtilis* strain | Bs 2084 | Bs 8 (LSSAO1) | Bs 3A-P4 | Bs 15A-P4 | Bs 278 | Bs 18 | Bs 22C-P1 |
|---|---|---|---|---|---|---|---|
| | X | X | X | X | | | |
| | X | X | X | | | | |
| | X | X | | | X | | |
| DFM Combination comprises | X | X | | X | | | |
| | X | | X | X | | | |
| | | X | X | X | | | |
| | X | X | | | | X | |
| | | | X | X | | | X |
| | X | X | | | X | | |

In one embodiment the DFM may be selected from the *Lactobacillus* spp *Lactobacillus rhamnosus* or *Lactobacillus farciminis* and combinations thereof.

The DFM according to the present invention comprises a bacterium from one or more of the following species: *Bacillus subtilis, Lactobacillus farciminis, Lactobacillus rhamnosus* and combinations thereof.

The direct fed bacterium used in the present invention may be of the same type (genus, species and strain) or may comprise a mixture of genera, species and/or strains.

Suitably the DFM according to the present invention may be one or more of the products or the microorganisms contained in those products as in the Table below:

| **Product Name** | **Company** | **Microorganism(s)** | **Symbiotic ingredients** |
|---|---|---|---|
| Enviva Pro®, (formerly known as Avicorr®) | Danisco A/S | *Bacillus subtilis* strain 2084 Accession No. NRRI B-50013, *Bacillus subtilis* strain LSSAO1 Accession No. NRRL B-50104 and *Bacillus subtilis* strain 15A-P4 ATCC Accession No. PTA-6507 | |
| Calsporin® | Calpis - Japan | *Bacillus subtilis* Strain C3102 | |
| Clostat® | Kemin Industries Inc. | *Bacillus subtilis* Strain PB6 | |
| | | | |
| Gallipro® & GalliproMax® | Chr. Hansen A/S | *Bacillus subtilis* Strain C3102 | |
| | | | |
| | | | |
| | | | |
| Proflora® | Alpharma Inc. | *Bacillus subtilis* strain QST 713 | β-mos β-mannan oligosaccha rides and β-glucans |
| | | | |
| | | | |
| | | | |
| | | | |
| CSI® | Danisco A/S | *Bacillus* strain | |
| | | | |
| | | | |
| | | | |
| | | | |
| | | | |
| | | | |
| Sorbiflore® | Danisco Animal Nutrition | *Lactobacillus rhamnosus and Lactobacillus farciminis* | |
| Animavit® | KRKA | *Bacillus subtilis* | |
| | | | |
| | | | |
| | | | |
| | | | |
| | | | |
| | | | |

In one embodiment suitably the DFM may be Enviva Pro®. Enviva Pro® is commercially available from Danisco A/S and is a combination of *Bacillus* strain 2084 Accession No. NRRI B-50013, *Bacillus* strain LSSA01 Accession No. NRRL B-50104 and *Bacillus* strain 15A-P4 ATCC Accession No. PTA-6507 (as taught in US 7,754,469).

Preferably the DFM to be used in accordance with the present invention is a microorganism which is generally recognised as safe and, which is preferably GRAS approved.

A skilled person will readily be aware of specific species and or strains of microorganisms from within the genera described herein which are used in the food and/or agricultural industries and which are generally considered suitable for animal consumption.

Preferably, the DFM used in accordance with the present invention is one which is suitable for animal consumption.

Advantageously, where the product is a feed or feed additive composition, the viable DFM should remain effective through the normal "sell-by" or "expiration" date of the product during which the feed or feed additive composition is offered for sale by the retailer. The desired lengths of time and normal shelf life will vary from feedstuff to feedstuff and those of ordinary skill in the art will recognise that shelf-life times will vary upon the type of feedstuff, the size of the feedstuff, storage temperatures, processing conditions, packaging material and packaging equipment.

In some embodiments it is important that the DFM is tolerant to heat, i.e. is thermotolerant. This is particularly the case where the feed is pelleted. Therefore in one embodiment the DFM may be a thermotolerant microorganism, such as a thermotolerant bacterium, including for example *Bacillus* spp.

In some embodiments it may be preferable that the DFM is a spore producing bacteria, such as *Bacilli,* e.g. *Bacillus* spp. Bacilli are able to from stable endospores when conditions for growth are unfavorable and are very resistant to heat, pH, moisture and disinfectants.

In one embodiment the DFM according to the present invention may be an inhibitory strain (or an antipathogen strain). In one embodiment the following assay "DFM ASSAY" may used to determine the suitability of a microorganism to be a DFM. For the avoidance of doubt in one embodiment a DFM selected as an inhibitory strain (or an antipathogen DFM) in accordance with the "DFM ASSAY" taught herein is a suitable DFM for use in accordance with the present invention, i.e. in the feed additive composition according to the present invention.

### DFM ASSAY:

Tubes were seeded each with a representative pathogen from a representative cluster.

Supernatant from a potential DFM grown aerobically or anaerobically was added to the seeded tubes and incubated.

After incubation, the optical density (OD) of the control and supernatant treated tubes was measured for each pathogen.

Colonies of (potential DFM) strains that produced a lowered OD compared with the control were classified as an inhibitory strain (or an antipathogen DFM).

The DFM assay as used herein is explained in more detail in US2009/0280090

Preferably the representative pathogen used in assay is one (or more) of the following: Clostridium, such as *Clostridium perfringens* and/or *Clostridium difficile,* and/or *E. coli* and/or *Salmonella* spp and/or *Campylobacter* spp. In one preferred embodiment the assay is conducted with one or more of *Clostridium perfringens* and/or *Clostridium difficile* and/or *E*. *coli,* preferably *Clostridium perfringens* and/or *Clostridium difficile,* more preferably *Clostridium perfringens.*

In one embodiment the DFM of the present invention is preferably an antipathogen.

The term "antipathogen" as used herein means that the DFM counters an effect (e.g. a negative effect) of a pathogen.

In one embodiment to determine if a DFM is an antipathogen in accordance with the present invention the above mentioned DFM assay may be used. A DFM is considered to be an antipathogen or an antipathogen DFM if it is classed as an inhibitory strain in the above mentioned DFM assay, particularly when the pathogen is *Clostridium perfringens.*

In one embodiment the antipathogen DFM may be one or more of the following bacteria:
*Bacillus subtilis* strain 2084 Accession No. NRRL B-50013,
*Bacillus subtilis* strain LSSAO1 Accession No. NRRL B-50104,
*Bacillus subtilis* strain 15A-P4 ATCC Accession No. PTA-6507,
*Bacillus subtilis* strain 3A-P4 ATCC Accession No. PTA-6506, and
*Bacillus subtilis* strain BS27 ATCC Accession No. NRRL B-50105. For the avoidance of doubt these strains are available and are referred to in US 7,754,459 B.

In one embodiment the DFM used in accordance with the present invention is not *Lactobacillus gasseri* BNR 17 Strain Acc No. KCTC 10902BP as taught in WO2008/016214.

Preferably the DFM is not an inactivated microorganism.

In one embodiment the DFM as used herein is a composition comprising one or more DFM microorganisms as described herein. The composition may additionally comprise the enzymes of the present invention. The composition can be fed to an animal as a direct-fed microbial (DFM). One or more carrier(s) or other ingredients can be added to the DFM. The DFM may be presented in various physical forms, for example, as a top dress, as a water soluble concentrate for use as a liquid drench or to be added to a milk replacer, gelatin capsule, or gels. In one embodiment of the top dress form, freeze-dried fermentation product is added to a carrier, such as whey, maltodextrin, sucrose, dextrose, limestone (calcium carbonate), rice hulls, yeast culture, dried starch, and/or sodium silico aluminate. In one embodiment of the water soluble concentrate for a liquid drench or milk replacer supplement, freeze-dried fermentation product is added to a water soluble carrier, such as whey, maltodextrin, sucrose, dextrose, dried starch, sodium silico aluminate, and a liquid is added to form the drench or the supplement is added to milk or a milk replacer. In one embodiment of the gelatin capsule form, freeze-dried fermentation product is added to a carrier, such as whey, maltodextrin, sugar, limestone (calcium carbonate), rice hulls, yeast culture dried starch, and/or sodium silico aluminate. In one embodiment, the bacteria and carrier are enclosed in a degradable gelatin capsule. In one embodiment of the gels form, freeze-dried fermentation product is added to a carrier, such as vegetable oil, sucrose, silicon dioxide, polysorbate 80, propylene glycol, butylated hydroxyanisole, citric acid, ethoxyquin, and/or artificial coloring to form the gel.

The DFM(s) may optionally be admixed with a dry formulation of additives including but not limited to growth substrates, enzymes, sugars, carbohydrates, extracts and growth promoting micro-ingredients. The sugars could include the following: lactose; maltose; dextrose; malto-dextrin; glucose; fructose; mannose; tagatose; sorbose; raffinose; and galactose. The sugars range from 50-95%, either individually or in combination. The extracts could include yeast or dried yeast fermentation solubles ranging from 5-50%. The growth substrates could include: trypticase, ranging from 5-25%; sodium lactate, ranging from 5-30%; and, Tween 80, ranging from 1-5%. The carbohydrates could include mannitol, sorbitol, adonitol and arabitol. The carbohydrates range from 5-50% individually or in combination. The micro-ingredients could include the following: calcium carbonate, ranging from 0.5-5.0%; calcium chloride, ranging from 0.5-5.0%; dipotassium phosphate, ranging from 0.5-5.0%; calcium phosphate, ranging from 0.5-5.0%; manganese proteinate, ranging from 0.25-1.00%; and, manganese, ranging from 0.25-1.0%.

To prepare DFMs described herein, the culture(s) and carrier(s) (where used) can be added to a ribbon or paddle mixer and mixed for about 15 minutes, although the timing can be increased or decreased. The components are blended such that a uniform mixture of the cultures and carriers result. The final product is preferably a dry, flowable powder. The DFM(s) or composition comprising same can then be added to animal feed or a feed premix, added to an animal's water, or administered in other ways known in the art (preferably simultaneously with the enzymes of the present invention). A feed for an animal can be supplemented with one or more DFM(s) described herein or with a composition described herein.

By "a mixture of at least two strains," is meant a mixture of two, three, four, five, six or even more strains. In some embodiments of a mixture of strains, the proportions can vary from 1 % to 99%. Other embodiments of a mixture of strains are from 25% to 75%. Additional embodiments of a mixture of strains are approximately 50% for each strain. When a mixture comprises more than two strains, the strains can be present in substantially equal proportions or in different proportions in the mixture.

The DFM may be dosed appropriately.

Suitably dosages of DFM in the feed may be between about 1x10³ CFU/g feed to about 1x10⁹ CFU/g feed, suitably between about 1x10⁴ CFU/g feed to about 1x10⁸ CFU/g feed, suitably between about 7.5x10⁴ CFU/g feed to about 1x10⁷ CFU/g feed.

In one embodiment the DFM is dosed in the feedstuff at more than about 1x10³ CFU/g feed, suitably more than about 1x10⁴ CFU/g feed, suitably more than about 7.5x10⁴ CFU/g feed.

Suitably dosages of DFM in the feed additive composition may be between about 1x10⁵ CFU/g composition to about 1x10¹³ CFU/g composition, suitably between about 1x10⁶ CFU/g composition to about 1x10¹² CFU/g composition, suitably between about 3.75x10⁷ CFU/g composition to about 1x10¹¹ CFU/g composition.

In one embodiment the DFM is dosed in the feed additive composition at more than about 1x10⁵ CFU/g composition, suitably more than about 1x10⁶ CFU/g composition, suitably more than about 3.75x10⁷ CFU/g composition.

In one embodiment the DFM is dosed in the feed additive composition at more than about 2x10⁵ CFU/g composition, suitably more than about 2x10⁶ CFU/g composition, suitably more than about 3.75x10⁷ CFU/g composition.

As used herein the term "CFU" means colony forming units and is a measure of viable cells in which a colony represents an aggregate of cells derived from a single progenitor cell.

### Phytase

Phytic acid (royo-inositol hexakisphosphate) is an important constituent in cereals, legumes and oilseed crops. The salt form, phytate, is the major storage form of phosphorous in these plants.

Phytases catalyse phosphate monoester hydrolysis of phytic acid which results in the stepwise formation of myo-inositol pentakis-, tetrakis-, tris-, bis- and monophosphates, as well as the liberation of inorganic phosphate.

The term "phytase" means a protein or polypeptide which is capable of catalysing the hydrolysis of esters of phosphoric acid including phytate and releasing inorganic phosphate. Phytases are capable to hydrolyse, in addition to phytate, at least some of the inositol-phosphates of intermediate degrees of phosphorylation.

The phytase for use in the present invention may be classified a 6-phytase (classified as E.C. 3.1.3.26) or a 3-phytase (classified as E.C. 3.1.3.8).

In one embodiment the phytase is preferably a 6-phytase (E.C. 3.1.3.26).

The phytase for use in the present invention is derivable (preferably derived) from a *Citrobacter* bacterium.

The phytase for use in the present invention is a *Citrobacter* phytase, preferably a *Citrobacter braakii* phytase.

In one embodiment the phytase for use in the present invention is derivable, preferably derived, from a *Citrobacter* bacterium selected from the group consisting of: *Citrobacter braakii, e.g. Citrobacter braakii* ATCC 51113; *Citrobacter freundii, e.g. C. freundii* NCIMB 41247; *Citrobacter amalonaticus,* e.g. *Citrobacter amalonaticus* ATCC 25405 or *Citrobacter amalonaticus* ATCC 25407; *Citrobacter gillenii,* e.g. *Citrobacter gillenii* DSM 13694; *Citrobacter intermedius, Citrobacter koseri, Citrobacter murliniae, Citrobacter rodentium, Citrobacter sedlakii, Citrobacter werkmanii, Citrobacter youngae,* or *Citrobacter* species.

In one embodiment the phytase is a *Citrobacterphytase* derived from e.g.
- *Citrobacter braakii* ATCC 51113 as disclosed in WO2006/037328 - the amino acid sequence for the enzyme is shown herein as SEQ ID No. 1, as well as variants thereof e.g. as disclosed in WO2007/112739 and WO2011/117396,
- *Citrobacter braakii* YH-15 as described in WO2004/085638 - the amino acid sequence for the enzyme is shown herein as SEQ ID No. 7.
- *Citrobacter freundii,* preferably *C*. *freundii* NCIMB 41247 and variants thereof e.g. as disclosed in WO2006/038062 and WO2006/038128 or *Citrobacter freundii* phytases taught in UniProtKB/TrEMBLaccession no. Q676V7 (shown herein as SEQ ID No. 9) or EBI Accession No. EM-PRO:AY390262 (shown herein as SEQ ID No. 10).
- *Citrobacter amalonaticus,* preferably *Citrobacter amalonaticus* ATCC 25405 or *Citrobacter amalonaticus* ATCC 25407 as disclosed in WO2006037327,
- *Citrobacter gillenii,* preferably *Citrobacter gillenii* DSM 13694 as disclosed in WO2006037327, or
- *Citrobacter intermedius,*
- *Citrobacter koseri,*
- *Citrobacter murliniae,*
- *Citrobacter rodentium,*
- *Citrobacter sedlakii,*
- *Citrobacter werkmanii,*
- *Citrobacter youngae,*
- *Citrobacter farmeri.*

In a preferred embodiment the phytase for use in the present invention is the phytase derivable or derived from *Citrobacter braakii* ATCC 51113 as disclosed in WO2006/037328 and having the amino acid sequence shown herein as SEQ ID No. 1, as well as variants thereof e.g. as disclosed in WO2007/112739 and WO2011/117396, or is the phytase derivable or derived from *Citrobacter freundii,* preferably *C*. *freundii* NCIMB 41247 and having the amino acid sequence shown herein as SEQ ID No. 5, or variants thereof e.g. as disclosed in WO2006/038062 and WO2006/038128, or is the *Citrobacter freundii* phytase taught in UniProtKB/TrEMBLaccession no. Q676V7 (shown herein as SEQ ID No. 9), or is the *Citrobacter freundii* phytase taught in EBI Accession No. EM-PRO:AY390262 (shown herein as SEQ ID No. 10), or is the phytase derivable or derived from *Citrobacter braakii* YH-15 as disclosed in WO2004/085638 and having the amino acid sequence shown herein as SEQ ID No. 7 or variants thereof.

In a preferred embodiment the phytase for use in the present invention is the phytase (e.g. 6-phytase) derivable (or derived) from *Citrobacter braakii* ATCC 51113 as disclosed in WO2006/037328, or a variant thereof e.g. as disclosed in WO2007/112739 and WO2011/117396.

The phytase for use in the present invention comprises a polypeptide having phytase activity comprising an amino acid sequence which has at least 99.1% identity with amino acids 23-433 of SEQ ID No 1 or 2.

In one embodiment the phytase for use in the present invention may be encoded by a polynucleotide, selected from the group consisting of: (a) a polynucleotide encoding a polypeptide having an amino acid sequence which has at least 98.6% identity with amino acids 23-433 of SEQ ID NO: 1 or SEQ ID No. 2; and (b) a polynucleotide having at least 98.3% identity with nucleotides 67 to 1299 of SEQ ID NO: 3 or SEQ ID NO. 4.

The phytase for use in the present invention may be encoded by polynucleotide operably linked to a nucleotide sequence encoding a signal peptide consisting of (i) nucleotides 1 to 66 of SEQ ID NO: 1 or (ii) nucleotides 1 to 66 of SEQ ID NO: 3.

In some embodiments the phytase for use in the present invention may have an improved thermostability indicated as residual activity determined by dividing a supernatant into two parts, one part is incubated for 30 minutes at 60°C, and the other part for 30 minutes at 5 °C, following which the activity of both is determined on p-nitrophenyl phosphate at 37 °C and pH 5.5, where the residual activity of the phytase is the activity of the sample having been incubated at 60 °C divided by the activity of the same sample having been incubated at 5 °C, where the residual activity of the phytase is at least 105% of the residual activity of the reference phytase shown herein as SEQ ID No. 1, measured in the same conditions.

In some embodiments the phytase as used in the present invention may comprise at least one alteration and no more than 4 alterations as compared to SEQ ID No. 1 or SEQ ID No. 2, wherein at least one of said one to four alterations is selected from the following: 4P, 46E, 107G, 111P, 119K, 162C, 223E, 241 Q, 273L, 276K, 379K, 385D, 91C/46C, 52C/99C, 31C/176C, 31C/177C, 59C/100C, 141C/199C, 162C/247C, 111P/241Q, 31C, 119K, 202N, 286Q and 362K,R.

In some embodiments the phytase as used in the present invention may comprise at least one alteration and no more than 4 alterations as compared to SEQ ID No. 1 or SEQ ID No. 2, wherein at least one of said one to four alterations is selected from the following: 91C/46C, 52C/99C, 31C/176C, 31C/177C, 59C/100C, 141C/199C, 162C/247C, 111P/241Q.

In a preferred embodiment the phytase for use in the present invention is the phytase sold commercially as Ronozyme HiPhos™.

The enzyme Ronozyme HiPhos™ has the amino acid sequence shown as SEQ ID No. 1 herein.

In one embodiment the phytase for use in the present invention is the *Citrobacter braakii* YH-15 phytase having the amino acid sequence shown herein as SEQ ID No. 7.

In one embodiment the phytase may be a phytase from *Citrobacter freundii,* such as the phytase enzyme(s) taught in WO2006/038128, or is the *Citrobacter freundii* phytase taught in UniProtKB/TrEMBLaccession no. Q676V7 (shown herein as SEQ 10, No.9), or is the *Citrobacter freundii* phytase taught in EBI Accession No. EM-PRO:AY390262 (shown herein as SEQ ID No. 10).

In one embodiment the phytase may be a phytase from *Citrobacter freundii,* such as the phytase enzyme(s) taught in WO2006/038128.

In one embodiment the phytase for use in the present invention may comprise the amino acid sequence as shown in SEQ ID NO: 5, SEQ ID No. 9 or SEQ ID No. 10.

In one embodiment the phytase for use in the present invention may comprise an amino acid sequence as shown in SEQ ID No. 5 which is a *Citrobacter freundii* phytase or a sequence having at least 90% identity thereto; wherein said polypeptide comprises
a combination of mutations selected from the group consisting of: R288M; K46E/Q82H/E168D/Q274L; Q82K/T1541/Q279E/N308T; Q82R/D112V/Q274H/T362A; D53N/D57Y/T199I/P229S/R288M; K46E/Q82H/N148D/T154I/T362I; D53N/D57Y/P229S/R288M/K358R; D53N/D57Y/T154I/P229S/R288M; K46E/Q82H/N95D/D112V/K142R/D383V; D53N/D57Y/M152V/P229S/R288M/A393P; D53K/D57Y/M152V/P229S/R288M/A393P; D53N/D57Y/F88Y/M152V/P229S/Q279E/N308T; D53N/D57Y/M152V/E204V/P229S/R288M/A393P; D53N/D57Y/M152V/T154I/P229S/R288M/A393P; D53N/D57Y/Q82H/G103E/M152V/P229S/R288M/A393P; K46E/D53N/D57Y/T1431/M152V/L176V/P229S/R288M/A393P; Q82K/F88Y/N96P/Q97T/T98G/V105I/Q274H/Q279E/A393P; Q82R/F88Y/N95P/N96P/Q97T/Q279E/I384L/P386Q/A393P; H18Q/D53N/D57Y/E75V/M152V/A170T/P229S/R288M/Q385R/A393P; Q82K/F88Y/N96P/T98G/Y136N/M152V/Y177F/T362I/I384F/A393P/D397N; D53N/D57Y/F88Y/N95P/N96P/V105I/D112V/Y136N/N148D/N164D/Q274H/T362I/I384L/A39 3P; D53N/D57Y/Q82K/F88Y/N95P/P102L/V105I/Y136N/N 148D/Y177F/Q274H/Q279E/T362I/A3 93P; D53N/D57Y/Q82K/F88Y/N96P/T98G/V105I/D112V/Y177F/Q274L/G343A/T362I/I384L/A393 P;
E23K/K46E/Q82H;
K46E/Q82H/Q385R;
D53N/D57Y/E75V/M152V/A170T/P229S/R288M/Q385R/A393P ;
numbered according to the numbering in SEQ ID No. 5, and
wherein the isolated polypeptide has increased thermostability compared to a polypeptide having the sequence set out in SEQ ID NO: 5.

In one embodiment the phytase for use in the present invention may comprise the amino acid sequence as shown in SEQ ID NO: 7.

In one embodiment preferably the *Citrobacter* phytase in accordance with the present invention has a pH optima in the range of 3-4.5. In one embodiment preferably the *Citrobacter* phytase according to the present invention is capable of its highest activity in the pH range of about 3-3.5.

Both *Citrobacter braakii* ATCC 51113 as disclosed in WO2006/037328 (incorporated herein by reference) - the amino acid sequence for the enzyme is shown herein as SEQ ID No. 1 and *Citrobacter braakii* YH-15 as described in WO2004/085638 - the amino acid sequence for the enzyme is shown herein as SEQ ID No. 7 have a pH optima in the range of 3-4.5.

*Citrobacter braakii* ATCC 51113 as disclosed in WO2006/037328 (incorporated herein by reference) - the amino acid sequence for the enzyme is shown herein as SEQ ID No. 1 is also capable of its highest activity in the pH range of about 3-3.5.

*Citrobacter braakii* YH-15 as described in WO2004/085638 - the amino acid sequence for the enzyme is shown herein as SEQ ID No. 7 has its highest activity at a pH of about 4.

Suitably more than one phytase may be used in combination, e.g. 2 or 3 phytases.

It is also contemplated in the present invention that more than one *Citrobacter* phytase (e.g. from the same or different species or strains) may be used in combination. Alternatively, the at least one *Citrobacter* phytase as detailed herein may be used in combination with one or more *non-Citrobacter* phytases.

In one embodiment preferably the *Citrobacter* phytase used in the present invention is a 6-phytase.

In one embodiment preferably the *Citrobacter* phytase used in the present invention is not used in combination a further phytase, e.g. a further *Citrobacter* phytase or a further non-*Citrobacter* phytase.

Preferably, the phytase is present in the feedstuff in range of about 200FTU/kg to about 1000FTU/kg feed, more preferably about 300FTU/kg feed to about 750FTU/kg feed, more preferably about 400FTU/kg feed to about 500FTU/kg feed.

In one embodiment the phytase is present in the feedstuff at more than about 200FTU/kg feed, suitably more than about 300FTU/kg feed, suitably more than about 400FTU/kg feed.

In one embodiment the phytase is present in the feedstuff at less than about 1000FTU/kg feed, suitably less than about 750FTU/kg feed.

Preferably, the phytase is present in the feed additive composition in range of about 40FTU/g to about 40,000FTU/g composition, more preferably about 80FTU/g composition to about 20,000FTU/g composition, and even more preferably about 100FTU/g composition to about 10,000FTU/g composition, and even more preferably about 200FTU/g composition to about 10,000FTU/g composition.

In one embodiment the phytase is present in the feed additive composition at more than about 40FTU/g composition, suitably more than about 60FTU/g composition, suitably more than about 100FTU/g composition, suitably more than about 150FTU/g composition, suitably more than about 200FTU/g composition.

In one embodiment the phytase is present in the feed additive composition at less than about 40,000FTU/g composition, suitably less than about 20,000FTU/g composition, suitably less than about 15,000FTU/g composition, suitably less than about 10,000FTU/g composition.

It will be understood that as used herein 1 FTU (phytase unit) is defined as the amount of enzyme required to release 1 µmol of inorganic orthophosphate from a substrate in one minute under the reaction conditions defined in the ISO 2009 phytase assay - A standard assay for determining phytase activity and 1 FTU can be found at *International Standard ISO*/*DIS 30024: 1-17, 2009.*

In one embodiment suitably the enzyme is classified using the E.C. classification above, and the E.C. classification designates an enzyme having that activity when tested in the assay taught herein for determining 1 FTU.

### Advantages

It has surprisingly been found that *Citrobacter* phytases (in particular *Citrobacter braakii* phytases and/or *Citrobacter freundii* phytases) in combination with DFMs improve a subject's resistance to necrotic enteritis, e.g. that a reduction in lesion scores for instance can be seen.

What is particularly surprising is that the Citrobacter phytases (e.g. from *Citrobacter braakii* and/or *Citrobacter freundii*) as taught herein above improve a subjects resistance to necrotic enteritis significantly more than other known phytases (e.g. from non-Citrobacter organisms) known in the art when combined with a DFM.

In one embodiment the effect is even more pronounced with the *Citrobacter braakii* phytase sold as Ronozyme HiPhos™ as taught herein compared with phytases from other *Citrobacter braakii* strains (e.g. *C. braakii* phytase from strain YH-15 - whose sequence is shown herein as SEQ ID No. 7) - which was completely unexpected.

Again without wishing to be bound by theory one suggestion how the combination of *Citrobacter* phytases and DFMs provides surprisingly better results compared with other phytases and DFMs is that *C*. *braakii* phytases have a higher activity at lower pHs (e.g. 3.5-4.5) compared with some other *non-Citrobacter* phytases. As the first part of the gastrointestinal (GI) tract of monogastric farm animals, e.g. swine or poultry, has a low pH - *C. braakii* phytases appear to have more activity in this part of the GI tract thus these phytases are capable of releasing phosphorus and other nutrients, such as protein, much faster from the phytate substrate compared with some other *non-Citrobacter* phytases. This is advantageous in many ways, including that it is desirable to act on the phytate as soon as possible as it has a tendency to complex with other substances such as minerals and proteins, particularly as the pH rises. Once the phytate complexes it can be less accessible by enzymes for breakdown. Therefore acting on the phytate substrate early on in the GI tract when the pH is still low is desirable. However the breakdown of the phytate in the early part of the GI tract means that there can be less phosphorus available in the jejunum and the lower part of the GI tract which can have a negative impact on the populations of commensal "good" bacteria such as the *Lactobacilli* (which have been shown to have beneficial effects such as immune modulation and the production of organic acids that lower the intestinal pH). This negative impact on resident "good" bacteria can result in opportunistic pathogens to flourish - thus upsetting the overall balance of bacteria in the gut.

With regard to the present invention, it has surprisingly been found that the negative impact of using *C. braakii* phytases can be overcome by combining their use with one or more DFM. The DFM reestablishes the balance of bacteria in the gut - thus leading to reduced gut damage due to pathogenic bacteria and higher performance of the animal subject.

Again without wishing to be bound by theory a further suggestion how the combination of *Citrobacter* phytases and DFMs provides surprisingly better results compared with other phytases and DFMs is that *Citrobacter braakii* phytases have a higher activity at lower pHs (e.g. 3.5-4.5) compared with some other *non-Citrobacter* phytases. As the first part of the gastrointestinal (GI) tract has a low pH - *C. braakii* phytases appear to have more activity in this part of the GI tract. This can improve protein digestion by a subject because phytate can form complexes by binding proteins. The results of this early increase in adsorption of proteins can result in the animal producing less hydrochloric acid (HCl) - this can have a negative impact later in the GI tract as it can increase the pH in the later part of the GI tract. Increasing pH in the later part of the GI tract is not advantageous as it increases the chances of pathogens being able to establish themselves within the gut. Surprisingly it has been found by the present inventions that these negative effects of using *Citrobacter* phytases can be overcome by combining them with DFMs.

Surprisingly the 6-phytase from *C. braakii* strain ATCC 51113 (with the amino acid sequence SEQ ID No. 1) is even more positively influenced that even other *C*. *braakii* 6-phytase enzymes such as the 6-phytase from *C. braakii* strain YH-15 (with the amino acid sequence SEQ ID No. 7).

### Formulation of the DFM with the enzyme

The DFM and the enzymes may be formulated in any suitable way to ensure that the formulation comprises viable DFMs and an active enzyme.

In one embodiment the DFM and enzymes may be formulated as a liquid, a dry powder or a granule.

The dry powder or granules may be prepared by means known to those skilled in the art, such as, in top-spray fluid bed coater, in a buttom spray Wurster or by drum granulation (e.g. High sheer granulation), extrusion, pan coating or in a microingredients mixer.

For some embodiments the DFM and/or the enzyme(s) may be coated, for example encapsulated. Suitably the DFM and enzymes may be formulated within the same coating or encapsulated within the same capsule. Alternatively one or both of the enzymes may be formulated within the same coating or encapsulated within the same capsule and the DFM could be formulated in a coating separate to the one or both of the enzymes. In some embodiments, such as where the DFM is capable of producing endospores, the DFM may be provided without any coating. In such circumstances, the DFM endospores may be simply admixed with one or both enzymes. In the latter case, the enzymes may be coated, e.g. encapsulated, for instance one or both of the enzymes may be coated, e.g. encapsulated. The enzymes may be encapsulated as mixtures (i.e. comprising one or both) of the enzymes or they may be encapsulated separately, e.g. as single enzymes. In one preferred embodiment both enzymes may be coated, e.g. encapsulated, together.

In one embodiment the coating protects the enzymes from heat and may be considered a thermoprotectant.

In one embodiment the feed additive composition is formulated to a dry powder or granules as described in WO2007/044968 (referred to as TPT granules) or WO1997/016076 or WO1992/012645 (each of which is incorporated herein by reference).

In one aspect a feed of the present invention comprises a steam treated pelletised feed composition comprising a granule comprising a core and one or more coatings. The core may be a salt granule or the like onto which an enzyme solution may have been sprayed so as to form a layer thereon. The core comprises one or more active compounds, such as at least the phytase and/or DFM of the present invention. At least one of the coatings can be a moisture barrier coating. In some embodiments at least one of the coatings comprises a salt. For certain embodiments, the granules are approximately 210 to 390 µm in size. In some embodiments, the granules may be up to 450 µm or more in size or up to 500 µm or more in size. Examples of such an embodiment may be found in WO 2006/034710, WO 00/01793, WO 99/32595, WO 2007/044968, WO 00/47060, WO 03/059086, WO 03/059087, WO 2006/053564 and US 2003/0054511.

A preferred salt for the coating of the pellets is one or more of that described in WO2006/034710. Examples of preferred salts for coating the pellets include one or more of: Na₂SO₄ NaCl, Na₂CO₃, NaNO₃, Na₂HPO₄, Na₃PO₄, NH₄CL, (NH₄)₂HPO₄, NH₄H₂PO₄, (NH₄)₂SO₄, KCI, K₂HPO₄, KH₂PO₄, KNO₃, K₂SO₄, KHSO₄, MgSO₄, ZnSO₄ and sodium citrate or mixtures thereof. For some aspects, examples of more preferred salts for coating the pellets include one or more sulphates, such as one or more Na₂SO₄, (NH₄)₂SO₄, K₂SO₄, KHSO₄, MgSO₄, ZnSO₄ or mixtures thereof. For some aspects, examples of more preferred salts for coating the pellets include one or more Na₂SO₄, (NH₄)₂SO₄, and MgSO₄ or mixtures thereof. For some aspects, a preferred salt for coating the pellets is or includes at least Na₂SO₄.

In certain aspects the feed of the present invention comprises a granule that comprises a core, wherein the core comprises at least a phytase and/or DFM according to the present invention, and wherein the core is coated with one or more coatings, wherein at least one of the coatings comprises a moisture barrier. The granule may be a steam treated granule. The granule may be a steam treated pelletised granule.

In certain aspects the feed of the present invention comprises a granule, wherein the granule comprises a core that comprises at least a phytase and/or DFM according to the present invention, and wherein the core is coated with one or more coatings, wherein at least one of the coatings comprises a salt that is capable of acting as a moisture barrier. The granule may be a steam treated granule. The granule may be a steam treated pelletised granule.

In certain aspects the feed of the present invention comprises a granule, wherein the granule comprises a core that comprises at least a phytase and/or DFM according to the present invention, and wherein the core is coated with one or more coatings, wherein at least one of the coatings comprises one or more of Na₂SO₄ NaCl, Na₂CO₃, NaNO₃, Na₂HPO₄, Na₃PO₄, NH₄CL, (NH₄)₂HPO₄, NH₄H₂PO₄, (NH₄)₂SO₄, KCI, K₂HPO₄, KH₂PO₄, KNO₃, K₂SO₄, KHSO₄, MgSO₄, ZnSO₄ and sodium citrate or mixtures thereof. The granule may be a steam treated granule. The granule may be a steam treated pelletised granule.

In certain aspects the feed of the present invention comprises a granule, wherein the granule comprises a core that comprises at least a phytase and/or DFM according to the present invention, and wherein the core is coated with one or more coatings, wherein at least one of the coatings comprises one or more sulphates, such as one or more Na₂SO₄, (NH₄)₂SO₄, K₂SO₄, KHSO₄, MgSO₄, ZnSO₄ or mixtures thereof. The granule may be a steam treated granule. The granule may be a steam treated pelletised granule.

In certain aspects the feed of the present invention comprises a granule, wherein the granule comprises a core that comprises at least a phytase and/or DFM according to the present invention, and wherein the core is coated with one or more coatings, wherein at least one of the coatings comprises one or more of Na₂SO₄, (NH₄)₂SO₄, and MgSO₄ or mixtures thereof. The granule may be a steam treated granule. The granule may be a steam treated pelletised granule.

In certain aspects the feed of the present invention comprises a granule, wherein the granule comprises a core that comprises at least a phytase and/or DFM according to the present invention, and wherein the core is coated with one or more coatings, wherein at least one of the coatings is or includes at least Na₂SO₄. The granule may be a steam treated granule. The granule may be a steam treated pelletised granule.

In one embodiment the feed additive composition may be formulated to a granule for feed compositions comprising: a core; an active agent; and at least one coating, the active agent of the granule retaining at least 50% activity, at least 60% activity, at least 70% activity, at least 80% activity after conditions selected from one or more of a) a feed pelleting process, b) a steam-heated feed pretreatment process, c) storage, d) storage as an ingredient in an unpelleted mixture, and e) storage as an ingredient in a feed base mix or a feed premix comprising at least one compound selected from trace minerals, organic acids, reducing sugars, vitamins, choline chloride, and compounds which result in an acidic or a basic feed base mix or feed premix.

With regard to the granule at least one coating may comprise a moisture hydrating material that constitutes at least 55% w/w of the granule; and/or at least one coating may comprise two coatings. The two coatings may be a moisture hydrating coating and a moisture barrier coating. In some embodiments, the moisture hydrating coating may be between 25% and 60% w/w of the granule and the moisture barrier coating may be between 2% and 15% w/w of the granule. The moisture hydrating coating may be selected from inorganic salts, sucrose, starch, and maltodextrin and the moisture barrier coating may be selected from polymers, gums, whey and starch.

The granule may be produced using a feed pelleting process and the feed pretreatment process may be conducted between 70°C and 95°C for up to several minutes, such as between 85°C and 95°C.

In one embodiment the feed additive composition may be formulated to a granule for animal feed comprising: a core; an active agent, the active agent of the granule retaining at least 80% activity after storage and after a steam-heated pelleting process where the granule is an ingredient; a moisture barrier coating; and a moisture hydrating coating that is at least 25% w/w of the granule, the granule having a water activity of less than 0.5 prior to the steam-heated pelleting process.

The granule may have a moisture barrier coating selected from polymers and gums and the moisture hydrating material may be an inorganic salt. The moisture hydrating coating may be between 25% and 45% w/w of the granule and the moisture barrier coating may be between 2% and 10% w/w of the granule.

The granule may be produced using a steam-heated pelleting process which may be conducted between 85°C and 95°C for up to several minutes.

In some embodiments the DFM (e.g. DFM endospores for example) may be diluted using a diluent, such as starch powder, limestone or the like.

In one embodiment, the composition is in a liquid formulation suitable for consumption preferably such liquid consumption contains one or more of the following: a buffer, salt, sorbitol and/or glycerol.

In another embodiment the feed additive composition may be formulated by applying, e.g. spraying, the enzyme(s) onto a carrier substrate, such as ground wheat for example.

In one embodiment the feed additive composition according to the present invention may be formulated as a premix. By way of example only the premix may comprise one or more feed components, such as one or more minerals and/or one or more vitamins.

In one embodiment the DFM and/or enzymes for use in the present invention are formulated with at least one physiologically acceptable carrier selected from at least one of maltodextrin, limestone (calcium carbonate), cyclodextrin, wheat or a wheat component, sucrose, starch, Na₂SO₄, Talc, PVA, sorbitol, benzoate, sorbiate, glycerol, sucrose, propylene glycol, 1,3-propane diol, glucose, parabens, sodium chloride, citrate, acetate, phosphate, calcium, metabisulfite, formate and mixtures thereof.

### Packaging

In one embodiment the feed additive composition and/or premix and/or feed or feedstuff according to the present invention is packaged.

In one preferred embodiment the feed additive composition and/or premix and/or feed or feedstuff is packaged in a bag, such as a paper bag.

In an alternative embodiment the feed additive composition and/or premix and/or feed or feedstuff may be sealed in a container. Any suitable container may be used.

### Feed

The feed additive composition of the present invention may be used as - or in the preparation of - a feed.

The term "feed" is used synonymously herein with "feedstuff".

The feed may be in the form of a solution or as a solid - depending on the use and/or the mode of application and/or the mode of administration.

When used as - or in the preparation of - a feed - such as functional feed - the composition of the present invention may be used in conjunction with one or more of: a nutritionally acceptable carrier, a nutritionally acceptable diluent, a nutritionally acceptable excipient, a nutritionally acceptable adjuvant, a nutritionally active ingredient.

In a preferred embodiment the feed additive composition of the present invention is admixed with a feed component to form a feedstuff.

The term "feed component" as used herein means all or part of the feedstuff. Part of the feedstuff may mean one constituent of the feedstuff or more than one constituent of the feedstuff, e.g. 2 or 3 or 4. In one embodiment the term "feed component" encompasses a premix or premix constituents.

Preferably the feed may be a fodder, or a premix thereof, a compound feed, or a premix thereof. In one embodiment the feed additive composition according to the present invention may be admixed with a compound feed, a compound feed component or to a premix of a compound feed or to a fodder, a fodder component, or a premix of a fodder.

The term fodder as used herein means any food which is provided to an animal (rather than the animal having to forage for it themselves). Fodder encompasses plants that have been cut.

The term fodder includes hay, straw, silage, compressed and pelleted feeds, oils and mixed rations, and also sprouted grains and legumes.

Fodder may be obtained from one or more of the plants selected from: alfalfa (lucerne), barley, birdsfoot trefoil, brassicas, Chau moellier, kale, rapeseed (canola), rutabaga (swede), turnip, clover, alsike clover, red clover, subterranean clover, white clover, grass, false oat grass, fescue, Bermuda grass, brome, heath grass, meadow grasses (from naturally mixed grassland swards, orchard grass, rye grass, Timothy-grass, corn (maize), millet, oats, sorghum, soybeans, trees (pollard tree shoots for tree-hay), wheat, and legumes.

The term "compound feed" means a commercial feed in the form of a meal, a pellet, nuts, cake or a crumble. Compound feeds may be blended from various raw materials and additives. These blends are formulated according to the specific requirements of the target animal.

Compound feeds can be complete feeds that provide all the daily required nutrients, concentrates that provide a part of the ration (protein, energy) or supplements that only provide additional micronutrients, such as minerals and vitamins.

The main ingredients used in compound feed are the feed grains, which include corn, soybeans, sorghum, oats, wheat and barley.

Suitably a premix as referred to herein may be a composition composed of microingredients such as vitamins, minerals, chemical preservatives, antibiotics, fermentation products, and other essential ingredients. Premixes are usually compositions suitable for blending into commercial rations.

Any feedstuff of the present invention may comprise one or more feed materials selected from the group comprising a) cereals, such as small grains (e.g., wheat, barley, rye, oats and combinations thereof) and/or large grains such as maize or sorghum; b) by products from cereals, such as corn gluten meal, Distillers Dried Grain Solubles (DDGS), wheat bran, wheat middlings, wheat shorts, rice bran, rice hulls, oat hulls, palm kernel, and citrus pulp; c) protein obtained from sources such as soya, sunflower, peanut, lupin, peas, fava beans, cotton, canola, fish meal, dried plasma protein, meat and bone meal, potato protein, whey, copra, sesame; d) oils and fats obtained from vegetable and animal sources; e) minerals and vitamins.

A feedstuff of the present invention may contain at least 30%, at least 40%, at least 50% or at least 60% by weight corn and soybean meal or corn and full fat soy, or wheat meal or sunflower meal.

In addition or in the alternative, a feedstuff of the present invention may comprise at least one high fibre feed material and/or at least one by-product of the at least one high fibre feed material to provide a high fibre feedstuff. Examples of high fibre feed materials include: wheat, barley, rye, oats, by products from cereals, such as corn gluten meal, Distillers Dried Grain Solubles (DDGS), wheat bran, wheat middlings, wheat shorts, rice bran, rice hulls, oat hulls, palm kernel, and citrus pulp. Some protein sources may also be regarded as high fibre: protein obtained from sources such as sunflower, lupin, fava beans and cotton.

In the present invention the feed may be one or more of the following: a compound feed and premix, including pellets, nuts or (cattle) cake; a crop or crop residue: corn, soybeans, sorghum, oats, barley, corn stover, copra, straw, chaff, sugar beet waste; fish meal; freshly cut grass and other forage plants; meat and bone meal; molasses; oil cake and press cake; oligosaccharides; conserved forage plants: hay and silage; seaweed; seeds and grains, either whole or prepared by crushing, milling etc.; sprouted grains and legumes; yeast extract.

As used herein the term "contacted" refers to the indirect or direct application of the composition of the present invention to the product (e.g. the feed). Examples of the application methods which may be used, include, but are not limited to, treating the product in a material comprising the feed additive composition, direct application by mixing the feed additive composition with the product, spraying the feed additive composition onto the product surface or dipping the product into a preparation of the feed additive composition.

In one embodiment the feed additive composition of the present invention is preferably admixed with the product (e.g. feedstuff). Alternatively, the feed additive composition may be included in the emulsion or raw ingredients of a feedstuff.

For some applications, it is important that the composition is made available on or to the surface of a product to be affected/treated. This allows the composition to impart one or more of the following favourable characteristics: performance benefits.

The feed additive compositions of the present invention may be applied to intersperse, coat and/or impregnate a product (e.g. feedstuff or raw ingredients of a feedstuff) with a controlled amount of DFM and enzymes.

The DFM and enzyme may be used simultaneously (e.g. when they are in admixture together or even when they are delivered by different routes) or sequentially (e.g. they may be delivered by different routes). In one embodiment preferably the DFM and enzymes are applied simultaneously. Preferably the DFM and enzymes are admixed prior to being delivered to a feedstuff or to a raw ingredient of a feedstuff.

The DFM in feed additive compositions according to the present invention - can be added in suitable concentrations - such as for example in concentrations in the final feed product which offer a daily dose of between about 2x10⁵ CFU to about 2x10¹¹ CFU, suitably between about 2x10⁶ to about 1x10¹⁰, suitably between about 3.75x10⁷ CFU to about 1x10¹⁰ CFU.

Preferably, the feed additive composition of the present invention will be thermally stable to heat treatment up to about 70 °C; up to about 85°C; or up to about 95°C. The heat treatment may be performed for up to about 1 minute; up to about 5 minutes; up to about 10 minutes; up to about 30 minutes; up to about 60 minutes. The term thermally stable means that at least about 75% of the enzyme components and/or DFM that were present/active in the additive before heating to the specified temperature are still present/active after it cools to room temperature. Preferably, at least about 80% of the enzyme components and/or DFM that were present and active in the additive before heating to the specified temperature are still present and active after it cools to room temperature.

In a particularly preferred embodiment the feed additive composition is homogenized to produce a powder.

In an alternative preferred embodiment, the feed additive composition is formulated to granules as described in WO2007/044968 (referred to as TPT granules).

In another preferred embodiment when the feed additive composition is formulated into granules the granules comprise a hydrated barrier salt coated over the protein core. The advantage of such salt coating is improved thermo-tolerance, improved storage stability and protection against other feed additives otherwise having adverse effect on the enzyme and/or DFM.

Preferably, the salt used for the salt coating has a water activity greater than 0.25 or constant humidity greater than 60 % at 20 °C.

Preferably, the salt coating comprises a Na₂SO₄.

The method of preparing a feed additive composition may also comprise the further step of pelleting the powder. The powder may be mixed with other components known in the art. The powder, or mixture comprising the powder, may be forced through a die and the resulting strands are cut into suitable pellets of variable length.

Optionally, the pelleting step may include a steam treatment, or conditioning stage, prior to formation of the pellets. The mixture comprising the powder may be placed in a conditioner, e.g. a mixer with steam injection. The mixture is heated in the conditioner up to a specified temperature, such as from 60-100°C, typical temperatures would be 70°C, 80°C, 85°C, 90°C or 95°C. The residence time can be variable from seconds to minutes and even hours. Such as 5 seconds, 10 seconds, 15 seconds, 30 seconds, 1 minutes, 2 minutes, 5 minutes, 10 minutes, 15 minutes, 30 minutes and 1 hour.

It will be understood that the feed additive composition of the present invention is suitable for addition to any appropriate feed material.

As used herein, the term feed material refers to the basic feed material to be consumed by an animal. It will be further understood that this may comprise, for example, at least one or more unprocessed grains, and/or processed plant and/or animal material such as soybean meal or bone meal.

As used herein, the term "feedstuff' refers to a feed material to which one or more feed additive compositions have been added.

It will be understood by the skilled person that different animals require different feedstuffs, and even the same animal may require different feedstuffs, depending upon the purpose for which the animal is reared.

Preferably, the feedstuff may comprise feed materials comprising maize or corn, wheat, barley, triticale, rye, rice, tapioca, sorghum, and/ or any of the by-products, as well as protein rich components like soybean mean, rape seed meal, canola meal, cotton seed meal, sunflower seed meal, animal-by-product meals and mixtures thereof. More preferably, the feedstuff may comprise animal fats and / or vegetable oils.

Optionally, the feedstuff may also contain additional minerals such as, for example, calcium and/or additional vitamins.

Preferably, the feedstuff is a corn soybean meal mix.

In one embodiment, preferably the feed is not pet food.

In another aspect there is provided a method for producing a feedstuff. Feedstuff is typically produced in feed mills in which raw materials are first ground to a suitable particle size and then mixed with appropriate additives. The feedstuff may then be produced as a mash or pellets; the later typically involves a method by which the temperature is raised to a target level and then the feed is passed through a die to produce pellets of a particular size. The pellets are allowed to cool. Subsequently liquid additives such as fat and enzyme may be added. Production of feedstuff may also involve an additional step that includes extrusion or expansion prior to pelleting - in particular by suitable techniques that may include at least the use of steam.

The feedstuff as according to the invention is for poultry, for example, broiler, layer, broiler breeders, turkey, duck, geese and water fowl.

In one embodiment the feedstuff is not for a layer.

By way of example only a feedstuff for chickens, e.g. broiler chickens may be comprised of one or more of the ingredients listed in the table below, for example in the %ages given in the table below:

| **Ingredients** | **Starter (%)** | **Finisher (%)** |
|---|---|---|
| Maize | 46.2 | 46.7 |
| Wheat Middlings | 6.7 | 10.0 |
| Maize DDGS | 7.0 | 7.0 |
| Soyabean Meal 48%CP | 32.8 | 26.2 |
| An/Veg Fat blend | 3.0 | 5.8 |
| L-Lysine HCl | 0.3 | 0.3 |
| DL-methionine | 0.3 | 0.3 |
| L-threonine | 0.1 | 0.1 |
| Salt | 0.3 | 0.4 |
| Limestone | 1.1 | 1.1 |
| Dicalcium Phosphate | 1.2 | 1.2 |
| Poultry Vitamins and Micro-minerals | 0.3 | 0.3 |

By way of example only the diet specification for chickens, such as broiler chickens, may be as set out in the Table below:

| **Diet specification** | | |
|---|---|---|
| Crude Protein (%) | 23.00 | 20.40 |
| Metabolizable Energy Poultry (kcal/kg) | 2950 | 3100 |
| Calcium (%) | 0.85 | 0.85 |
| Available Phosphorus (%) | 0.38 | 0.38 |
| Sodium (%) | 0.18 | 0.19 |
| Dig. Lysine (%) | 1.21 | 1.07 |
| Dig. Methionine (%) | 0.62 | 0.57 |
| Dig. Methionine + Cysteine (%) | 0.86 | 0.78 |
| Dig. Threonine (%) | 0.76 | 0.68 |

By way of example only a feedstuff laying hens may be comprises of one or more of the ingredients listed in the table below, for example in the %ages given in the table below:

| **Ingredient** | **Laying phase (%)** |
|---|---|
| Maize | 10.0 |
| Wheat | 53.6 |
| Maize DDGS | 5.0 |
| Soybean Meal 48%CP | 14.9 |
| Wheat Middlings | 3.0 |
| Soybean Oil | 1.8 |
| L-Lysine HCl | 0.2 |
| DL-methionine | 0.2 |
| L-threonine | 0.1 |
| Salt | 0.3 |
| Dicalcium Phosphate | 1.6 |
| Limestone | 8.9 |
| Poultry Vitamins and Micro-minerals | 0.6 |

By way of example only the diet specification for laying hens may be as set out in the Table below:

| **Diet specification** | |
|---|---|
| Crude Protein (%) | 16.10 |
| Metabolizable Energy Poultry (kcal/kg) | 2700 |
| Lysine (%) | 0.85 |
| Methionine (%) | 0.42 |
| Methionine + Cysteine (%) | 0.71 |
| Threonine (%) | 0.60 |
| Calcium (%) | 3.85 |
| Available Phosphorus (%) | 0.42 |
| Sodium (%) | 0.16 |

By way of example only a feedstuff for turkeys may be comprises of one or more of the ingredients listed in the table below, for example in the %ages given in the table below:

| **Ingredient** | **Phase 1 (%)** | **Phase 2 (%)** | **Phase 3 (%)** | **Phase 4 (%)** |
|---|---|---|---|---|
| Wheat | 33.6 | 42.3 | 52.4 | 61.6 |
| Maize DDGS | 7.0 | 7.0 | 7.0 | 7.0 |
| Soyabean Meal 48%CP | 44.6 | 36.6 | 27.2 | 19.2 |
| Rapeseed Meal | 4.0 | 4.0 | 4.0 | 4.0 |
| Soyabean Oil | 4.4 | 4.2 | 3.9 | 3.6 |
| L-Lysine HCl | 0.5 | 0.5 | 0.4 | 0.4 |
| DL-methionine | 0.4 | 0.4 | 0.3 | 0.2 |
| L-threonine | 0.2 | 0.2 | 0.1 | 0.1 |
| Salt | 0.3 | 0.3 | 0.3 | 0.3 |
| Limestone | 1.0 | 1.1 | 1.1 | 1.0 |
| Dicalcium Phosphate | 3.5 | 3.0 | 2.7 | 2.0 |
| Poultry Vitamins and Micro-minerals | 0.4 | 0.4 | 0.4 | 0.4 |

By way of example only the diet specification for turkeys may be as set out in the Table below:

| **Diet specification** | | | | |
|---|---|---|---|---|
| Crude Protein (%) | 29.35 | 26.37 | 22.93 | 20.00 |
| Metabolizable Energy Poultry (kcal/kg) | 2.850 | 2.900 | 2.950 | 3.001 |
| Calcium (%) | 1.43 | 1.33 | 1.22 | 1.02 |
| Available Phosphorus (%) | 0.80 | 0.71 | 0.65 | 0.53 |
| Sodium (%) | 0.16 | 0.17 | 0.17 | 0.17 |
| Dig. Lysine (%) | 1.77 | 1.53 | 1.27 | 1.04 |
| Dig. Methionine (%) | 0.79 | 0.71 | 0.62 | 0.48 |
| Dig. Methionine + Cysteine (%) | 1.12 | 1.02 | 0.90 | 0.74 |
| Dig. Threonine (%) | 1.03 | 0.89 | 0.73 | 0.59 |

By way of example only a feedstuff for piglets may be comprises of one or more of the ingredients listed in the table below, for example in the %ages given in the table below:

| **Ingredient** | **Phase 1 (%)** | **Phase 2 (%)** |
|---|---|---|
| Maize | 20.0 | 7.0 |
| Wheat | 25.9 | 46.6 |
| Rye | 4.0 | 10.0 |
| Wheat middlings | 4.0 | 4.0 |
| Maize DDGS | 6.0 | 8.0 |
| Soyabean Meal 48% CP | 25.7 | 19.9 |
| Dried Whey | 10.0 | 0.0 |
| Soyabean Oil | 1.0 | 0.7 |
| L-Lysine HCI | 0.4 | 0.5 |
| DL-methionine | 0.2 | 0.2 |
| L-threonine | 0.1 | 0.2 |
| L-tryptophan | 0.03 | 0.04 |
| Limestone | 0.6 | 0.7 |
| Dicalcium Phosphate | 1.6 | 1.6 |
| Swine Vitamins and Micro-minerals | 0.2 | 0.2 |
| Salt | 0.2 | 0.4 |

By way of example only the diet specification for piglets may be as set out in the Table below:

| **Diet specification** | | |
|---|---|---|
| Crude Protein (%) | 21.50 | 20.00 |
| Swine Digestible Energy (kcal/kg) | 3380 | 3320 |
| Swine Net Energy (kcal/kg) | 2270 | 2230 |
| Calcium (%) | 0.80 | 0.75 |
| Digestible Phosphorus (%) | 0.40 | 0.35 |
| Sodium (%) | 0.20 | 0.20 |
| Dig. Lysine (%) | 1.23 | 1.14 |
| Dig. Methionine (%) | 0.49 | 0.44 |
| Dig. Methionine + Cysteine (%) | 0.74 | 0.68 |
| Dig. Threonine (%) | 0.80 | 0.74 |

By way of example only a feedstuff for grower/finisher pigs may be comprises of one or more of the ingredients listed in the table below, for example in the %ages given in the table below:

| **Ingredient** | **Grower/ Finisher (%)** |
|---|---|
| Maize | 27.5 |
| Soyabean Meal 48% CP | 15.4 |
| Maize DDGS | 20.0 |
| Wheat bran | 11.1 |
| Rice bran | 12.0 |
| Canola seed meal | 10.0 |
| Limestone | 1.6 |
| Dicalcium phosphate | 0.01 |
| Salt | 0.4 |
| Swine Vitamins and Micro-minerals | 0.3 |
| Lysine-HCI | 0.2 |
| Vegetable oil | 0.5 |

By way of example only the diet specification for grower/finisher pigs may be as set out in the Table below:

| **Diet specification** | |
|---|---|
| Crude Protein (%) | 22.60 |
| Swine Metabolizable Energy (kcal/kg) | 3030 |
| Calcium (%) | 0.75 |
| Available Phosphorus (%) | 0.29 |
| Digestible Lysine (%) | 1.01 |
| Dig. Methionine + Cysteine (%) | 0.73 |
| Digestible Threonine (%) | 0.66 |

### Forms

The feed additive composition of the present invention and other components and/or the feedstuff comprising same may be used in any suitable form.

The feed additive composition of the present invention may be used in the form of solid or liquid preparations or alternatives thereof. Examples of solid preparations include powders, pastes, boluses, capsules, pellets, tablets, dusts, and granules which may be wettable, spray-dried or freeze-dried. Examples of liquid preparations include, but are not limited to, aqueous, organic or aqueous-organic solutions, suspensions and emulsions.

In some applications, DFM or feed additive compositions of the present invention may be mixed with feed or administered in the drinking water. In one embodiment the dosage range for inclusion into water is about 1x10³ CFU/animal/day to about 1x10¹⁰ CFU/animal/day, and more preferably about 1x10⁷ CFU/animal/day.

Suitable examples of forms include one or more of: powders, pastes, boluses, pellets, tablets, pills, capsules, ovules, solutions or suspensions, which may contain flavouring or colouring agents, for immediate-, delayed-, modified-, sustained-, pulsed- or controlled-release applications.

By way of example, if the composition of the present invention is used in a solid, e.g. pelleted form, it may also contain one or more of: excipients such as microcrystalline cellulose, lactose, sodium citrate, calcium carbonate, dibasic calcium phosphate and glycine; disintegrants such as starch (preferably corn, potato or tapioca starch), sodium starch glycollate, croscarmellose sodium and certain complex silicates; granulation binders such as polyvinylpyrrolidone, hydroxypropylmethylcellulose (HPMC), hydroxypropylcellulose (HPC), sucrose, gelatin and acacia; lubricating agents such as magnesium stearate, stearic acid, glyceryl behenate and talc may be included.

Examples of nutritionally acceptable carriers for use in preparing the forms include, for example, water, salt solutions, alcohol, silicone, waxes, petroleum jelly, vegetable oils, polyethylene glycols, propylene glycol, liposomes, sugars, gelatin, lactose, amylose, magnesium stearate, talc, surfactants, silicic acid, viscous paraffin, perfume oil, fatty acid monoglycerides and diglycerides, petroethral fatty acid esters, hydroxymethyl-cellulose, polyvinylpyrrolidone, and the like.

Preferred excipients for the forms include lactose, starch, a cellulose, milk sugar or high molecular weight polyethylene glycols.

For aqueous suspensions and/or elixirs, the composition of the present invention may be combined with various sweetening or flavouring agents, colouring matter or dyes, with emulsifying and/or suspending agents and with diluents such as water, propylene glycol and glycerin, and combinations thereof.

Non-hydroscopic whey is often used as a carrier for DFMs (particularly bacterial DFMs) and is a good medium to initiate growth.

Bacterial DFM containing pastes may be formulated with vegetable oil and inert gelling ingredients.

Fungal products may be formulated with grain by-products as carriers.

In one embodiment preferably the feed additive composition according to the present invention is not in the form of a microparticle system, such as the microparticle system taught in WO2005/123034.

### Dosing

The DFM and/or feed additive composition according to the present invention may be designed for one-time dosing or may be designed for feeding on a daily basis.

The optimum amount of the composition (and each component therein) to be used in the combination of the present invention will depend on the product to be treated and/or the method of contacting the product with the composition and/or the intended use for the same.

The amount of DFM and enzymes used in the compositions should be a sufficient amount to be effective and to remain sufficiently effective in improving the performance of the animal fed feed products containing said composition. This length of time for effectiveness should extend up to at least the time of utilisation of the product (e.g. feed additive composition or feed containing same).

The ratio of DFM to each enzyme in the feed can be in the ranges given below:
DFM:phytase (CFU/FTU): In range from 5.0x10²CFU DFM: 1FTU, enzyme to 5.0x10⁹ CFU :1FTU enzyme; preferably in the range from 7.5x10⁴CFU DFM: 1FTU enzyme to 2.5x10⁷ CFU :1FTU enzyme.

In one embodiment preferably the feedstuff comprises the following:
a phytase at at least 500FTU/kg of feed; and
Enviva Pro® (DFM) at 75,000 CFU/g to 150,000 CFU/g of feed.

In one embodiment preferably the feedstuff comprises the following:
a phytase at 500FTU/kg of feed; and
Enviva Pro® (DFM) at 75,000 CFU/g to 150,000 CFU/g of feed.

In another embodiment the feedstuff comprises the following:
a phytase at 625FTU/kg of feed; and
Enviva Pro® (DFM) at 37,500 CFU/g to 75,000 CFU/g of feed.

In a preferred embodiment the feed additive composition comprises sufficient enzyme and DFMs to dose the feedstuff as follows:
a phytase at 500FTU/kg of feed; and
Enviva Pro® (DFM) at 75,000 CFU/g to 150,000 CFU/g of feed.

In a preferred embodiment the feed additive composition comprises sufficient enzyme and DFMs to dose the feedstuff as follows:
a phytase at 500FTU/kg of feed; and
Enviva Pro® (DFM) at 37,500 CFU/g to 75,000 CFU/g of feed.

The Enviva Pro® listed in these preferred embodiments can be replaced by any other DFM taught herein.

Preferably the phytase listed in these preferred embodiments is the enzyme Ronozyme HiPhos™ or has an amino acid which has at least 98.6% identity with amino acids 23-433 of SEQ ID No. 1 or SEQ ID No. 2.

In a preferred combination the feed additive composition comprises a combination of the phytase which is the enzyme Ronozyme HiPhos™ or has an amino acid which has at least 98.6% identity with amino acids 23-433 of SEQ ID No. 1 or SEQ ID No. 2 with a DFM, preferably the DFM is selected from the group consisting of Envivo Pro®, Calsporin®, Clostat®, Gallipro®, GalliproMax®, Proflora®, CSI®, Sorbiflore® and Animavit®,

In one embodiment the feed additive composition comprises a combination of the phytase which is the enzyme Ronozyme HiPhos™ or has an amino acid which has at least 98.6% identity with amino acids 23-433 of SEQ ID No. 1 or SEQ ID No. 2 with a DFM wherein the DFM is selected from the group consisting of bacterium from one or more of the following species: *Bacillus subtilis, Lactobacillus farciminis, Lactobacillus rhamnosusand* combinations thereof.

In a preferred combination the feed additive composition comprises a combination of the phytase derivable, preferably derived, from a *Citrobacter* bacterium selected from the group consisting of: *Citrobacter braakii, e.g. Citrobacter braakii* ATCC 51113; *Citrobacter freundii, e.g. C. freundii* NCIMB 41247; *Citrobacter amalonaticus,* e.g. *Citrobacter amaloriaticus* ATCC 25405 or *Citrobacter amalonaticus* ATCC 25407; *Citrobacter gillenii,* e.g. *Citrobacter gillenii* DSM 13694; *Citrobacter intermedius, Citrobacter koseri, Citrobacter murliniae, Citrobacter rodentium, Citrobacter sedlakii, Citrobacter werkmanii* and *Citrobacter youngae* with a DFM selected from the group consisting of Enviva Pro®, Calsporin®, Clostat®, Gallipro®, GalliproMax®, Gallipro®Tect®, Poultry star®, Protexin®, Proflora®, Ecobiol®, Ecobiol® Plus, Fortiflora®, BioPlus2B®, Lactiferm®, CSI®, Yea-Sac®, Biomin IMB52®, Biomin C5®, Biacton®, Oralin E1707®, Probios-pioneer PDFM®, Sorbiflore®, Animavit®, Bonvital®, Levucell SB 20®, Levucell SC 0 & SC10®, ME Bactocell ActiSaf® (formerly BioSaf®), Miya-Gold®, Fecinor, Fecinor Plus®, InteSwine®, BioSprint®, Provita®, PepSoyGen-C®, Toyocerin®, and TOYOCERIN®.

### Combination with Other Components

The DFM and enzyme(s) for use in the present invention may be used in combination with other components. Thus, the present invention also relates to combinations. The DFM in combination with a *Citrobacter* phytase (e.g. a *Citrobacter braakii* phytase or *Citrobacter freundii* phytase) may be referred to herein as "the feed additive composition of the present invention".

The combination of the present invention comprises the feed additive composition of the present invention (or one or more of the constituents thereof) and another component which is suitable for animal consumption and is capable of providing a medical or physiological benefit to the consumer.

In one embodiment the "another component" may be one or more further feed enzymes.

Suitable additional enzymes for use in the present invention may be one or more of the enzymes selected from the group consisting of: amylases, xylanases and/or proteases.

In one embodiment preferably the "another component" is not a further enzyme or a further DFM.

The components may be prebiotics. Prebiotics are typically non-digestible carbohydrate (oligo- or polysaccharides) or a sugar alcohol which is not degraded or absorbed in the upper digestive tract. Known prebiotics used in commercial products and useful in accordance with the present invention include inulin (fructo-oligosaccharide, or FOS) and transgalacto-oligosaccharides (GOS or TOS). Suitable prebiotics include palatinoseoligosaccharide, soybean oligosaccharide, alginate, xanthan, pectin, locust bean gum (LBG), inulin, guar gum, galacto-oligosaccharide (GOS), fructo-oligosaccharide (FOS), non-degradable starch, lactosaccharose, lactulose, lactitol, maltitol, maltodextrin, polydextrose (i.e. Litesse®), lactitol, lactosucrose, soybean oligosaccharides, palatinose, isomalto-oligosaccharides, gluco-oligosaccharides and xylo-oligosaccharides, pectin fragments, dietary fibres, mannan-oligosaccharides.

Dietary fibres may include non-starch polysaccharides, such as arabinoxylans, cellulose and many other plant components, such as resistant dextrins, inulin, lignin, waxes, chitins, pectins, beta-glucans and oligosaccharides.

In one embodiment the present invention relates to the combination of the feed additive composition according to the present invention (or one or more of the constituents thereof) with a prebiotic. In another embodiment the present invention relates to a feed additive composition comprising (or consisting essentially of or consisting of) a DFM in combination with a *Citrobacter* phytase and a prebiotic.

The prebiotic may be administered simultaneously with (e.g. in admixture together with or delivered simultaneously by the same or different routes) or sequentially to (e.g. by the same or different routes) the feed additive composition (or constituents thereof) according to the present invention.

Other components of the combinations of the present invention include polydextrose, such as Litesse®, and/or a maltodextrin and/or lactitol. These other components may be optionally added to the feed additive composition to assist the drying process and help the survival of DFM.

Further examples of other suitable components include one or more of: thickeners, gelling agents, emulsifiers, binders, crystal modifiers, sweeteners (including artificial sweeteners), rheology modifiers, stabilisers, anti-oxidants, dyes, enzymes, carriers, vehicles, excipients, diluents, lubricating agents, flavouring agents, colouring matter, suspending agents, disintegrants, granulation binders etc. These other components may be natural. These other components may be prepared by use of chemical and/or enzymatic techniques.

In one embodiment the DFM and/or enzyme may be encapsulated. In one embodiment the feed additive composition and/or DFM and/or enzyme is/are formulated as a dry powder or granule as described in WO2007/044968 (referred to as TPT granules).

In one preferred embodiment the DFM and/or enzyme for use in the present invention may be used in combination with one or more lipids.

For example, the DFM and/or enzyme for use in the present invention may be used in combination with one or more lipid micelles. The lipid micelle may be a simple lipid micelle or a complex lipid micelle.

The lipid micelle may be an aggregate of orientated molecules of amphipathic substances, such as a lipid and/or an oil.

As used herein the term "thickener or gelling agent" refers to a product that prevents separation by slowing or preventing the movement of particles, either droplets of immiscible liquids, air or insoluble solids. Thickening occurs when individual hydrated molecules cause an increase in viscosity, slowing the separation. Gelation occurs when the hydrated molecules link to form a three-dimensional network that traps the particles, thereby immobilising them.

The term "stabiliser" as used here is defined as an ingredient or combination of ingredients that keeps a product (e.g. a feed product) from changing over time.

The term "emulsifier" as used herein refers to an ingredient (e.g. a feed ingredient) that prevents the separation of emulsions. Emulsions are two immiscible substances, one present in droplet form, contained within the other. Emulsions can consist of oil-in-water, where the droplet or dispersed phase is oil and the continuous phase is water; or water-in-oil, where the water becomes the dispersed phase and the continuous phase is oil. Foams, which are gas-in-liquid, and suspensions, which are solid-in-liquid, can also be stabilised through the use of emulsifiers.

As used herein the term "binder" refers to an ingredient (e.g. a feed ingredient) that binds the product together through a physical or chemical reaction. During "gelation" for instance, water is absorbed, providing a binding effect. However, binders can absorb other liquids, such as oils, holding them within the product. In the context of the present invention binders would typically be used in solid or low-moisture products for instance baking products: pastries, doughnuts, bread and others.

"Carriers" or "vehicles" mean materials suitable for administration of the DFM and/or enzymes and include any such material known in the art such as, for example, any liquid, get, solvent, liquid diluent, solubilizer, or the like, which is non-toxic and which does not interact with any components of the composition in a deleterious manner.

The present invention provides a method for preparing a feed additive composition comprising admixing a DFM and a *Citrobacter* phytase with at least one physiologically acceptable carrier selected from at least one of maltodextrin, limestone (calcium carbonate), cyclodextrin, wheat or a wheat component, sucrose, starch, Na₂SO₄, Talc, PVA, sorbitol, benzoate, sorbiate, glycerol, sucrose, propylene glycol, 1,3-propane diol, glucose, parabens, sodium chloride, citrate, acetate, phosphate, calcium, metabisulfite, formate and mixtures thereof.

Examples of excipients include one or more of: microcrystalline cellulose and other celluloses, lactose, sodium citrate, calcium carbonate, dibasic calcium phosphate, glycine, starch, milk sugar and high molecular weight polyethylene glycols.

Examples of disintegrants include one or more of: starch (preferably corn, potato or tapioca starch), sodium starch glycollate, croscarmellose sodium and certain complex silicates.

Examples of granulation binders include one or more of: polyvinylpyrrolidone, hydroxypropylmethylcellulose (HPMC), hydroxypropylcellulose (HPC), sucrose, maltose, gelatin and acacia.

Examples of lubricating agents include one or more of: magnesium stearate, stearic acid, glyceryl behenate and talc.

Examples of diluents include one or more of: water, ethanol, propylene glycol and glycerin, and combinations thereof.

The other components may be used simultaneously (e.g. when they are in admixture together or even when they are delivered by different routes) or sequentially (e.g. they may be delivered by different routes).

Preferably, when the feed additive composition of the present invention is admixed with another component(s), the DFM remains viable.

In one embodiment preferably the feed additive composition according to the present invention does not comprise chromium or organic chromium

In one embodiment preferably the feed additive according to the present invention does not contain glucanase.

In one embodiment preferably the feed additive according to the present invention does not contain sorbic acid.

### Concentrates

The DFMs for use in the present invention may be in the form of concentrates. Typically these concentrates comprise a substantially high concentration of a DFM.

Feed additive compositions according to the present invention may have a content of viable cells (colony forming units, CFUs) which is in the range of at least 10⁴ CFU/g (suitably including at least 10⁵ CFU/g, such as at least 10⁶ CFU/g, e.g. at least 10⁷ CFU/g, at least 10⁸ CFU/g) to about 10¹⁰ CFU/g (or even about 10¹¹ CFU/g or about 10¹² CFU/g).

When the DFM is in the form of a concentrate the feed additive compositions according to the present invention may have a content of viable cells in the range of at least 10⁹ CFU/g to about 10¹² CFU/g, preferably at least 10¹⁰ CFU/g to about 10¹² CFU/g.

Powders, granules and liquid compositions in the form of concentrates may be diluted with water or resuspended in water or other suitable diluents, for example, an appropriate growth medium such as milk or mineral or vegetable oils, to give compositions ready for use.

The DFM or feed additive composition of the present invention or the combinations of the present invention in the form of concentrates may be prepared according to methods known in the art.

In one aspect of the present invention the enzymes or feed is contacted by a composition in a concentrated form.

The compositions of the present invention may be spray-dried or freeze-dried by methods known in the art.

Typical processes for making particles using a spray drying process involve a solid material which is dissolved in an appropriate solvent (e.g. a culture of a DFM in a fermentation medium). Alternatively, the material can be suspended or emulsified in a non-solvent to form a suspension or emulsion. Other ingredients (as discussed above) or components such as anti-microbial agents, stabilising agents, dyes and agents assisting with the drying process may optionally be added at this stage.

The solution then is atomised to form a fine mist of droplets. The droplets immediately enter a drying chamber where they contact a drying gas. The solvent is evaporated from the droplets into the drying gas to solidify the droplets, thereby forming particles. The particles are then separated from the drying gas and collected.

### Subject

The term "subject", as used herein, means an animal that is to be or has been administered with a feed additive composition according to the present invention or a feedstuff comprising said feed additive composition according to the present invention.

According to the invention, the "subject" animal is poultry (including broilers, chickens and turkeys).

In one embodiment the term poultry and/or chickens does not include egg layers.

In one embodiment the subject may be challenged by an enteric pathogen.

By way of example a subject may have one or more enteric pathogens present in its gut or digestive tract. For example a subject may have one or more enteric pathogens in its gut or digestive tract at a level which:
i) results in loss of performance of the animal and/or
ii) is at clinically relevant levels; or
iii) is at sub-clinical levels.

The enteric pathogen may be *Clostridium perfringens* for example.

### Feed Conversion Ratio (FCR)

As used herein, the term "feed conversion ratio" refers to the amount of feed fed to an animal to increase the weight of the animal by a specified amount.

An improved feed conversion ratio means a lower feed conversion ratio.

By "lower feed conversion ratio" or "improved feed conversion ratio" it is meant that the use of a feed additive composition in feed results in a lower amount of feed being required to be fed to an animal to increase the weight of the animal by a specified amount compared to the amount of feed required to increase the weight of the animal by the same amount when the feed does not comprise said feed additive composition.

Energy digestibility as used herein means the gross energy of the feed consumed minus the gross energy of the faeces or the gross energy of the feed consumed minus the gross energy of the remaining digesta on a specified segment of the gastro-intestinal tract of the animal, e.g. the ileum. Metabolizable energy as used herein refers to apparent metabolizable energy and means the gross energy of the feed consumed minus the gross energy contained in the faeces, urine, and gaseous products of digestion. Energy digestibility and metabolizable energy may be measured as the difference between the intake of gross energy and the gross energy excreted in the faeces or the digesta present in specified segment of the gastro-intestinal tract using the same methods to measure the digestibility of nutrients, with appropriate corrections for nitrogen excretion to calculate metabolizable energy of feed.

### Nitrogen retention

Nitrogen retention as used herein means a subject's ability to retain nitrogen from the diet as body mass. A negative nitrogen balance occurs when the excretion of nitrogen exceeds the daily intake and is often seen when the muscle is being lost. A positive nitrogen balance is often associated with muscle growth, particularly in growing animals.

Nitrogen retention may be measured as the difference between the intake of nitrogen and the excreted nitrogen by means of the total collection of excreta and urine during a period of time. It is understood that excreted nitrogen includes undigested protein from the feed, endogenous proteinaceous secretions, microbial protein, and urinary nitrogen.

### Survival

The term survival as used herein means the number of subject remaining alive. The term "improved survival" may be another way of saying "reduced mortality".

### Carcass yield and meat yield

The term carcass yield as used herein means the amount of carcass as a proportion of the live body weight, after a commercial or experimental process of slaughter. The term carcass means the body of an animal that has been slaughtered for food, with the head, entrails, part of the limbs, and feathers or skin removed. The term meat yield as used herein means the amount of edible meat as a proportion of the live body weight, or the amount of a specified meat cut as a proportion of the live body weight.

### Weight gain

The present invention further provides a method of increasing weight gain in a subject, e.g. poultry or swine, comprising feeding said subject a feedstuff comprising a feed additive composition according to the present invention.

An "increased weight gain" refers to an animal having increased body weight on being fed feed comprising a feed additive composition compared with an animal being fed a feed without said feed additive composition being present.

### Necrotic enteritis

Necrotic enteritis is an acute or chronic enterotoxemia seen in chickens, turkeys and ducks worldwide, caused by *Clostridium perfringens.* Necrotic enteritis is often characterised by a fibrino-necrotic enteritis, usually of the mid- small intestine. Mortality may be 5-50%, usually around 10%. Infection occurs by faecal-oral transmission. Spores of the causative organism are highly resistant. Predisposing factors include coccidiosis/coccidiasis, diet (high protein), in ducks possibly heavy strains, high viscosity diets (often associated with high rye and wheat inclusions in the diet), contaminated feed and/or water, other debilitating diseases.

The present invention relates to increasing the subject's resistance to necrotic enteritis. In other words, the present invention relates to avoiding or reducing the negative effect of necrotic enteritis.

The term "resistance to" as used herein may encompass the term "tolerance of". Therefore in one embodiment the subject may not be resistant to necrotic enteritis but the subject may be able to tolerate the necrotic enteritis, i.e. without negative effects on performance of the subject.

In one embodiment the present invention relates to a feed additive composition according to the present invention for treating or preventing necrotic enteritis in a subject. Typically the subject will be one which has been or will be challenged with *Clostridium perfringens* and/or *Eimeria* species. Such challenge may come from the environment or the application of live microorganisms in the feed or drinking water, e.g. when live coccidia vaccines are used.

In another embodiment the present invention relates to a feed additive composition for preventing and/or treating coccidiosis in a subject.

The present invention yet further provides a method of preventing and/or treating necrotic enteritis and/or coccidiosis wherein an effective amount of a feed additive composition according to the present invention is administered to a subject.

### Pathogenic bacteria

The term pathogenic bacteria as used herein means for example toxigenic clostridia species, e.g. *Clostridium perfringens* and/or *E. coli* and/or *Salmonella* spp and/or *Campylobacter* spp. In one embodiment the pathogenic bacteria may be Avian pathogenic *E.* coli species.

### Nutrient Exretion

In one embodment the present invention relates to reducing nutrient excretion in manure. This has positive effects on reducing environmental hazards. For example, in a preferred embodiment the present invention relates to reducing nitrogen and/or phosphorus content in the subject's manure. This, therefore, reduces the amount of nitrogen and/or phosphorus in the environment, which can be beneficial.

### Probiotic

For some applications, it is believed that the DFM in the composition of the present invention can exert a probiotic culture effect. It is also within the scope of the present invention to add to the composition of the present invention further probiotic and/or prebiotics.

Here, a prebiotic is:
*"a non-digestible food ingredient that beneficially affects the host by selectively stimulating the growth and*/*or the activity of one or a limited number of beneficial bacteria".*

The term "probiotic culture" as used herein defines live microorganisms (including bacteria or yeasts for example) which, when for example ingested or locally applied in sufficient numbers, beneficially affects the host organism, i.e. by conferring one or more demonstrable health benefits on the host organism. Probiotics may improve the microbial balance in one or more mucosal surfaces. For example, the mucosal surface may be the intestine, the urinary tract, the respiratory tract or the skin. The term "probiotic" as used herein also encompasses live microorganisms that can stimulate the beneficial branches of the immune system and at the same time decrease the inflammatory reactions in a mucosal surface, for example the gut.

Whilst there are no lower or upper limits for probiotic intake, it has been suggested that at least 10⁶-10¹², preferably at least 10⁶-10¹⁰, preferably 10⁸-10⁹, cfu as a daily dose will be effective to achieve the beneficial health effects in a subject.

The term "good bacteria" as used herein means bacteria which are commensal non-pathogenic bacteria in the gut. "Good bacteria" include Lactobacillus spp, e.g. Lactobacillus acidophilus and and bifidobacterium. Good bacteria may prevent disease by making an unfavorable environment for less desirable bacteria.

### Isolated

In one aspect, suitably the enzyme or DFM used in the present invention may be in an isolated form. The term "isolated" means that the enzyme or DFM is at least substantially free from at least one other component with which the enzyme or DFM is naturally associated in nature and as found in nature. The enzyme or DFM of the present invention may be provided in a form that is substantially free of one or more contaminants with which the substance might otherwise be associated. Thus, for example it may be substantially free of one or more potentially contaminating polypeptides and/or nucleic acid molecules.

### Purified

In one aspect, preferably the enzyme and/or DFM according to the present invention is in a purified form. The term "purified" means that the enzyme and/or DFM is present at a high level. The enzyme and/or DFM is desirably the predominant component present in a composition. Preferably, it is present at a level of at least about 90%, or at least about 95% or at least about 98%, said level being determined on a dry weight/dry weight basis with respect to the total composition under consideration.

It is envisaged within the scope of the present invention that the embodiments of the invention can be combined such that combinations of any of the features described herein are included within the scope of the present invention. In particular, it is envisaged within the scope of the present invention that any of the therapeutic effects of the bacteria may be exhibited concomitantly.

### Nucleotide sequence

The scope of the present invention encompasses nucleotide sequences encoding proteins having the specific properties as defined herein.

The term "nucleotide sequence" as used herein refers to an oligonucleotide sequence or polynucleotide sequence, and variant, homologues, fragments and derivatives thereof (such as portions thereof). The nucleotide sequence may be of genomic or synthetic or recombinant origin, which may be double-stranded or single-stranded whether representing the sense or anti-sense strand.

The term "nucleotide sequence" in relation to the present invention includes genomic DNA, cDNA, synthetic DNA, and RNA. Preferably it means DNA, more preferably cDNA sequence coding for the present invention.

In a preferred embodiment, the nucleotide sequence when relating to and when encompassed by the *per se* scope of the present invention does not include the native nucleotide sequence according to the present invention when in its natural environment and when it is linked to its naturally associated sequence(s) that is/are also in its/their natural environment. For ease of reference, we shall call this preferred embodiment the "non-native nucleotide sequence". In this regard, the term "native nucleotide sequence" means an entire nucleotide sequence that is in its native environment and when operatively linked to an entire promoter with which it is naturally associated, which promoter is also in its native environment. However, the amino acid sequence encompassed by the scope of the present invention can be isolated and/or purified post expression of a nucleotide sequence in its native organism. Preferably, however, the amino acid sequence encompassed by scope of the present invention may be expressed by a nucleotide sequence in its native organism but wherein the nucleotide sequence is not under the control of the promoter with which it is naturally associated within that organism.

Typically, the nucleotide sequence encompassed by the scope of the present invention is prepared using recombinant DNA techniques. (i.e. recombinant DNA). However, in an alternative embodiment of the invention, the nucleotide sequence could be synthesised, in whole or in part, using chemical methods well known in the art (see Caruthers MH et al., (1980) Nuc Acids Res Symp Ser 215-23 and Horn T et al., (1980) Nuc Acids Res Symp Ser 225-232).

### Preparation of the nucleotide sequence

A nucleotide sequence encoding either a protein which has the specific properties as defined herein or a protein which is suitable for modification may be identified and/or isolated and/or purified from any cell or organism producing said protein. Various methods are well known within the art for the identification and/or isolation and/or purification of nucleotide sequences. By way of example, PCR amplification techniques to prepare more of a sequence may be used once a suitable sequence has been identified and/or isolated and/or purified.

By way of further example, a genomic DNA and/or cDNA library may be constructed using chromosomal DNA or messenger RNA from the organism producing the enzyme. If the amino acid sequence of the enzyme is known, labelled oligonucleotide probes may be synthesised and used to identify enzyme-encoding clones from the genomic library prepared from the organism. Alternatively, a labelled oligonucleotide probe containing sequences homologous to another known enzyme gene could be used to identify enzyme-encoding clones. In the latter case, hybridisation and washing conditions of lower stringency are used.

Alternatively, enzyme-encoding clones could be identified by inserting fragments of genomic DNA into an expression vector, such as a plasmid, transforming enzyme-negative bacteria with the resulting genomic DNA library, and then plating the transformed bacteria onto agar plates containing a substrate for enzyme (i.e. maltose), thereby allowing clones expressing the enzyme to be identified.

In a yet further alternative, the nucleotide sequence encoding the enzyme may be prepared synthetically by established standard methods, e.g. the phosphoroamidite method described by Beucage S.L. et al., (1981) Tetrahedron Letters 22, p 1859-1869, or the method described by Matthes et al., (1984) EMBO J. 3, p 801-805. In the phosphoroamidite method, oligonucleotides are synthesised, e.g. in an automatic DNA synthesiser, purified, annealed, ligated and cloned in appropriate vectors.

The nucleotide sequence may be of mixed genomic and synthetic origin, mixed synthetic and cDNA origin, or mixed genomic and cDNA origin, prepared by ligating fragments of synthetic, genomic or cDNA origin (as appropriate) in accordance with standard techniques. Each ligated fragment corresponds to various parts of the entire nucleotide sequence. The DNA sequence may also be prepared by polymerase chain reaction (PCR) using specific primers, for instance as described in US 4,683,202 or in Saiki R K et al., (Science (1988) 239, pp 487-491).

### Amino acid sequences

The scope of the present invention also encompasses amino acid sequences of enzymes having the specific properties as defined herein.

As used herein, the term "amino acid sequence" is synonymous with the term "polypeptide" and/or the term "protein". In some instances, the term "amino acid sequence" is synonymous with the term "peptide". In some instances, the term "amino acid sequence" is synonymous with the term "enzyme".

The amino acid sequence may be prepared/isolated from a suitable source, or it may be made synthetically or it may be prepared by use of recombinant DNA techniques.

The protein encompassed in the present invention may be used in conjunction with other proteins, particularly enzymes. Thus the present invention also covers a combination of proteins wherein the combination comprises the protein/enzyme of the present invention and another protein/enzyme, which may be another protein/enzyme according to the present invention.

Preferably the amino acid sequence when relating to and when encompassed by the *per se* scope of the present invention is not a native enzyme. In this regard, the term "native enzyme" means an entire enzyme that is in its native environment and when it has been expressed by its native nucleotide sequence.

### Sequence Identity or Sequence Homology

The present invention also encompasses the use of sequences having a degree of sequence identity or sequence homology with amino acid sequence(s) of a polypeptide having the specific properties defined herein or of any nucleotide sequence encoding such a polypeptide (hereinafter referred to as a "homologous sequence(s)"). Here, the term "homologue" means an entity having a certain homology with the subject amino acid sequences and the subject nucleotide sequences. Here, the term "homology" can be equated with "identity".

The homologous amino acid sequence and/or nucleotide sequence should provide and/or encode a polypeptide which retains the functional activity and/or enhances the activity of the enzyme.

In the present context, a homologous sequence is taken to include an amino acid sequence which may be at least 75, 85 or 90% identical, preferably at least 95 or 98% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, a homologous sequence is taken to include a nucleotide sequence which may be at least 75, 85 or 90% identical, preferably at least 95 or 98% identical to a nucleotide sequence encoding a polypeptide of the present invention (the subject sequence). Typically, the homologues will comprise the same sequences that code for the active sites etc. as the subject sequence. Although homology can also be considered in terms of similarity (i.e. amino acid residues having similar chemical properties/functions), in the context of the present invention it is preferred to express homology in terms of sequence identity.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons.

Calculation of maximum % homology therefore firstly requires the production of an optimal alignment, taking into consideration gap penalties. A suitable computer program for carrying out such an alignment is the Vector NTI (Invitrogen Corp.). Examples of software that can perform sequence comparisons include, but are not limited to, the BLAST package (see Ausubel et al 1999 Short Protocols in Molecular Biology, 4th Ed - Chapter 18), BLAST 2 (see FEMS Microbiol Lett 1999 174(2): 247-50; FEMS Microbiol Lett 1999 177(1): 187-8 and tatiana@ncbi.nlm.nih.gov). FASTA (Altschul et al 1990 J. Mol. Biol. 403-410) and AlignX for example. At least BLAST, BLAST 2 and FASTA are available for offline and online searching (see Ausubel et al 1999, pages 7-58 to 7-60).

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. Vector NTI programs generally use either the public default values or a custom symbol comparison table if supplied (see user manual for further details). For some applications, it is preferred to use the default values for the Vector NTI package.

Alternatively, percentage homologies may be calculated using the multiple alignment feature in Vector NTI (Invitrogen Corp.), based on an algorithm, analogous to CLUSTAL (Higgins DG & Sharp PM (1988), Gene 73(1), 237-244).

Once the software has produced an optimal alignment, it is possible to calculate % homology, preferably % sequence identity. The software typically does this as part of the sequence comparison and generates a numerical result.

Should Gap Penalties be used when determining sequence identity, then preferably the following parameters are used for pairwise alignment:

| FOR BLAST | |
|---|---|
| GAP OPEN | 0 |
| GAP EXTENSION | 0 |

| FOR CLUSTAL | DNA | PROTEIN | |
|---|---|---|---|
| WORD SIZE | 2 | 1 | K triple |
| GAP PENALTY | 15 | 10 | |
| GAP EXTENSION | 6.66 | 0.1 | |

In one embodiment, CLUSTAL may be used with the gap penalty and gap extension set as defined above.

Suitably, the degree of identity with regard to a nucleotide sequence is determined over at least 20 contiguous nucleotides, preferably over at least 30 contiguous nucleotides, preferably over at least 40 contiguous nucleotides, preferably over at least 50 contiguous nucleotides, preferably over at least 60 contiguous nucleotides, preferably over at least 100 contiguous nucleotides.

Suitably, the degree of identity with regard to a nucleotide sequence may be determined over the whole sequence.

### Hybridisation

The present invention also encompasses sequences that are complementary to the nucleic acid sequences of the present invention or sequences that are capable of hybridising either to the sequences of the present invention or to sequences that are complementary thereto.

The term "hybridisation" as used herein shall include "the process by which a strand of nucleic acid joins with a complementary strand through base pairing" as well as the process of amplification as carried out in polymerase chain reaction (PCR) technologies.

The present invention also encompasses the use of nucleotide sequences that are capable of hybridising to the sequences that are complementary to the sequences presented herein, or any derivative, fragment or derivative thereof.

The term "variant" also encompasses sequences that are complementary to sequences that are capable of hybridising to the nucleotide sequences presented herein.

Preferably, complementary sequences are those capable of hybridising under stringent conditions (e.g. 50°C and 0.2xSSC {1xSSC = 0.15 M NaCl, 0.015 M Na₃citrate pH 7.0}) to the nucleotide sequences presented herein.

More preferably, complementary sequences are those that are capable of hybridising under high stringency conditions (e.g. 65°C and 0.1xSSC {1xSSC = 0.15 M NaCl, 0.015 M Na₃citrate pH 7.0}) to the nucleotide sequences presented herein.

In a more preferred aspect, the present invention covers nucleotide sequences that can hybridise to the nucleotide sequence of the present invention, or the complement thereof, under high stringent conditions (e.g. 65°C and 0.1xSSC).

### EXAMPLES

### Example 1

### Methods

Four thousand one-day-old Cobb male chicks are purchased from a commercial hatchery. At study initiation, fifty males are allocated to each treatment pen by blocks, for a total of 9 pens per treatment. The study consists of the following treatments (Table 1):

**Table 1. Experimental design of Example 1.**

| Treatment | Clostridium perfringens Challenge | Phytase | DFM |
|---|---|---|---|
| 1 | No | None | None |
| 2 | Yes | None | None |
| 3 | Yes | None | Bacillus DFM¹ |
| 4 | Yes | None | Lactobacillus DFM² |
| 5 | Yes | Ronozyme P®³ 500 FTU/kg | None |
| 6 | Yes | Citrobacter phytase A⁴ 500 FTU/kg | None |
| 7 | Yes | Citrobacter phytase B⁵ 500 FTU/kg | None |
| 8 | Yes | Ronozyme P® 500 FTU/kg | Bacillus DFM |
| 9 | Yes | Citrobacter phytase A 500 FTU/kg | Bacillus DFM |
| 10 | Yes | Citrobacter phytase B 500 FTU/kg | Bacillus DFM |
| 11 | Yes | Ronozyme P® 500 FTU/kg | Lactobacillus DFM |
| 12 | Yes | Citrobacter phytase A 500 FTU/kg | Lactobacillus DFM |
| 13 | Yes | Citrobacter phytase B 500 FTU/kg | Lactobacillus DFM |

| | | | |
|---|---|---|---|
| ¹ "Bacillus DFM" - is Enviva Pro ® which is a combination of Bacillus subtilis strains Bs2084, LSSAO1 and 15AP4, provided by Danisco A/S and is dosed at 150,000 CFU/g of feed. ² "Lactobacillus DFM" - is Sorbiflore® which is a combination of *Lactobacillus rhamnosus* and *Lactobacillus farciminis,* provided by Danisco Animal Nutrition and is dosed at 350,000 CFU/g of feed. ³ "Ronozyme P®" is a 6-phytase from *Peniphora lycii* expressed in *Aspergillus oryzae -* the amino acid sequence of which is given herein as SEQ ID No. 8. (This is a *"non-Citrobacter"* 6 phytase) ⁴ *Citrobacter* phytase A - is a 6-phytase from *Citrobacter braakii* strain ATCC 51113. The amino acid sequence of this enzyme is given herein as SEQ ID No. 1. ⁵ *Citrobacter* phytase B - is a 6-phytase from *Citrobacter braakii* strain YH-15. The amino acid sequence of this enzyme is given herein as SEQ ID No. 7. | | | |

Bird weights by pen are recorded at study initiation, 23 d, 35 d, and termination (42d). The pen is the unit of measure. Broiler diets are fed as mash feed. Diets meet or exceed NRC standards (Table 2). The mixer is flushed to prevent cross contamination of diets. All treatment feeds are mixed using a Davis S-20 mixer. Samples are collected from each treatment diet from the beginning, middle, and end of each batch and blended together to confirm enzyme activities and DFM presence in feed.

**Table 2. Experimental diet composition of Example 1.**

| Ingredient (%) | Starter (0 to 21 days) | Grower (21 to 35 days) | Finisher (35 to 42 days) |
|---|---|---|---|
| Maize | 53.62 | 57.87 | 59.82 |
| Maize DDGS | 10.00 | 10.00 | 10.00 |
| Soybean Meal 49%CP | 26.93 | 23.97 | 21.36 |
| Ampro 55 | 5.00 | 5.00 | 5.00 |
| Soy oil | 2.07 | 0.91 | 1.74 |
| Lysine | 0.24 | 0.24 | 0.24 |
| DL-methionine | 0.21 | 0.19 | 0.18 |
| L-threonine | 0.01 | 0.01 | 0.01 |
| Salt | 0.30 | 0.34 | 0.35 |
| Limestone | 1.04 | 1.07 | 0.94 |
| Dicalcium phosphate | 0.26 | 0.11 | 0.02 |
| Vitamin and trace mineral premix | 0.33 | 0.33 | 0.33 |

| Calculated Nutrient Composition (%) | | | |
|---|---|---|---|
| CP | 22.60 | 21.50 | 20.39 |
| Energy, kcal/kg | 3060 | 3025 | 3100 |
| Digestible lysine | 1.36 | 1.26 | 1.21 |
| Digestible methionine | 0.58 | 0.61 | 0.53 |
| Digestible threonine | 0.83 | 0.83 | 0.80 |

Birds receive feed *ad-libitum* appropriate to the treatment from day 0 to 42. Enzymes and DFMs are provided by Danisco in the appropriate mixtures and levels for all experimental treatments. The pens are arranged within the facility to prevent direct contact in order to avoid contamination. A change from starter to grower occurred on day 21. Grower diet is replaced with the finisher diet on day 35. At each feed change, feeders are removed from pens by block, weighed back, emptied, and refilled with the appropriate treatment diet. On the final day of the study feed is weighed. Pens are checked daily for mortality. When a bird is culled or found dead, the date and removal weight (kg) are recorded. A gross necropsy is performed on all dead or culled birds to determine the sex and probable cause of death. Signs of Necrotic Enteritis are noted.

All pens have approximately 4 inches of built up litter with a coating of fresh pine shavings. All birds are spray vaccinated prior to placement into pens with a commercial coccidiosis vaccine (Coccivac-B). On days 18, 19, and 20 all birds, except Treatment 1, are dosed with a broth culture of C. perfringens. A field isolate of C. perfringens known to cause NE and originating from a commercial broiler operation is utilized as the challenge organism. Fresh inoculum is used each day. The titration levels are approximately 1.0 X 10⁸⁻⁹. Each pen receives the same amount of inoculum. The inoculum is administered by mixing into the feed found in the base of the tube feeder. On day 21, five birds from each pen are selected, euthanized, group weighed, and examined for the degree of presence of Necrotic Enteritis lesions. The scoring is based on a 0 to 3 score, with 0 being normal and 3 being the most severe (0 = none, 1 = mild, 2 = moderate, 3 = marked/severe; Hofacre et al., 2003 J. Appl. Poult. Res. 12:60-64). No concomitant drug therapy is used during the study. The pH of jejunal digesta is measured on fresh samples (0.5 g) diluted with 5 mL of deionized water and using a combined glass-reference microelectrode.

### Sample Collection

On day 21, a total of 8 birds per treatment are euthanised and the total gastrointestinal tract from below the gizzard to the ileal-cecal junction is collected from each bird and sent overnight on ice to the laboratory. The samples are further dissected in the laboratory to obtain a 20 cm portion of the jejunum surrounding the Meckle's diverticulum; the remainders of the intestinal tract is discarded. The sections are rinsed with 0.1% peptone to remove the intestinal contents and opened longitudinally to expose the epithelial lining. The sections are masticated in 99 ml of 0.1% peptone at 7.0 strokes/s for 60 s to release mucosa-associated bacterial cells. Bacteria are harvested from the masticated solution by centrifugation at 12,000 x g for 10 minutes. The resultant bacterial pellet is resuspended in 10 ml of MRS broth + 10% glycerol, flash-frozen in liquid nitrogen, and stored at -20oC until further analysis.

### DNA isolation

Genomic DNA is isolated from all samples by phenol chloroform extraction and purified using Roche Applied Science High Pure PCR Template Purification Kit (Roche Diagnostics Corp., Indianapolis, IN).

### Pyrosequencing

Bacterial tag-encoded FLX amplicon pyrosequencing is performed as described by Dowd (Dowd et al. 2008; 8, 125). An equivalent amount of DNA isolated from the intestinal mucosa from each bird is analyzed. The V1-V3 region of the 16S rRNA gene is amplified in each sample using the primers 28 F (5'- GAGTTTGATCNTGGCTCAG) and 519R (5'-GTNTTACNGCGGCKGCTG). Following sequencing, raw data is screened and trimmed based on quality. Sequences are sorted by individual samples based on barcode sequences. Barcode tags are removed and non-bacteria ribosomal sequences are removed. The bacterial community composition is determined using BlastN comparison to a quality controlled and manually curated database derived from NCBI. The relative abundance of each bacterial ID is determined for each sample. Data is compiled at each taxonomic level using NCBI nomenclature.

### Statistical analysis

For performance data means are separated using pair wise t-tests. Significant differences are considered at P<0.05. Pens are used as the experimental unit. Proportions of bacterial species are calculated from count data and the results analysed using a categorical model analysis and then a Chi-square probability calculated using JMP 8.0.2 (SAS institute, Cary, NC), where each sample representing one bird is considered an experimental unit.

### Results and Discussion

Body weight gain and feed efficiency are significantly reduced by the *C. perfringens* challenge compared to the unchallenged control. Citrobacter phytases, with or without DFM significantly improve body weight gain and feed efficiency of broilers before the NE challenge, compared to the other treatments. In the overall experiment (0 to 42 days), including the challenge from 18 to 20 days, supplementation with a Citrobacter phytase, Ronozyme P, Bacillus DFM or Lactobacillus DFM slightly improves body weight gain and feed efficiency (from 0 to 42 days) compared to the challenged control, to a level between the challenged and unchallenged controls. The combination of Ronozyme P and the Bacillus DMF or Lactobacillus DFM does not significantly improve body weight gain or feed efficiency of broilers compared to Ronozyme P or the DFMs by themselves. In contrast, the combination of a *Citrobacter* phytase (either Citrobacter phytase A or B) and either of the DFMs improves body weight gain and feed efficiency of broilers to a level that is greater than the unchallenged control, indicating a synergistic effect of the *Citrobacter* phytases and a DFM product. This effect is more pronounced for Citrobacter phytase A compared with Citrobacter phytase B - indicating that unexpectedly an event better effect can be obtained using Citrobacter A in combination with a DFM compared with Citrobacter phytase B in combination with a DFM.

There is a reduction in mortality and lesion scores observed with the supplementation of either a Bacillus or a Lactobacillus DFM to a level near, but not equal to the unchallenged control. Ronozyme P reduces the mortality and lesion scores compared to the challenged control, although not to the level of the unchallenged control. Citrobacter phytases A and B do not significantly change the severity of lesion scores or the mortality due to NE compared to the challenged control. The combination of Ronozyme P and either Bacillus DFM or Lactobacillus DFM reduces the mortality due to NE and the lesion scores to the level of the unchallenged control. The combination of Citrobacter phytase and either Bacillus DFM or Lactobacillus DFM reduces the mortality due to NE and the lesion scores to a level that was slightly better (less mortality and lesion scores) compared to the unchallenged control. This effect is more pronounced for Citrobacter phytase A compared with Citrobacter phytase B - indicating that unexpectedly an event better effect can be obtained using Citrobacter A in combination with a DFM compared with Citrobacter phytase B in combination with a DFM.

Both DFMs reduce the pH of the jejunal digesta compared to the challenged control; Ronozyme P does not change the pH, and the Citrobacter phytases increase the pH. The combination of Ronozyme P or Citrobacter phytase A or Citrobacter phytase B, and Bacillus DFM or Lactobacillus DFM reduces the pH of jejunal digesta to a level that was lower compared to the challenged and unchallenged control treatments. The pH of the jejunal digesta is negatively correlated with the proportion of Lactobacillus spp. as a proportion of the total bacterial DNA in jejunal mucosa at 21 d. In turn, the relative proportion of Lactobacillus spp. is positively correlated with body weight gain and feed efficiency after the challenge (21 to 35 days).

There is a benefit of utilising Citrobacter phytases (particularly Citrobacter phytase A) in combination with a DFM, to prevent losses particularly during a *C. perfringens* challenge and to maximize the positive effects of this phytase in the digestibility of minerals, protein, and energy, which are translated on increased feed efficiency and body weight gain compared to the challenged and unchallenged controls. These benefits are also evident on the reduced intestinal damage and mortality due to NE with the combination compared to the phytase by itself and the challenged control treatment.

### Discussion

Again without wishing to be bound by theory one suggestion how the combination of *Citrobacter* phytases and DFMs provides surprisingly better results compared with other phytases and DFMs is that *Citrobacter braakii* phytases have a higher activity at a lower pH (e.g. 3.5-4.5) compared with some other non-*Citrobacter* phytases. As the first part of the gastrointestinal (GI) tract of monogastric farm animals, e.g. swine or poultry, has a low pH - *Citrobacter braakii* phytases appear to have more activity in this part of the GI tract thus these phytases are capable of releasing phosphorus and other nutrients, such as protein, much faster from the phytate substrate compared with some other non-*Citrobacter* phytases. This is advantageous in many ways, including that it is desirable to act on the phytate as soon as possible as it has a tendency to complex with other substances such as minerals, particularly as the pH rises. Once the phytate complexes it can be less accessible by enzymes for breakdown. Therefore acting on the phytate substrate early on in the GI tract when the pH is still low is desirable. However the breakdown of the phytate in the early part of the GI tract means that there can be less phosphorus available in the jejunum and the lower part of the GI tract which can have a negative impact on the populations of commensal "good" bacteria such as the *Lactobacilli* (which have been shown to have beneficial effects such as immune modulation and the production of organic acids that lower the intestinal pH). This negative impact on resident "good" bacteria can result in opportunistic pathogens to flourish - thus upsetting the overall balance of bacteria in the gut.

With regard to the present invention, it has surprisingly been found that the negative impact of using *Citrobacter braakii* phytases can be overcome by combining their use with one or more DFM. The DFM reestablishes the balance of bacteria in the gut - thus leading to reduced gut damage due to pathogenic bacteria and higher performance of the animal subject.

Again without wishing to be bound by theory a further suggestion how the combination of *Citrobacter* phytases and DFMs provides surprisingly better results compared with other phytases and DFMs is that *Citrobacter braakii* phytases have a higher activity at lower pHs (e.g. 3.5-4.5) compared with some other non-*Citrobacter* phytases. As the first part of the gastrointestinal (GI) tract has a low pH - *Citrobacter braakii* phytases appear to have more activity in this part of the GI tract. This can improve protein digestion by a subject because phytate can form complexes by binding proteins. The results of this early increase in adsorption of proteins can result in the animal producing less hydrochloric acid (HCl) - this can have a negative impact later in the GI tract as it can increase the pH in the later part of the GI tract. Increasing pH in the later part of the GI tract is not advantageous as it increases the chances of pathogens being able to establish themselves within the gut. Surprisingly it has been found by the present inventions that these negative effects of using *Citrobacter* phytases can be overcome by combining them with DFMs.

Surprisingly the 6-phytase from *Citrobacter braakii* strain ATCC 51113 (with the amino acid sequence SEQ ID No. 1) is even more positively influenced that even other *Citrobacter braakii* 6-phytase enzymes such as the 6-phytase from *Citrobacter braakii* strain YH-15 (with the amino acid sequence SEQ ID No. 7).

### Example 2 - Performance Study

### Methods

Four thousand one-day-old Cobb male chicks are purchased from a commercial hatchery. At study initiation, fifty males are allocated to each treatment pen by blocks, for a total of 8 pens per treatment. The study consists of the following treatments (Table 1):

**Table 1. Experimental design of Example 2.**

| Treatment | Phytase | DFM | Available P level | Ca Level |
|---|---|---|---|---|
| 1 PC | None | None | Basal | Basal |
| 2 NC | None | None | -0.17 | -0.16 |
| 3 | None | Bacillus DFM¹ | -0.17 | -0.16 |
| 4 | None | Lactobacillus DFM² | -0.17 | -0.16 |
| 5 | Ronozyme P®³ 500 FTU/kg | None | -0.17 | -0.16 |
| 6 | Citrobacter phytase A⁴ 500 FTU/kg | None | -0.17 | -0.16 |
| 7 | Citrobacter phytase B⁴ 500 FTU/kg | None | -0.17 | -0.16 |
| 8 | Ronozyme P®³ 500 FTU/kg | Bacillus DFM | -0.17 | -0.16 |
| 9 | Citrobacter phytase A 500 FTU/kg | Bacillus DFM | -0.17 | -0.16 |
| 10 | Citrobacter phytase B 500 FTU/kg | Bacillus DFM | -0.17 | -0.16 |
| 11 | Ronozyme P®³ 500 FTU/kg | Lactobacillus DFM | -0.17 | -0.16 |
| 12 | Citrobacter phytase A 500 FTU/kg | Lactobacillus DFM | -0.17 | -0.16 |
| 13 | Citrobacter phytase B 500 FTU/kg | Lactobacillus DFM | -0.17 | -0.16 |

| | | | | |
|---|---|---|---|---|
| ¹ "Bacillus DFM" - is Enviva Pro ® which is a combination of Bacillus subtilis strains Bs2084, LSSAO1 and 15AP4, provided by Danisco A/S and is dosed at 150,000 CFU/g of feed. ² "Lactobacillus DFM" - is Sorbiflore® which is a combination of *Lactobacillus rhamnosus* and *Lactobacillus farciminis,* provided by Danisco Animal Nutrition and is dosed at 350,000 CFU/g of feed. ³ "Ronozyme P®" is a 6-phytase from *Peniphora lycii* expressed in *Aspergillus oryzae -* the amino acid sequence of which is given herein as SEQ ID No. 8. (This is a *"non-Citrobacter"* 6 phytase) ⁴ *Citrobacter* phytase A - is a 6-phytase from *Citrobacter braakii* strain ATCC 51113. The amino acid sequence of this enzyme is given herein as SEQ ID No. 1. ⁵ *Citrobacter* phytase B - is a 6-phytase from *Citrobacter braakii* strain YH-15. The amino acid sequence of this enzyme is given herein as SEQ ID No. 7. | | | | |

**Table 2. Experimental diet composition of Example 2.**

| | **Starter** | | **Grower** | | **Finisher** | |
|---|---|---|---|---|---|---|
| | **PC** | **NC** | **PC** | **NC** | **PC** | **NC** |
| **MAIZE** | 59.54 | 62.20 | 60.10 | 63.30 | 61.24 | 64.50 |
| **SOYBEAN MEAL** | 34.35 | 33.90 | 32.80 | 32.20 | 31.41 | 30.80 |
| **SODIUM BENTONITE** | - | 0.28 | - | -- | - | -- |
| **DICALCIUM PHOSPHATE** | 2.14 | 1.17 | 1.87 | 0.91 | 1.72 | 0.76 |
| **LIMESTONE-FINE** | 1.162 | 1.32 | 0.94 | 1.10 | 0.91 | 1.08 |
| **SALT-FINE** | 0.38 | 0.38 | 0.38 | 0.38 | 0.38 | 0.38 |
| **L-LYSINE HCL** | 0.34 | 0.34 | 0.16 | 0.16 | 0.015 | 0.01 |
| **DL-METHIONINE** | 0.17 | 0.16 | 0.12 | 0.11 | 0.09 | 0.08 |
| **THREONINE** | 0.09 | 0.07 | 0.01 | -- | - | -- |
| **VIT-MIN PREMIX** | 0.20 | 0.20 | 0.20 | 0.20 | 0.20 | 0.20 |
| **SOY OIL** | 1.62 | -- | 3.46 | 1.66 | 4.03 | 2.22 |
| | | | | | | |
| **CRUDE PROTEIN** | 22.00 | 22.00 | 21.00 | 21.00 | 20.34 | 20.3 |
| **AME POULTRY (MJ/KG)** | 12.6 | 12.4 | 13.2 | 12.9 | 13.4 | 13.1 |
| **LYSINE-AVL** | 1.31 | 1.30 | 1.12 | 1.11 | 0.99 | 0.97 |
| **METHIONINE** | 0.51 | 0.50 | 0.45 | 0.44 | 0.41 | 0.40 |
| **THREONINE** | 0.94 | 0.92 | 0.83 | 0.82 | 0.80 | 0.80 |
| **CALCIUM** | 1.05 | 0.89 | 0.9 | 0.74 | 0.85 | 0.69 |
| **PHOSPHORUS (AVL)** | 0.50 | 0.33 | 0.45 | 0.28 | 0.42 | 0.25 |
| **PHOSPHORUS (TOT)** | 0.77 | 0.60 | 0.72 | 0.54 | 0.68 | 0.51 |

Birds receive feed *ad-libitum* appropriate to the treatment from day 0 to 42. Enzymes and DFMs are provided by Danisco in the appropriate mixtures and levels for all experimental treatments. The pens are arranged within the facility to prevent direct contact in order to avoid cross contamination of diets. Starter diet is replaced with the grower diet on day 21, and grower diet is replaced with the finisher diet on day 35. At each feed change, feeders are removed from pens by block, weighed back, emptied, and refilled with the appropriate treatment diet. On the final day of the study feed is weighed. Pens are checked daily for mortality. When a bird is culled or found dead, the date and removal weight (kg) are recorded.

All pens had approximately 4 inches of built up litter with a coating of fresh pine shavings. All birds are spray vaccinated prior to placement into pens with a commercial coccidiosis vaccine (Coccivac-B). No concomitant drug therapy is used during the study.

Means are separated using pair wise t-tests. Significant differences are considered at P<0.05. Pens are used as the experimental unit.

### Results and Discussion

Body weight gain is significantly reduced by the negative control treatment at 42 days. There is no significant improvement in performance when either of the DFMs are supplemented. Contrary to this, supplementation of the Citrobacter phytases to the diets results in significant improvements in body weight gain, to a level similar to the positive control. Ronozyme P however, does not lead to a significant improvement against the negative control. When Ronozyme P is supplemented in combination with Bacillus DMF or Lactobacillus DFM there is a numerical improvement in body weight gain at 42 days, compared to the negative control. The combination of Citrobacter phytase and Bacillus DFM or Lactobacillus DMF demonstrates a significant improvement compared to the positive control diet, showing an enhanced effect of the Citrobacter phytase and the DFMs.

Similar observations are observed when the feed conversion ratio (FCR) is analysed. There is no significant effect of supplementing either of the probiotics on feed conversion efficiency, only a slight numerical increase. There are significant effects of the Citrobacter phytases but not Ronozyme P, when phytase is supplemented alone. Similarly, when a combination of either lactobacillus or bacillus and Ronozyme P are supplemented there is no significant improvement on top of the negative control. However, the combination of Citrobacter phytases and either Bacillus DFM or Lactobacillus DFM results in significant improvements in FCR to a level lower (improved feed efficiency) than the positive control, and lower (improved feed efficiency) than the added effect of either Lactobacillus DFM or Bacillus DFM, and phytase were supplemented alone. This effect is more pronounced for Citrobacter phytase A compared with Citrobacter phytase B - indicating that unexpectedly an event better effect can be obtained using Citrobacter A in combination with a DFM compared with Citrobacter phytase B in combination with a DFM.

In conclusion, there is a positive effect between the Citrobacter phytase and each of the DFMs - Bacillus and Lactobacillus. This is supported by increase in body weight gain and feed efficiency, greater than the sum of the effect of either supplement alone. This effect is more pronounced for Citrobacter phytase A compared with Citrobacter phytase B.

### Example 3 - Digestibility Study

### Methods

One thousand male Ross 308 broiler chickens are raised to 12 days in floor pens and are fed a typical commercial starter diet. On day 13, birds are allocated to produce 8 replicates per treatment, totalling 64 birds per treatment (8 birds per cage). From day 13 to 21, treatment diets are fed (Table 1).

**Table 1. Experimental design of Example 3.**

| Treatment | Phytase | DFM | P level | Ca Level |
|---|---|---|---|---|
| 1 PC | None | None | 0.684 | 0.9 |
| 2 NC | None | None | 0.474 | 0.73 |
| 3 | None | Bacillus DFM¹ | 0.474 | 0.73 |
| 4 | None | Lactobacillus DFM² | 0.474 | 0.73 |
| 5 | Ronozyme P®³ 500 FTU/kg | None | 0.474 | 0.73 |
| 6 | Citrobacter phytase A⁴ 500 FTU/kg | None | 0.474 | 0.73 |
| 7 | Citrobacter phytase B⁴ 500 FTU/kg | None | 0.474 | 0.73 |
| 8 | Ronozyme P®³ 500 FTU/kg | Bacillus DFM | 0.474 | 0.73 |
| 9 | Citrobacter phytase A 500 FTU/kg | Bacillus DFM | 0.474 | 0.73 |
| 10 | Citrobacter phytase B 500 FTU/kg | Bacillus DFM | 0.474 | 0.73 |
| 11 | Ronozyme P®³ 500 FTU/kg | Lactobacillus DFM | 0.474 | 0.73 |
| 12 | Citrobacter phytase A 500 FTU/kg | Lactobacillus DFM | 0.474 | 0.73 |
| 13 | Citrobacter phytase B 500 FTU/kg | Lactobacillus DFM | 0.474 | 0.73 |

| | | | | |
|---|---|---|---|---|
| ¹ "Bacillus DFM" - is Enviva Pro ® which is a combination of Bacillus subtilis strains Bs2084, LSSAO1 and 15AP4, provided by Danisco A/S and is dosed at 150,000 CFU/g of feed. ² "Lactobacillus DFM" - is Sorbiflore® which is a combination of *Lactobacillus rhamnosus* and *Lactobacillus farciminis,* provided by Danisco Animal Nutrition and is dosed at 350,000 CFU/g of feed. ³ "Ronozyme P®" is a 6-phytase from *Peniphora lycii* expressed in *Aspergillus oryzae -* the amino acid sequence of which is given herein as SEQ ID No. 8. (This is a *"non-Citrobacter" 6* phytase) ⁴ *Citrobacter* phytase A - is a 6-phytase from *Citrobacter braakii* strain ATCC 51113. The amino acid sequence of this enzyme is given herein as SEQ ID No. 1. ⁵ *Citrobacter* phytase B - is a 6-phytase from *Citrobacter braakii* strain YH-15. The amino acid sequence of this enzyme is given herein as SEQ ID No. 7. | | | | |

Broiler diets are fed as mash. Diets met or exceeded NRC standards (Table 2). The mixer is flushed to prevent cross contamination of diets. All treatment feeds are mixed using a Davis S-20 mixer. Samples are collected from each treatment diet from the beginning, middle, and end of each batch and blended together to confirm enzyme activities and DFM presence in feed.

**Table 2. Experimental diet composition of Example 3.**

| Ingredient | Positive control | Negative control |
|---|---|---|
| Maize | 55.67 | 57.06 |
| Soybean meal, 48% | 37.60 | 37.35 |
| Soybean oil | 2.56 | 2.13 |
| L-Lysine HCl | 0.16 | 0.16 |
| DL-methionine | 0.28 | 0.28 |
| L-threonine | 0.07 | 0.07 |
| Salt | 0.38 | 0.38 |
| Limestone | 0.81 | 0.81 |
| Dicalcium phosphate | 1.87 | 1.17 |
| Vitamin/trace mineral premix1 | 0.30 | 0.30 |
| Titanium oxide | 0.30 | 0.30 |
| | | |
| Calculated analysis | | |
| Dry matter | 88.9 | 88.8 |
| Crude protein | 23.0 | 23.0 |
| ME (MJ/kg) | 12.7 | 12.7 |
| ME (kcal/kg) | 3025 | 3025 |
| Calcium | 0.90 | 0.73 |
| Available P | 0.45 | 0.33 |
| Sodium | 0.18 | 0.18 |
| Dig. lysine | 1.21 | 1.21 |
| Dig. methionine | 0.60 | 0.60 |
| Dig. methionine + cystine | 0.86 | 0.86 |
| Dig. threonine | 0.76 | 0.76 |
| Dig. tryptophan | 0.22 | 0.22 |

Birds receive feed *ad-libitum* appropriate to the treatment from day 0 to 21, the entire duration of the study. No concomitant drug therapy is used during the study. Enzymes and DFMs are provided by Danisco in the appropriate mixtures and levels for all experimental treatments. The cages are physically divided to prevent direct contact in order to avoid contamination. On the final day of the study, the birds, as well as the feed in the feeders are weighed.

Faecal samples are collected on days 18, 19 and 20, weighed and recorded, and then frozen at -20°C on the day of collection. Excreta are subsequently pooled within each treatment group, mixed with a blender and two samples are taken. The samples are freeze dried and ground to pass through a 0.5mm sieve re-frozen and stored until being analysed for; dry matter (DM), gross energy (GE), nitrogen (N), phosphorus (P) and calcium (Ca).

On Day 21, all birds are euthanized and contents of the lower half of the ileum are obtained by flushing with distilled water. Digesta from birds within each cage are pooled and frozen immediately after collection. Duplicate proximate analyses of diets, ileal digesta and excreta are performed for DM, N, amino acids, GE, Ca, P, and an inert marker.

DM is determined by drying all samples at 100°C for 24h. High performance liquid chromatography (HPLC) is carried out on diets and ileal digesta to determine the relative amino acid concentrations. Phosphorus and calcium levels are determined by inductively coupled plasma atomic emission spectroscopy and energy determined using adiabatic bomb calorimeter (Model 1261, Parr Instrument Co., Moline, IL). Finally, N content is measured by the combustion method (Model FP2000, LECO Corp., St. Joseph, MI). Digestibility coefficients are calculated for both faecal and ileal digestibility.
Means are separated using pair wise t-tests. Significant differences are considered at P<0.05, using each cage as an experimental unit.

### Results and Discussion

There is no significant effect on ileal phosphorus digestibility, compared to the negative control, when Bacillus DFM or Lactobacillus DFM is supplemented alone. However, supplementation with Citrobacter phytase increases ileal phosphorus digestibility to a level equal to the positive control. There is a significant increase in ileal phosphorus digestibility when Ronozyme P is added to the diets alone, to a lever lower than the positive control diet.

When the combination of Citrobacter phytase and DFMs are added to the diets, ileal phosphorus digestibility increases compared to Citrobacter phytase supplemented alone. In contrast, when the Ronozyme P and DFM combinations are added to the diet, the ileal phosphorus digestibility coefficients are similar to those observed when Ronozyme P was supplemented alone. These trends are reflected in total tract digestibility of phosphorus.

There is a significant increase in ileal amino acid digestibility when the Citrobacter phytase is supplemented to the diet, compared to the negative control. When Ronozyme P is supplemented there is a slight increase in ileal amino acid digestibility compared to then negative control. When the Bacillus DFM or Lactobacillus DFM is supplemented alone there is only a slight increase on ileal amino acid digestibility. There is a significant effect of supplementing a combination of Citrobacter phytase and Bacillus DFM or Lactobacillus DFM on amino acid digestibility, compared to supplementation of the DFM or Citrobacter phytase alone, which is greater than the sum of the increases when either was supplemented alone.

This incremental effect is not observed when the RonozymeP + DFM combination is added to diets. This effect is more pronounced for Citrobacter phytase A compared with Citrobacter phytase B - indicating that unexpectedly an event better effect can be obtained using Citrobacter A in combination with a DFM compared with Citrobacter phytase B in combination with a DFM.

Energy digestibility (AME) is significantly increased from the level of the negative control when Citrobacter phytase is supplemented to the diets. This is not the case when Ronozyme P is added to the diets; where only a slight numerical increase in AME is observed. When either Bacillus DFM or Lactobacillus DFM is supplemented to the diets there is a very slight increase in AME, however it is not significantly different from the negative control. When either of the DFMs are supplemented in combination with the Citrobacter phytase, there is a significant increase in AME greater than the sum of the increase from the phytase or either of the DFMs alone. This was not the case when either of the DFMs was supplemented with the Ronozyme P phytase.

In conclusion, there is a significant effect between the Citrobacter phytase and the Bacillus DFM or Lactobacillus DFM. This effect is more pronounced for Citrobacter phytase A compared with Citrobacter phytase B - indicating that unexpectedly an event better effect can be obtained using Citrobacter A in combination with a DFM compared with Citrobacter phytase B in combination with a DFM. This is reflected in increases in P, AME, and ileal amino acid digestibility. The increases in digestibility coefficients observed when combinations of products are supplemented are greater than the sum of the improvements when either are supplemented alone.

### SEQUENCE LISTING

<110> DuPont Nutrition Biosciences ApS
<120> Feed Additive Composition
<130> P043063EPJ
<140> 127283034
   <141> 2012-01-19
<150> GB1102865.1
   <151> 2011-02-18
<150> GB1102857.8
   <151> 2011-02-18
<160> 13
<170> PatentIn version 3.5
<210> 1
   <211> 433
   <212> PRT
   <213> Citrobacter braakii
<220>
   <221> misc_feature
   <222> (40)..(40)
   <223> Xaa can be any naturally occurring amino acid
<220>
   <221> misc_feature
   <222> (345)..(345)
   <223> Xaa can be any naturally occurring amino acid
<400> 1
   Met Ser Thr Phe Ile Ile Arg Leu Leu Phe Phe Ser Leu Leu Cys Gly
   1               5                   10                  15
   Ser Phe Ser Ile His Ala Glu Glu Gln Asn Gly Met Lys Leu Glu Arg
               20                  25                  30
   Val Val Ile Val Ser Arg His Xaa Val Arg Ala Pro Thr Lys Phe Thr
           35                  40                  45
   Pro Ile Met Lys Asn Val Thr Pro Asp Gln Trp Pro Gln Trp Asp Val
       50                  55                  60
   Pro Leu Gly Trp Leu Thr Pro Arg Gly Gly Glu Leu Val Ser Glu Leu
   65                  70                  75                  80
   Gly Gln Tyr Gln Arg Leu Trp Phe Thr Ser Lys Gly Leu Leu Asn Asn
                   85                  90                  95
   Gln Thr Cys Pro Ser Pro Gly Gln Val Ala Val Ile Ala Asp Thr Asp
               100                 105                 110
   Gln Arg Thr Arg Lys Thr Gly Glu Ala Phe Leu Ala Gly Leu Ala Pro
           115                 120                 125
   Lys Cys Gln Ile Gln Val His Tyr Gln Lys Asp Glu Glu Lys Asn Asp
       130                 135                 140
   Pro Leu Phe Asn Pro Val Lys Met Gly Lys Cys Ser Phe Asn Thr Leu
   145                 150                 155                 160
   Gln Val Lys Asn Ala Ile Leu Glu Arg Ala Gly Gly Asn Ile Glu Leu
                   165                 170                 175
   Tyr Thr Gln Arg Tyr Gln Ser Ser Phe Arg Thr Leu Glu Asn Val Leu
               180                 185                 190
   Asn Phe Ser Gln Ser Glu Thr Cys Lys Thr Thr Glu Lys Ser Thr Lys
           195                 200                 205
   Cys Thr Leu Pro Glu Ala Leu Pro Ser Glu Leu Lys Val Thr Pro Asp
       210                 215                 220
   Asn Val Ser Leu Pro Gly Ala Trp Ser Leu Ser Ser Thr Leu Thr Glu
   225                 230                 235                 240
   Ile Phe Leu Leu Gln Glu Ala Gln Gly Met Pro Gln Val Ala Trp Gly
                   245                 250                 255
   Arg Ile Thr Gly Glu Lys Glu Trp Arg Asp Leu Leu Ser Leu His Asn
               260                 265                 270
   Ala Gln Phe Asp Leu Leu Gln Arg Thr Pro Glu Val Ala Arg Ser Arg
           275                 280                 285
   Ala Thr Pro Leu Leu Asp Met Ile Asp Thr Ala Leu Leu Thr Asn Gly
       290                 295                 300
   Thr Thr Glu Asn Arg Tyr Gly Ile Lys Leu Pro Val Ser Leu Leu Phe
   305                 310                 315                 320
   Ile Ala Gly His Asp Thr Asn Leu Ala Asn Leu Ser Gly Ala Leu Asp
                   325                 330                 335
   Leu Asn Trp Ser Leu Pro Gly Gln Xaa Asp Asn Thr Pro Pro Gly Asp
               340                 345                 350
   Lys Leu Val Phe Glu Lys Trp Lys Arg Thr Ser Asp Asn Thr Asp Trp
   355 360 365
   Val Gln Val Ser Phe Val Tyr Gln Thr Leu Arg Asp Met Arg Asp Ile
   370 375 380
   Gln Pro Leu Ser Leu Glu Lys Pro Ala Gly Lys Val Asp Leu Lys Leu
   385 390 395 400
   Ile Ala Cys Glu Glu Lys Asn Ser Gln Gly Met Cys Ser Leu Lys Ser
   405 410 415
   Phe Ser Arg Leu Ile Lys Glu Ile Arg Val Pro Glu Cys Ala Val Thr
   420 425 430
   Glu
<210> 2
   <211> 433
   <212> PRT
   <213> Citrobacter braakii
<400> 2
   Met Ser Thr Phe Ile Ile Arg Leu Leu Phe Phe Ser Leu Leu Cys Gly
   1 5 10 15
   Ser Phe Ser Ile His Ala Glu Glu Gln Asn Gly Met Lys Leu Glu Arg
   20 25 30
   Val Val Ile Val Ser Arg His Gly Val Arg Ala Pro Thr Lys Phe Thr
   35 40 45
   Pro Ile Met Lys Asn Val Thr Pro Asp Gln Trp Pro Gln Trp Asp Val
   50 55 60
   Pro Leu Gly Trp Leu Thr Pro Arg Gly Gly Glu Leu Val Ser Glu Leu
   65 70 75 80
   Gly Gln Tyr Gln Arg Leu Trp Phe Thr Ser Lys Gly Leu Leu Asn Asn
   85 90 95
   Gln Thr Cys Pro Ser Pro Gly Gln Val Ala Val Ile Ala Asp Thr Asp
   100 105 110
   Gln Arg Thr Arg Lys Thr Gly Glu Ala Phe Leu Ala Gly Leu Ala Pro
   115 120 125
   Lys Cys Gln Ile Gln Val His Tyr Gln Lys Asp Glu Glu Lys Asn Asp
       130                 135                 140
   Pro Leu Phe Asn Pro Val Lys Met Gly Lys Cys Ser Phe Asn Thr Leu
   145                 150                 155                 160
   Gln Val Lys Asn Ala Ile Leu Glu Arg Ala Gly Gly Asn Ile Glu Leu
                   165                 170                 175
   Tyr Thr Gln Arg Tyr Gln Ser Ser Phe Arg Thr Leu Glu Asn Val Leu
               180                 185                 190
   Asn Phe Ser Gln Ser Glu Thr Cys Lys Thr Thr Glu Lys Ser Thr Lys
           195                 200                 205
   Cys Thr Leu Pro Glu Ala Leu Pro Ser Glu Leu Lys Val Thr Pro Asp
       210                 215                 220
   Asn Val Ser Leu Pro Gly Ala Trp Ser Leu Ser Ser Thr Leu Thr Glu
   225                 230                 235                 240
   Ile Phe Leu Leu Gln Glu Ala Gln Gly Met Pro Gln Val Ala Trp Gly
                   245                 250                 255
   Arg Ile Thr Gly Glu Lys Glu Trp Arg Asp Leu Leu Ser Leu His Asn
               260                 265                 270
   Ala Gln Phe Asp Leu Leu Gln Arg Thr Pro Glu Val Ala Arg Ser Arg
           275                 280                 285
   Ala Thr Pro Leu Leu Asp Met Ile Asp Thr Ala Leu Leu Thr Asn Gly
       290                 295                 300
   Thr Thr Glu Asn Arg Tyr Gly Ile Lys Leu Pro Val Ser Leu Leu Phe
   305                 310                 315                 320
   Ile Ala Gly His Asp Thr Asn Leu Ala Asn Leu Ser Gly Ala Leu Asp
                   325                 330                 335
   Leu Asn Trp Ser Leu Pro Gly Gln Pro Asp Asn Thr Pro Pro Gly Gly
               340                 345                 350
   Glu Leu Val Phe Glu Lys Trp Lys Arg Thr Ser Asp Asn Thr Asp Trp
           355                 360                 365
   Val Gln Val Ser Phe Val Tyr Gln Thr Leu Arg Asp Met Arg Asp Ile
       370                 375                 380
   Gln Pro Leu Ser Leu Glu Lys Pro Ala Gly Lys Val Asp Leu Lys Leu
   385 390 395 400
   Ile Ala Cys Glu Glu Lys Asn Ser Gln Gly Met Cys Ser Leu Lys Ser
   405 410 415
   Phe Ser Arg Leu Ile Lys Glu Ile Arg Val Pro Glu Cys Ala Val Thr
   420 425 430
   Glu
<210> 3
   <211> 1302
   <212> DNA
   <213> Citrobacter braakii
<400> 3
   atgagtacat tcatcattcg tttattattt ttttctctct tatgcggttc tttctcaata 60
   catgctgaag agcagaatgg tatgaaactt gagcgggttg tgatagtgag tcgtcatggr 120
   gtaagagcac ctacgaagtt cactccaata atgaaaaatg tcacacccga tcaatggcca 180
   caatgggatg tgccgttagg atggctaacg cctcgtgggg gagaacttgt ttctgaatta 240
   ggtcagtatc aacgtttatg gttcacgagc aaaggtctgt tgaataatca aacgtgccca 300
   tctccagggc aggttgctgt tattgcagac acggatcaac gcacccgtaa aacgggtgag 360
   gcgtttctgg ctgggttagc accaaaatgt caaattcaag tgcattatca gaaggatgaa 420
   gaaaaaaatg atcctctttt taatccggta aaaatgggga aatgttcgtt taacacattg 480
   caggttaaaa acgctattct ggaacgggcc ggaggaaata ttgaactgta tacccaacgc 540
   tatcaatctt catttcggac cctggaaaat gttttaaatt tctcacaatc ggagacatgt 600
   aagactacag aaaagtctac gaaatgcaca ttaccagagg ctttaccgtc tgaacttaag 660
   gtaactcctg acaatgtatc attacctggt gcctggagtc tttcttccac gctgactgag 720
   atatttctgt tgcaagaggc ccagggaatg ccacaggtag cctgggggcg tattacggga 780
   gaaaaagaat ggagagattt gttaagtctg cataacgctc agtttgatct tttgcaaaga 840
   actccagaag ttgcccgtag tagggccaca ccattactcg atatgataga cactgcatta 900
   ttgacaaatg gtacaacaga aaacaggtat ggcataaaat tacccgtatc tctgttgttt 960
   attgctggtc atgataccaa tcttgcaaat ttaagcgggg ctttagatct taactggtcg 1020
   ctacccggtc aaccsgataa yaccccgccg ggcgacaagc ttgtattcga aaagtggaaa 1080
   agaaccagtg ataatacgga ttgggttcag gtttcatttg tttatcagac gctgagagat 1140
   atgagggata tacaaccgtt gtcgttagaa aaacctgctg gcaaagttga tttaaaatta 1200
   attgcatgtg aagagaaaaa tagtcaggga atgtgttcgt taaaaagttt ttccaggctc 1260
   attaaggaaa ttcgcgtgcc agagtgtgca gttacggaat aa 1302
<210> 4
   <211> 1299
   <212> DNA
   <213> Citrobacter braakii
<400> 4
   atgagtacat tcatcattcg tttattattt ttttctctct tatgcggttc tttctcaata 60
   catgctgaag agcagaatgg tatgaaactt gagcgggttg tgatagtgag tcgtcatgga 120
   gtaagagcac ctacgaagtt cactccaata atgaaaaatg tcacacccga tcaatggcca 180
   caatgggatg tgccgttagg atggctaacg cctcgtgggg gagaacttgt ttctgaatta 240
   ggtcagtatc aacgtttatg gttcacgagc aaaggtctgt tgaataatca aacgtgccca 300
   tctccagggc aggttgctgt tattgcagac acggatcaac gcacccgtaa aacgggtgag 360
   gcgtttctgg ctgggttagc accaaaatgt caaattcaag tgcattatca gaaggatgaa 420
   gaaaaaaatg atcctctttt taatccggta aaaatgggga aatgttcgtt taacacattg 480
   caggttaaaa acgctattct ggaacgggcc ggaggaaata ttgaactgta tacccaacgc 540
   tatcaatctt catttcggac cctggaaaat gttttaaatt tctcacaatc ggagacatgt 600
   aagactacag aaaagtctac gaaatgcaca ttaccagagg ctttaccgtc tgaacttaag 660
   gtaactcctg acaatgtatc attacctggt gcctggagtc tttcttccac gctgactgag 720
   atatttctgt tgcaagaggc ccagggaatg ccacaggtag cctgggggcg tattacggga 780
   gaaaaagaat ggagagattt gttaagtctg cataacgctc agtttgatct tttgcaaaga 840
   actccagaag ttgcccgtag tagggccaca ccattactcg atatgataga cactgcatta 900
   ttgacaaatg gtacaacaga aaacaggtat ggcataaaat tacccgtatc tctgttgttt 960
   attgctggtc atgataccaa tcttgcaaat ttaagcgggg ctttagatct taactggtcg 1020
   ctacccggtc aacccgataa tacccctcct ggtggggagc ttgtattcga aaagtggaaa 1080
   agaaccagtg ataatacgga ttgggttcag gtttcatttg tttatcagac gctgagagat 1140
   atgagggata tacaaccgtt gtcgttagaa aaacctgctg gcaaagttga tttaaaatta 1200
   attgcatgtg aagagaaaaa tagtcaggga atgtgttcgt taaaaagttt ttccaggctc 1260
   attaaggaaa ttcgcgtgcc agagtgtgca gttacggaa 1299
<210> 5
   <211> 433
   <212> PRT
   <213> Citrobacter freundii
<400> 5
<210> 6
   <211> 1401
   <212> DNA
   <213> Citrobacter freundii
<400> 6
   aaaggtggtg ctggtaatga gtacattcat cattcgttta ttattttttt ctctcttatg 60
   cggttctttc tcaatacatg ctgaagagcc gaacggtatg aaacttgagc gggttgtgat 120
   agtgagccgt catggagtaa gagcacctac gaagttcact ccaataatga aagatgttac 180
   acccgatcaa tggccacaat gggatgtgcc gttaggatgg ctaacgcctc gtgggggaga 240
   acttgtttct gaattaggtc agtatcaacg tttatggttc acaagcaaag gtctgttgaa 300
   taatcaaacg tgcccatctc cagggcaggt tgctgttatt gcagacacgg atcaacgcac 360
   ccgtaaaacg ggtgaggcgt ttctggctgg gttagcacca aaatgtcaaa ttcaagtgca 420
   ttatcagaag gatgaagaaa aaactgatcc tctttttaat ccagtaaaaa tggggacatg 480
   ttcgtttaac acattgaagg ttaaaaacgc tattctggaa cgggccggag gaaatattga 540
   actgtatacc caacgctatc aatcttcatt tcggaccctg gaaaatgttt taaatttctc 600
   acaatcggag acatgtaaga ctacagaaaa gtctacgaaa tgcacattac cagaggcttt 660
   accgtctgaa cttaaggtaa ctcctgacaa tgtatcatta cctggtgcct ggagtctttc 720
   ttccacgctg actgagatat ttctgttgca agaggcccag ggaatgccac aggtagcctg 780
   ggggcgtatt acgggagaaa aagaatggag agatttgtta agtctgcata acgctcagtt 840
   tgatcttttg caaagaactc cagaagttgc ccgtagtagg gccacaccat tactcgatat 900
   gatagacact gcattattga caaatggtac aacagaaaac aggtatggca taaaattacc 960
   cgtatctctg ttgtttattg ctggtcatga taccaatctt gcaaatttaa gcggggcttt 1020
   agatcttaac tggtcgctgc ccggtcaacc cgataatacc cctcctggtg gggagcttgt 1080
   attcgaaaag tggaaaagaa ccagtgataa tacggattgg gttcaggttt catttgttta 1140
   tcagacgctg agagatatga gggatataca accgttgtcg ttagaaaaac ctgccggcaa 1200
   agttgattta aaattaattg catgtgaaga gaaaaatagt cagggaatgt gttcgttaaa 1260
   aagtttttcc aggctcatta aggaaattcg cgtgccagag tgtgcagtta cggaataagt 1320
   aactaattac tatatatagc gtattaaaaa atagaaaccc ccggtttgta gtcgggggta 1380
   ttcgtattgt tcataattac a 1401
<210> 7
   <211> 433
   <212> PRT
   <213> Citrobacter braakii
<400> 7
   Met Ser Thr Phe Ile Ile Arg Leu Leu Ile Phe Ser Leu Leu Cys Gly
   1               5                   10                  15
   Ser Phe Ser Ile His Ala Glu Glu Gln Asn Gly Met Lys Leu Glu Arg
               20                  25                  30
   Val Val Ile Val Ser Arg His Gly Val Arg Ala Pro Thr Lys Phe Thr
           35                  40                  45
   Pro Ile Met Lys Asp Val Thr Pro Asp Gln Trp Pro Gln Trp Asp Val
       50                  55                  60
   Pro Leu Gly Trp Leu Thr Pro Arg Gly Gly Glu Leu Val Ser Glu Leu
   65                  70                  75                  80
   Gly Gln Tyr Gln Arg Leu Trp Phe Thr Ser Lys Gly Leu Leu Asn Asn
                   85                  90                  95
   Gln Thr Cys Pro Ser Pro Gly Gln Val Ala Val Ile Ala Asp Thr Asp
               100                 105                 110
   Gln Arg Thr Arg Lys Thr Gly Glu Ala Phe Leu Ala Gly Leu Ala Pro
           115                 120                 125
   Lys Cys Gln Ile Gln Val His Tyr Gln Lys Asp Glu Glu Lys Asn Asp
       130                 135                 140
   Pro Leu Phe Asn Pro Val Lys Met Gly Lys Cys Ser Phe Asn Thr Leu
   145                 150                 155                 160
   Lys Val Lys Asn Ala Ile Leu Glu Arg Ala Gly Gly Asn Ile Glu Leu
                   165                 170                 175
   Tyr Thr Gln Arg Tyr Gln Ser Ser Phe Arg Thr Leu Glu Asn Val Leu
               180                 185                 190
   Asn Phe Ser Gln Ser Glu Thr Cys Lys Thr Thr Glu Lys Ser Thr Lys
           195                 200                 205
   Cys Thr Leu Pro Glu Ala Leu Pro Ser Glu Phe Lys Val Thr Pro Asp
       210                 215                 220
   Asn Val Ser Leu Pro Gly Ala Trp Ser Leu Ser Ser Thr Leu Thr Glu
   225                 230                 235                 240
   Ile Phe Leu Leu Gln Glu Ala Gln Gly Met Pro Gln Val Ala Trp Gly
                   245                 250                 255
   Arg Ile Thr Gly Glu Lys Glu Trp Arg Asp Leu Leu Ser Leu His Asn
               260                 265                 270
   Ala Gln Phe Asp Leu Leu Gln Arg Thr Pro Glu Val Ala Arg Ser Arg
           275                 280                 285
   Ala Thr Pro Leu Leu Asp Met Ile Asp Thr Ala Leu Leu Thr Asn Gly
       290                 295                 300
   Thr Thr Glu Asn Arg Tyr Gly Ile Lys Leu Pro Val Ser Leu Leu Phe
   305 310 315 320
   Ile Ala Gly His Asp Thr Asn Leu Ala Asn Leu Ser Gly Ala Leu Asp
   325 330 335
   Leu Lys Trp Ser Leu Pro Gly Gln Pro Asp Asn Thr Pro Pro Gly Gly
   340 345 350
   Glu Leu Val Phe Glu Lys Trp Lys Arg Thr Ser Asp Asn Thr Asp Trp
   355 360 365
   Val Gln Val Ser Phe Val Tyr Gln Thr Leu Arg Asp Met Arg Asp Ile
   370 375 380
   Gln Pro Leu Ser Leu Glu Lys Pro Ala Gly Lys Val Asp Leu Lys Leu
   385 390 395 400
   Ile Ala Cys Glu Glu Lys Asn Ser Gln Gly Met Cys Ser Leu Lys Ser
   405 410 415
   Phe Ser Arg Leu Ile Lys Glu Ile Arg Val Pro Glu Cys Ala Val Thr
   420 425 430
   Glu
<210> 8
   <211> 423
   <212> PRT
   <213> Peniphora lycii
<400> 8
   Ser Leu Ala Leu Ser Thr Gln Phe Ser Phe Val Ala Ala Gln Leu Pro
   1 5 10 15
   Ile Pro Ala Gln Asn Thr Ser Asn Trp Gly Pro Tyr Asp Pro Phe Phe
   20 25 30
   Pro Val Glu Pro Tyr Ala Ala Pro Pro Glu Gly Cys Thr Val Thr Gln
   35 40 45
   Val Asn Leu Ile Gln Arg His Gly Ala Arg Trp Pro Thr Ser Gly Ala
   50 55 60
   Arg Ser Arg Gln Val Ala Ala Val Ala Lys Ile Gln Met Ala Arg Pro
   65 70 75 80
   Phe Thr Asp Pro Lys Tyr Glu Phe Leu Asn Asp Phe Val Tyr Lys Phe
   85 90 95
   Gly Val Ala Asp Leu Leu Pro Phe Gly Ala Asn Gln Ser His Gln Thr
   100 105 110
   Gly Thr Asp Met Tyr Thr Arg Tyr Ser Thr Leu Phe Glu Gly Gly Asp
   115 120 125
   Val Pro Phe Val Arg Ala Ala Gly Asp Gln Arg Val Val Asp Ser Ser
   130 135 140
   Thr Asn Trp Thr Ala Gly Phe Gly Asp Ala Ser Gly Glu Thr Val Leu
   145 150 155 160
   Pro Thr Leu Gln Val Val Leu Gln Glu Glu Gly Asn Cys Thr Leu Cys
   165 170 175
   Asn Asn Met Cys Pro Asn Glu Val Asp Gly Asp Glu Ser Thr Thr Trp
   180 185 190
   Leu Gly Val Phe Ala Pro Asn Ile Thr Ala Arg Leu Asn Ala Ala Ala
   195 200 205
   Pro Ser Ala Asn Leu Ser Asp Ser Asp Ala Leu Thr Leu Met Asp Met
   210 215 220
   Cys Pro Phe Asp Thr Leu Ser Ser Gly Asn Ala Ser Pro Phe Cys Asp
   225 230 235 240
   Leu Phe Thr Ala Glu Glu Tyr Val Ser Tyr Glu Tyr Tyr Tyr Asp Leu
   245 250 255
   Asp Lys Tyr Tyr Gly Thr Gly Pro Gly Asn Ala Leu Gly Pro Val Gln
   260 265 270
   Gly Val Gly Tyr Val Asn Glu Leu Leu Ala Arg Leu Thr Gly Gln Ala
   275 280 285
   Val Arg Asp Glu Thr Gln Thr Asn Arg Thr Leu Asp Ser Asp Pro Ala
   290 295 300
   Thr Phe Pro Leu Asn Arg Thr Phe Tyr Ala Asp Phe Ser His Asp Asn
   305 310 315 320
   Thr Met Val Pro Ile Phe Ala Ala Leu Gly Leu Phe Asn Ala Thr Ala
   325 330 335
   Leu Asp Pro Leu Lys Pro Asp Glu Asn Arg Leu Trp Val Asp Ser Lys
   340 345 350
   Leu Val Pro Phe Ser Gly His Met Thr Val Glu Lys Leu Ala Cys Ser
   355 360 365
   Gly Lys Glu Ala Val Arg Val Leu Val Asn Asp Ala Val Gln Pro Leu
   370 375 380
   Glu Phe Cys Gly Gly Val Asp Gly Val Cys Glu Leu Ser Ala Phe Val
   385 390 395 400
   Glu Ser Gln Thr Tyr Ala Arg Glu Asn Gly Gln Gly Asp Phe Ala Lys
   405 410 415
   Cys Gly Phe Val Pro Ser Glu
   420
<210> 9
   <211> 433
   <212> PRT
   <213> Citrobacter freundii
<400> 9
   Met Ser Thr Phe Ile Ile Arg Leu Leu Phe Phe Ser Leu Leu Cys Gly
   1 5 10 15
   Ser Phe Ser Ile His Ala Glu Glu Gln Asn Gly Met Lys Leu Glu Arg
   20 25 30
   Val Val Ile Val Ser Arg His Gly Val Arg Ala Pro Thr Lys Phe Thr
   35 40 45
   Pro Ile Met Lys Asp Val Thr Pro Asp Gln Trp Pro Gln Trp Asp Val
   50 55 60
   Pro Leu Gly Trp Leu Thr Pro Arg Gly Gly Glu Leu Val Ser Glu Leu
   65 70 75 80
   Gly Gln Tyr Gln Arg Leu Trp Phe Thr Ser Lys Gly Leu Leu Asn Asn
   85 90 95
   Gln Thr Cys Pro Ser Pro Gly Gln Val Ala Val Ile Ala Asp Thr Asp
   100 105 110
   Gln Arg Thr Arg Lys Thr Gly Glu Ala Phe Leu Ala Gly Leu Ala Pro
   115 120 125
   Lys Cys Gln Ile Gln Val His Tyr Gln Lys Asp Glu Glu Lys Thr Asp
       130                 135                 140
   Pro Leu Phe Asn Pro Val Lys Met Gly Thr Cys Ser Phe Asn Thr Leu
   145                 150                 155                 160
   Lys Val Lys Asn Ala Ile Leu Glu Arg Ala Gly Gly Asn Ile Glu Leu
                   165                 170                 175
   Tyr Thr Gln Arg Tyr Gln Ser Ser Phe Arg Thr Leu Glu Asn Val Leu
               180                 185                 190
   Asn Phe Ser Gln Ser Glu Thr Cys Lys Thr Thr Glu Lys Ser Thr Lys
           195                 200                 205
   Cys Thr Leu Pro Glu Ala Leu Pro Ser Glu Leu Lys Val Thr Pro Asp
       210                 215                 220
   Asn Val Ser Leu Pro Gly Ala Trp Ser Leu Ser Ser Thr Leu Thr Glu
   225                 230                 235                 240
   Ile Phe Leu Leu Gln Glu Ala Gln Gly Met Pro Gln Val Ala Trp Gly
                   245                 250                 255
   Arg Ile Thr Gly Glu Lys Glu Trp Arg Asp Leu Leu Ser Leu His Asn
               260                 265                 270
   Ala Gln Phe Asp Leu Leu Gln Arg Thr Pro Glu Val Ala Arg Ser Arg
           275                 280                 285
   Ala Thr Pro Leu Leu Asp Met Ile Asp Thr Ala Leu Leu Thr Asn Gly
       290                 295                 300
   Thr Thr Glu Asn Arg Tyr Gly Ile Lys Leu Pro Val Ser Leu Leu Phe
   305                 310                 315                 320
   Ile Ala Gly His Asp Thr Asn Leu Ala Asn Leu Ser Gly Ala Leu Asp
                   325                 330                 335
   Leu Asn Trp Ser Leu Pro Gly Gln Pro Asp Asn Thr Pro Pro Gly Gly
               340                 345                 350
   Glu Leu Val Phe Glu Lys Trp Lys Arg Thr Ser Asp Asn Thr Asp Trp
           355                 360                 365
   Val Gln Val Ser Phe Val Tyr Gln Thr Leu Arg Asp Met Arg Asp Ile
       370                 375                 380
   Gln Pro Leu Ser Leu Glu Lys Pro Ala Gly Lys Val Asp Leu Lys Leu
   385 390 395 400
   Ile Ala Cys Glu Glu Lys Asn Ser Gln Gly Met Cys Ser Leu Lys Ser
   405 410 415
   Phe Ser Arg Leu Ile Lys Glu Ile Arg Val Pro Glu Cys Ala Val Thr
   420 425 430
   Glu
<210> 10
   <211> 433
   <212> PRT
   <213> Citrobacter freundii
<400> 10
   Met Ser Thr Phe Ile Ile Arg Leu Leu Phe Phe Ser Leu Leu Cys Gly
   1 5 10 15
   Ser Phe Ser Ile His Ala Glu Glu Gln Asn Gly Met Lys Leu Glu Arg
   20 25 30
   Val Val Ile Val Ser Arg His Gly Val Arg Ala Pro Thr Lys Phe Thr
   35 40 45
   Pro Ile Met Lys Asp Val Thr Pro Asp Gln Trp Pro Gln Trp Asp Val
   50 55 60
   Pro Leu Gly Trp Leu Thr Pro Arg Gly Gly Glu Leu Val Ser Glu Leu
   65 70 75 80
   Gly Gln Tyr Gln Arg Leu Trp Phe Thr Ser Lys Gly Leu Leu Asn Asn
   85 90 95
   Gln Thr Cys Pro Ser Pro Gly Gln Val Ala Val Ile Ala Asp Thr Asp
   100 105 110
   Gln Arg Thr Arg Lys Thr Gly Glu Ala Phe Leu Ala Gly Leu Ala Pro
   115 120 125
   Lys Cys Gln Ile Gln Val His Tyr Gln Lys Asp Glu Glu Lys Thr Asp
   130 135 140
   Pro Leu Phe Asn Pro Val Lys Met Gly Thr Cys Ser Phe Asn Thr Leu
   145 150 155 160
   Lys Val Lys Asn Ala Ile Leu Glu Arg Ala Gly Gly Asn Ile Glu Leu
               165                 170                 175
   Tyr Thr Gln Arg Tyr Gln Ser Ser Phe Arg Thr Leu Glu Asn Val Leu
           180                 185                 190
   Asn Phe Ser Gln Ser Glu Thr Cys Lys Thr Thr Glu Lys Ser Thr Lys
       195                 200                 205
   Cys Thr Leu Pro Glu Ala Leu Pro Ser Glu Leu Lys Val Thr Pro Asp
       210                 215                 220
   Asn Val Ser Leu Pro Gly Ala Trp Ser Leu Ser Ser Thr Leu Thr Glu
   225                 230                 235                 240
   Ile Phe Leu Leu Gln Glu Ala Gln Gly Met Pro Gln Val Ala Trp Gly
               245                 250                 255
   Arg Ile Thr Gly Glu Lys Glu Trp Arg Asp Leu Leu Ser Leu His Asn
           260                 265                 270
   Ala Gln Phe Asp Leu Leu Gln Arg Thr Pro Glu Val Ala Arg Ser Arg
       275                 280                 285
   Ala Thr Pro Leu Leu Asp Met Ile Asp Thr Ala Leu Leu Thr Asn Gly
       290                 295                 300
   Thr Thr Glu Asn Arg Tyr Gly Ile Lys Leu Pro Val Ser Leu Leu Phe
   305                 310                 315                 320
   Ile Ala Gly His Asp Thr Asn Leu Ala Asn Leu Ser Gly Ala Leu Asp
               325                 330                 335
   Leu Asn Trp Ser Leu Pro Gly Gln Pro Asp Asn Thr Pro Pro Gly Gly
           340                 345                 350
   Glu Leu Val Phe Glu Lys Trp Lys Arg Thr Ser Asp Asn Thr Asp Trp
       355                 360                 365
   Val Gln Val Ser Phe Val Tyr Gln Thr Leu Arg Asp Met Arg Asp Ile
       370                 375                 380
   Gln Pro Leu Ser Leu Glu Lys Pro Ala Gly Lys Val Asp Leu Lys Leu
   385                 390                 395                 400
   Ile Ala Cys Glu Glu Lys Asn Ser Gln Gly Met Cys Ser Leu Lys Ser
               405                 410                 415
   Phe Ser Arg Leu Ile Lys Glu Ile Arg Val Pro Glu Cys Ala Val Thr
                   420                     425                     430
   Glu
<210> 11
   <211> 3279
   <212> DNA
   <213> Citrobacter freundii
<220>
   <221> misc_feature
   <222> (185)..(185)
   <223> n is a, c, g, or t
<220>
   <221> misc_feature
   <222> (515)..(515)
   <223> n is a, c, g, or t
<220>
   <221> misc_feature
   <222> (536)..(536)
   <223> n is a, c, g, or t
<220>
   <221> misc_feature
   <222> (612)..(612)
   <223> n is a, c, g, or t
<220>
   <221> misc_feature
   <222> (903)..(903)
   <223> n is a, c, g, or t
<400> 11
   aaagggaagc ccgttgtcgt tctttcaaac aacgatggct gcgttattgc cagtagcgta      60
   gaggcaaagg cgcttggcgt taatatgggc gctccgtact tcaaacagaa agatctgttc     120
   aggcgctatg gcgtgttctg ttttagttcg aactacgaac tgtatgcgga tatgagcagc     180
   agggntgatg tcgattctgg aggagttgtc gccacgcgtg gagatataca gtattgatga     240
   agccttttgt gatttaacgg gcgtgcgaaa ctgcagggat cttacggatt ttggtcagga     300
   aatcagagca accattttgc agaaaacacg tctcacggtt ggggtcggca ttgctcagac     360
   caaaaccctg gccaaactgg cgaaccatgc ggccaaaaag tggcaggaac agacgggcgg     420
   agtcgtggat ctctcaaata ttgaacgcca gcgtaagctg atggcagcac tcccggttga     480
   tgaagtctgg ggcatcggac gccgtatcgg caagnaactg gaacgtcatg ggcatncaaa     540
   cggttctcga acctcgcggg tcccgatcat tcgcttcatt cgtcaaacat cttcagcgtc     600
   gtgcttgaag cncaaccgtc cgggcattgc gcggtgagcc ttgttctggc gctggaaaaa     660
   gtttgcgccg gaatcagcag gcaattatct gctcagaatc gtttggtgaa aaactcacgg 720
   aattacaacg ccatgaaaaa aggccatttg cacctatgct tcacgtgctg cggaaaagct 780
   gcgcagaaaa caccagtact gccggtttat ctctacgttt atcaaaacca gtccgtttgc 840
   cctgaatgaa ccttattacg gtaacagcgc atcgataaaa ctgctgacgc caactcagga 900
   tanccgggac attattacag cagccacgcg cagcctggac gccgtttggc gagaggggct 960
   acgttatcaa aaagcgggcg ttatgctggg ggattttttt agctctggcg ttgcacagct 1020
   gaatcttttt gataataacg ccccgcggcg taacagcgat aaattgatgg acctactgga 1080
   tactcttaat gctgaaaagg ggaaaggaac gctctacttt gccgggcaag ggatacagca 1140
   gccgtgggcg atgaagcgag acatgctttc accacgttat acaacaagat acagtgattt 1200
   gctgcgggta aattgatagc caaacaaggc ttccgcccac cttatttata ccggatcaat 1260
   tatcctttca atgacaggac gttgtaaatt gccaacgatt ccataacctt gggatacctt 1320
   tccagcgcgg ctaaattagc gctcctttaa tacttcgata agatacaaag ttgatatgca 1380
   aaaagttgga ttgttgtgaa ctcaggagta ggattatttc tatctgatag aaccagttat 1440
   cgaactggct tatacgagtt gtttgttttt cacttacgaa aaggtggtgc tggctaatga 1500
   gtacattcat cattcgttta ttattttttt ctctcttatg cggttctttc tcaatacatg 1560
   ctgaagagca gaacggtatg aaacttgagc gggttgtgat agtgagccgc catggagtaa 1620
   gagcacctac gaagttcact ccaataatga aagatgtcac acccgatcaa tggccacaat 1680
   gggatgtgcc gttaggatgg ctaacgcctc gtgggggaga acttgtttct gaattaggtc 1740
   agtatcaacg tttatggttc acgagcaaag gtctgttgaa taatcaaacg tgcccatctc 1800
   cagggcaggt tgctgttatt gcagacacgg atcaacgcac ccgtaaaacg ggtgaggcgt 1860
   ttctggctgg gttagcacca aaatgtcaaa ttcaagtgca ttatcagaag gatgaagaaa 1920
   aaactgatcc tctttttaat ccagtaaaaa tggggacatg ttcgtttaac acattgaagg 1980
   ttaaaaacgc tattctggaa cgggccggag gaaatattga actgtatacc caacgctatc 2040
   aatcttcatt tcggaccctg gaaaatgttt taaatttctc acaatcggag acatgtaaga 2100
   ctacagaaaa gtctacgaaa tgcacattac cagaggcttt accgtctgaa cttaaggtaa 2160
   ctcctgacaa tgtatcatta cctggtgcct ggagcctttc ttccacgctg actgagatat 2220
   ttctgttgca agaggcccag ggaatgccac aggtagcctg ggggcgtatt acgggggaaa 2280
   aagaatggag agatttgtta agtctgcata acgctcagtt tgatcttttg caaagaactc 2340
   cagaagttgc ccgtagtaga gccacaccat tactcgatat gatagacact gcattattga 2400
   caaatggtac aacagaaaac aggtatggca taaaattacc cgtatctctg ttgtttattg 2460
   ctggtcatga taccaatctt gcaaatttaa gcggggcttt agatcttaac tggtcgctac 2520
   ccggtcaacc cgataatacc cctcctggtg gggagcttgt attcgaaaag tggaaaagaa 2580
   ccagtgataa tacggattgg gttcaggttt catttgttta tcagacgctg agagatatga 2640
   gggatataca accgttgtcg ttagaaaaac ctgccggcaa agttgattta aaattaattg 2700
   catgtgaaga gaaaaatagt cagggaatgt gttcgttaaa aagtttttcc aggctcatta 2760
   aggaaattcg cgtgccagag tgtgcagtta cggaataagt aattaattat tatatatata 2820
   gcgtattaaa aaatagaaac ccccggtttg tagtcggggg tattcgtatt gtttcataat 2880
   tacatgagtc ttcaactgac tgctctttgc gagtgacaat ccagttacgc tgctccagaa 2940
   aaaatccgga ctgttcagcg atgcagaata catcgaaaag cgctcggtgt tggactgcga 3000
   ctctaaaacc gcttaacacg gttatatgcc cacatatgtc taaatgtgct gtttgtgcaa 3060
   cgtattaaat aataacaagt tattaatttt acaattagtt aaaaaaactg atagtatacc 3120
   cccctatagt atttggaggg cgtatgccgc attcacccga agataaaaaa cgcattctca 3180
   cccgtgtacg ccgtattcgc ggtcaggttg atgcgcttga gcgcgcgctg gagtccggcg 3240
   agccgtgttt ggctatcctg caacaaattg ccgccgtgc 3279
<210> 12
   <211> 19
   <212> DNA
   <213> Artificial Sequence
<220>
   <223> Primer 28F
<220>
   <221> misc_feature
   <222> (11)..(11)
   <223> n is a, c, g, or t
<400> 12
   gagtttgatc ntggctcag 19
<210> 13
   <211> 18
   <212> DNA
   <213> Artificial Sequence
<220>
   <223> Primer 519R
<220>
   <221> misc_feature
   <222> (3)..(3)
   <223> n is a, c, g, or t
<220>
   <221> misc_feature
   <222> (8)..(8)
   <223> n is a, c, g, or t
<400> 13
   gtnttacngc ggckgctg 18

## Claims

1. A feed additive composition comprising a direct fed microbial (DFM) in combination with a phytase derivable from *Citrobacter* spp, wherein the phytase comprises a polypeptide comprising an amino acid sequence which has at least 99.1% identity with amino acids 23-433 of SEQ ID No 1 or 2, and wherein the direct fed microbial comprises a bacterium from one or more of the following species: *Bacillus subtilis, Lactobacillus farciminus, Lactobacillus rhamnosus* and combinations thereof.

2. A method for improving nitrogen retention or for improving feed conversion ratio (FCR) or for improving weight gain in a subject or for improving feed efficiency in a subject or for reducing nutrient excretion in manure, which method comprisies administering to a subject a direct fed microbial (DFM) in combination with a phytase derivable from *Citrobacter* spp, wherein the phytase comprises a polypeptide comprising an amino acid sequence which has at least 99.1% identity with amino acids 23-433 of SEQ ID No 1 or 2, wherein the direct fed microbial comprises a bacterium from one or more of the following species: *Bacillus subtilis, Lactobacillus farciminus, Lactobacillus rhamnosus* and combinations thereof, and wherein the subject is poultry.

3. Use of a direct fed microbial (DFM) in combination with a phytase derivable from *Citrobacter* spp. for improving nitrogen retention or for improving feed conversion ratio (FCR) or for improving weight gain in a subject or for improving feed efficiency in a subject or for reducing nutrient excretion in manure, wherein the phytase comprises a polypeptide comprising an amino acid sequence which has at least 99.1% identity with amino acids 23-433 of SEQ ID No 1 or 2, wherein the direct fed microbial comprises a bacterium from one or more of the following species: *Bacillus subtilis, Lactobacillus farciminus, Lactobacillus rhamnosus* and combinations thereof, and wherein the subject is poultry.

4. A kit comprising a direct fed microbial (DFM), a phytase derivable from *Citrobacter* spp., and instructions for administration, wherein the phytase comprises a polypeptide comprising an amino acid sequence which has at least 99.1% identity with amino acids 23-433 of SEQ ID No 1 or 2, wherein the direct fed microbial comprises a bacterium from one or more of the following species: *Bacillus subtilis, Lactobacillus farciminus, Lactobacillus rhamnosus* and combinations thereof.

5. A feed additive composition according to claim 1 or a method according to claim 2 or a use according to claim 3 or a kit according to claim 4- wherein the phytase is derivable from a *Citrobacter* bacterium selected from the group consisting of: *Citrobacter braakii, Citrobacter freundii, Citrobacter amalonaticus, Citrobacter gillenii, Citrobacter intermedius, Citrobacter koseri, Citrobacter murliniae, Citrobacter rodentium, Citrobacter sedlakii, Citrobacter werkmanii* and *Citrobacter youngae,* preferably wherein the phytase is derived from *Citrobacter braakii.*

6. A feed additive composition according to any one of claims 1 or 5 or a method according to claims 2 or 5 or a use according to claim 3 or 5 or a kit according to claims 4-5 wherein the phytase is derived from *Citrobacter braakii* ATCC 51113.

7. A feed additive composition according to claim 1 or a method according to claim 2 or 5-6 or a use according to claims 3 or 5-6 or a kit according to claims 4-6 wherein the phytase is a 6-phytase (E.C. 3.1.3.26).

8. A feed additive composition according to claim 1 or a method according to claims 2 or 5-7 or a use according to claims 3 or 5-7 or a kit according to claims 4-7 wherein the phytase comprises at least one alteration and no more than 4 alterations as compared to SEQ ID No. 1 or SEQ ID No. 2, wherein at least one of said one to four alterations is selected from the following: 4P, 46E, 107G, 111 P, 119K, 162C, 223E, 241Q, 273L, 276K, 379K, 385D, 91C/46C, 52C/99C, 31C/176C, 31C/177C, 59C/100C, 141C/199C, 162C/247C, 111P/241Q, 31C, 119K, 202N, 286Q and 362K,R.

9. A feed additive composition, a method, a use or a kit according to claim 8 wherein the phytase comprises at least one alteration and no more than 4 alterations as compared to SEQ ID No. 1 or SEQ ID No. 2, wherein at least one of said one to four alterations is selected from the following: 91C/46C, 52C/99C, 31C/176C, 31C/177C, 59C/100C, 141C/199C, 162C/247C, 111P/241Q.

10. A feed additive composition according to any one of claims 1 or 5-9 or a method according to claims 2 or 5-9 or a use according to claims 3 or 5-9 or a kit according to claims 4-9 wherein the phytase has a pH optima in the range of 3-4.5.

11. A feed additive composition, a method, a use or a kit according to claim 10, wherein the phytase has a pH optimum between 3 and 3.5.

12. A feed additive composition according to any one of claims 1 or 5-11 or a method according to claims 2 or 5-11 or a use according to claims 3 or 5-11 or a kit according to claims 4-11 wherein the direct fed microbial is an antipathogen direct fed microbial.

13. A feed additive composition according to any one of claims 1 or 5-12 or a method according to claims 2 or 5-12 or a use according to claims 3 or 5-12 or a kit according to claims 4-12 wherein the direct fed microbial is a viable bacteria.

14. A feed additive composition according to any one of claims 1 or 8-13 or a method according to claims 2 or 5-13 or a use according to claims 4 or 5-13 or a kit according to claims 4-13 wherein the direct fed microbial is one or more of the following: *Bacillus subtilis* strains 3A-P4 (PTA-6506); 15A-P4 (PTA-6507); 22C-P1 (PTA-6508); 2084 (NRRL B-500130); LSSA01 (NRRL-B-50104); BS27 (NRRL B-50105); BS 18 (NRRL B-50633); and BS 278 (NRRL B-50634).

15. A feed additive composition according to any one of claims 1 or 5-14 or a method according to claims 2 or -5-14 or a use according to claims 3 or 5-14 or a kit according to claims 4-14 wherein the direct fed microbial is in the form of an endospore.

16. A feed additive composition according to any one of claims 1 or 5-15 wherein the phytase is present at a dosage of between 200FTU/g feed additive composition and 10000 FTU/g feed additive composition.

17. A feed additive composition according to any one of claims 1 or 5-16 wherein the DFM is present at a dosage of between 3.75x10⁷CFU/g feed additive composition and 1x10¹¹CFU/g feed additive composition.

18. A method according to claim 2 comprising administering the feed additive composition according to any one of claims 1 or 5-17.

19. Use according to claim 3 wherein the feed additive composition according to any one of claims 1 or 5-17 is used.

20. A kit according to claim 4 wherein said kit comprises the feed additive composition according to any one of claims 1 or 5-17.

21. A kit according to any one of claims 4-15 or 20 which comprises at least one vitamin and/or at least one mineral.

22. A method of preparing a feed additive composition, comprising admixing a direct fed microbial (DFM) with a phytase derivable from *Citrobacter* spp., wherein the phytase comprises a polypeptide comprising an amino acid sequence which has at least 99.1% identity with amino acids 23-433 of SEQ ID No 1 or 2 and wherein the direct fed microbial comprises a bacterium from one or more of the following species: *Bacillus subtilis, Lactobacillus farciminus, Lactobacillus rhamnosus* and combinations thereof.

23. A method according to claim 22, which further comprises packaging.

24. A feed comprising a feed additive composition according to any one of claims 1 or 5-17.

25. A feed according to claim 24 wherein the phytase is present at a dosage of between 400FTU/kg feed and 1000 FTU//kg feed.

26. A feed according to any one of claims 24-25 wherein the DFM is present at a dosage of between 7.5x10⁴CFU/kg feed and 1x10⁷CFU/kg feed.

27. A method of preparing a feedstuff comprising admixing a feed component with a feed additive composition according to any one of claims 1 or 5-17.

28. A premix comprising a feed additive composition comprising a direct fed microbial in combination with a *Citrobacter* phytase, and at least one mineral and/or at least one vitamin, wherein the phytase comprises a polypeptide comprising an amino acid sequence which has at least 99.1% identity with amino acids 23-433 of SEQ ID No 1 or 2 and wherein the direct fed microbial comprises a bacterium from one or more of the following species: *Bacillus subtilis, Lactobacillus farciminus, Lactobacillus rhamnosus* and combinations thereof.

29. A premix comprising a feed additive composition according to any one of claims 1 or 5-17 in combination with at least one mineral and/or at least one vitamin.

30. A feed additive composition according to any one of claims 1 or 5-17 for use in preventing and/or treating necrotic enteritis and/or coccidiosis in poultry.

## Patentansprüche

1. Futterzusatzzusammensetzung, umfassend einen direkt verfütterten Mikroorganismus (DFM, direct fed microbial) in Kombination mit einer von *Citrobacter-* Spez. ableitbaren Phytase, wobei die Phytase ein Polypeptid umfasst, das eine Aminosäuresequenz umfasst, die zumindest 99,1% Identität mit Aminosäuren 23-433 von SEQ ID Nr. 1 oder 2 aufweist, und wobei der direkt verfütterte Mikroorganismus ein Bakterium aus einer oder mehreren der folgenden Spezies umfasst: *Bacillus subtilis, Lactobacillus farciminus, Lactobacillus rhamnosus* und Kombinationen davon.

2. Verfahren zur Verbesserung der Stickstoffretention oder zur Verbesserung der Futterverwertung (FCR, feed conversion ratio) oder zur Verbesserung der Gewichtszunahme bei einem Individuum oder zur Verbesserung der Futtereffizienz bei einem Individuum oder zur Verringerung der Nährstoffausscheidung im Mist, welches Verfahren Verabreichen, an ein Individuum, eines direkt verfütterten Mikroorganismus (DFM, direct fed microbial) in Kombination mit einer von *Citrobacter-Spez.* ableitbaren Phytase umfasst, wobei die Phytase ein Polypeptid umfasst, das eine Aminosäuresequenz umfasst, die zumindest 99,1 % Identität mit Aminosäuren 23-433 von SEQ ID Nr. 1 oder 2 aufweist, wobei der direkt verfütterte Mikroorganismus ein Bakterium aus einer oder mehreren der folgenden Spezies umfasst: *Bacillus subtilis, Lactobacillus farciminus*, *Lactobacillus rhamnosus* und Kombinationen davon, und wobei das Individuum Geflügel ist.

3. Verwendung eines direkt verfütterten Mikroorganismus (DFM, direct fed microbial) in Kombination mit einer von *Citrobacter-Spez.* ableitbaren Phytase zur Verbesserung der Stickstoffretention oder zur Verbesserung der Futterverwertung (FCR, feed conversion ratio) oder zur Verbesserung der Gewichtszunahme bei einem Individuum oder zur Verbesserung der Futtereffizienz bei einem Individuum oder zur Verringerung der Nährstoffausscheidung im Mist, wobei die Phytase ein Polypeptid umfasst, das eine Aminosäuresequenz umfasst, die zumindest 99,1% Identität mit Aminosäuren 23-433 von SEQ ID Nr. 1 oder 2 aufweist, wobei der direkt verfütterte Mikroorganismus ein Bakterium aus einer oder mehreren der folgenden Spezies umfasst: *Bacillus subtilis, Lactobacillus farciminus*, *Lactobacillus rhamnosus* und Kombinationen davon, und wobei das Individuum Geflügel ist.

4. Kit, umfassend einen direkt verfütterten Mikroorganismus (DFM, direct fed microbial), eine von *Citrobacter-Spez.* ableitbare Phytase, und Anweisungen zur Verabreichung, wobei die Phytase ein Polypeptid umfasst, das eine Aminosäuresequenz umfasst, die zumindest 99,1% Identität mit Aminosäuren 23-433 von SEQ ID Nr. 1 oder 2 aufweist, wobei der direkt verfütterte Mikroorganismus ein Bakterium aus einer oder mehreren der folgenden Spezies umfasst: *Bacillus subtilis, Lactobacillus farciminus*, *Lactobacillus rhamnosus* und Kombinationen davon.

5. Futterzusatzzusammensetzung nach Anspruch 1 oder Verfahren nach Anspruch 2 oder Verwendung nach Anspruch 3 oder Kit nach Anspruch 4, wobei die Phytase ableitbar ist von einem *Citrobacter*-Bakterium*,* ausgewählt aus der Gruppe, bestehend aus: *Citrobacter braakii, Citrobacter freundii*, *Citrobacter amalonaticus*, *Citrobacter gillenii, Citrobacter intermedius*, *Citrobacter koseri, Citrobacter marrliniae, Citrobacter rodentium*, *Citrobacter sedlakii, Citrobacter werkmanii* und *Citrobacter yoarngae,* wobei bevorzugt die Phytase von *Citrobacter braakii* abgeleitet ist.

6. Futterzusatzzusammensetzung nach einem der Ansprüche 1 oder 5 oder Verfahren nach den Ansprüchen 2 oder 5 oder Verwendung nach Anspruch 3 oder 5 oder Kit nach den Ansprüchen 4-5, wobei die Phytase von *Citrobacter braakii* ATCC 51113 abgeleitet ist.

7. Futterzusatzzusammensetzung nach Anspruch 1 oder Verfahren nach Anspruch 2 oder 5-6 oder Verwendung nach den Ansprüchen 3 oder 5-6 oder Kit nach den Ansprüchen 4-6, wobei die Phytase eine 6-Phytase (E.C. 3.1.3.26) ist.

8. Futterzusatzzusammensetzung nach Anspruch 1 oder Verfahren nach den Ansprüchen 2 oder 5-7 oder Verwendung nach den Ansprüchen 3 oder 5-7 oder Kit nach den Ansprüchen 4-7, wobei die Phytase zumindest eine Alteration und nicht mehr als 4 Alterationen gegenüber SEQ ID Nr. 1 oder SEQ ID Nr. 2 umfasst, wobei zumindest eine der ein bis vier Alterationen aus den folgenden ausgewählt ist: 4P, 46E, 107G, 111P, 119K, 162C, 223E, 241Q, 273L, 276K, 379K, 385D, 91C/46C, 52C/99C, 31C/176C, 31C/177C, 59C/100C, 141C/199C, 162C/247C, 111P/241Q, 31C, 119K, 202N, 286Q und 362K,R.

9. Futterzusatzzusammensetzung, Verfahren, Verwendung oder Kit nach Anspruch 8, wobei die Phytase zumindest eine Alteration und nicht mehr als 4 Alterationen gegenüber SEQ ID Nr. 1 oder SEQ ID Nr. 2 umfasst, wobei zumindest eine der ein bis vier Alterationen aus den folgenden ausgewählt ist: 91C/46C, 52C/99C, 31C/176C, 31C/177C, 59C/100C, 141C/199C, 162C/247C, 111P/241Q.

10. Futterzusatzzusammensetzung nach einem der Ansprüche 1 oder 5-9 oder Verfahren nach den Ansprüchen 2 oder 5-9 oder Verwendung nach den Ansprüchen 3 oder 5-9 oder Kit nach den Ansprüchen 4-9, wobei die Phytase ein pH-Optimum im Bereich von 3-4,5 aufweist.

11. Futterzusatzzusammensetzung, Verfahren, Verwendung oder Kit nach Anspruch 10, wobei die Phytase ein pH-Optimum zwischen 3 und 3,5 aufweist.

12. Futterzusatzzusammensetzung nach einem der Ansprüche 1 oder 5-11 oder Verfahren nach den Ansprüchen 2 oder 5-11 oder Verwendung nach den Ansprüchen 3 oder 5-11 oder Kit nach den Ansprüchen 4-11, wobei der direkt verfütterte Mikroorganismus ein direkt verfütterter Antipathogen-Mikroorganismus ist.

13. Futterzusatzzusammensetzung nach einem der Ansprüche 1 oder 5-12 oder Verfahren nach den Ansprüchen 2 oder 5-12 oder Verwendung nach den Ansprüchen 3 oder 5-12 oder Kit nach den Ansprüchen 4-12, wobei der direkt verfütterte Mikroorganismus ein lebensfähiges Bakterium ist.

14. Futterzusatzzusammensetzung nach einem der Ansprüche 1 oder 8-13 oder Verfahren nach den Ansprüchen 2 oder 5-13 oder Verwendung nach den Ansprüchen 4 oder 5-13 oder Kit nach den Ansprüchen 4-13, wobei der direkt verfütterte Mikroorganismus einer oder mehrere der folgenden ist: *Bacillus*-*subtilis*-Stämme 3A-P4 (PTA-6506); 15A-P4 (PTA-6507); 22C-P1 (PTA-6508); 2084 (NRRL B-500130); LSSA01 (NRRL-B-50104); BS27 (NRRL B-50105); BS 18 (NRRL B-50633); und BS 278 (NRRL B-50634).

15. Futterzusatzzusammensetzung nach einem der Ansprüche 1 oder 5-14 oder Verfahren nach den Ansprüchen 2 oder 5-14 oder Verwendung nach den Ansprüchen 3 oder 5-14 oder Kit nach den Ansprüchen 4-14, wobei der direkt verfütterte Mikroorganismus in Form einer Endospore ist.

16. Futterzusatzzusammensetzung nach einem der Ansprüche 1 oder 5-15, wobei die Phytase in einer Dosierung zwischen 200 FTU/g Futterzusatzzusammensetzung und 10000 FTU/g Futterzusatzzusammensetzung vorliegt.

17. Futterzusatzzusammensetzung nach einem der Ansprüche 1 oder 5-16, wobei der DFM in einer Dosierung zwischen 3,75x10⁷ CFU/g Futterzusatzzusammensetzung und 1x10¹¹ CFU/g Futterzusatzzusammensetzung vorliegt.

18. Verfahren nach Anspruch 2, umfassend Verabreichen der Futterzusatzzusammensetzung nach einem der Ansprüche 1 oder 5-17.

19. Verwendung nach Anspruch 3, wobei die Futterzusatzzusammensetzung nach einem der Ansprüche 1 oder 5-17 verwendet wird.

20. Kit nach Anspruch 4, wobei das Kit die Futterzusatzzusammensetzung nach einem der Ansprüche 1 oder 5-17 umfasst.

21. Kit nach einem der Ansprüche 4-15 oder 20, das zumindest ein Vitamin und/oder zumindest ein Mineral umfasst.

22. Verfahren zur Herstellung einer Futterzusatzzusammensetzung, umfassend Vermischen eines direkt verfütterten Mikroorganismus (DFM) mit einer von *Citrobacter-* Spez. ableitbaren Phytase, wobei die Phytase ein Polypeptid umfasst, das eine Aminosäuresequenz umfasst, die zumindest 99,1% Identität mit Aminosäuren 23-433 von SEQ ID Nr. 1 oder 2 aufweist, und wobei der direkt verfütterte Mikroorganismus ein Bakterium aus einer oder mehreren der folgenden Spezies umfasst: *Bacillus subtilis, Lactobacillus farciminus, Lactobacillus rhamnosus* und Kombinationen davon.

23. Verfahren nach Anspruch 22, das weiterhin Verpacken umfasst.

24. Futter, umfassend eine Futterzusatzzusammensetzung nach einem der Ansprüche 1 oder 5-17.

25. Futter nach Anspruch 24, wobei die Phytase in einer Dosierung zwischen 400 FTU/kg Futter und 1000 FTU/kg Futter vorliegt.

26. Futter nach einem der Ansprüche 24-25, wobei der DFM in einer Dosierung zwischen 7,5x10⁴ CFU/kg Futter und 1x10⁷ CFU/kg Futter vorliegt.

27. Verfahren zur Herstellung eines Futtermittels, umfassend Vermischen einer Futterkomponente mit einer Futterzusatzzusammensetzung nach einem der Ansprüche 1 oder 5-17.

28. Vormischung, umfassend eine Futterzusatzzusammensetzung, umfassend einen direkt verfütterten Mikroorganismus in Kombination mit einer *Citrobacter*-Phytase*,* und zumindest ein Mineral und/oder zumindest ein Vitamin, wobei die Phytase ein Polypeptid umfasst, das eine Aminosäuresequenz umfasst, die zumindest 99,1% Identität mit Aminosäuren 23-433 von SEQ ID Nr. 1 oder 2 aufweist, und wobei der direkt verfütterte Mikroorganismus ein Bakterium aus einem oder mehreren der folgenden Spezies umfasst: *Bacillus subtilis, Lactobacillus farciminus*, *Lactobacillus rhamnosus* und Kombinationen davon.

29. Vormischung, umfassend eine Futterzusatzzusammensetzung nach einem der Ansprüche 1 oder 5-17 in Kombination mit zumindest einem Mineral und/oder zumindest einem Vitamin.

30. Futterzusatzzusammensetzung nach einem der Ansprüche 1 oder 5-17 zur Verwendung bei Verhütung und/oder Behandlung nekrotisierender Enteritis und/oder Kokzidiose bei Geflügel.

## Revendications

1. Composition d'additif alimentaire comprenant un produit microbien administré directement (DFM) en combinaison avec une phytase pouvant être dérivée de *Citrobacter* spp, où la phytase comprend un polypeptide comprenant une séquence d'acides aminés qui a au moins 99,1 % d'identité avec les acides aminés 23-433 de la SEQ ID NO: 1 ou 2, et où le produit microbien administré directement comprend une bactérie de l'une ou de plusieurs des espèces suivantes: *Bacillus subtilis*, *Lactobacillus farciminus, Lactobacillus rhamnosus* et des combinaisons de celles-ci.

2. Procédé destiné à améliorer la rétention d'azote ou à améliorer le rapport de conversion alimentaire (FCR) ou à améliorer le gain de poids chez un sujet ou à améliorer l'efficacité alimentaire chez un sujet ou à réduire l'excrétion de nutriments dans les excréments, lequel procédé comprend administrer à un sujet un produit microbien administré directement (DFM) en combinaison avec une phytase pouvant être dérivée de *Citrobacter* spp, où la phytase comprend un polypeptide comprenant une séquence d'acides aminés qui a au moins 99,1 % d'identité avec les acides aminés 23-433 de la SEQ ID NO: 1 ou 2, et où le produit microbien administré directement comprend une bactérie de l'une ou de plusieurs des espèces suivantes: *Bacillus subtilis*, *Lactobacillus farciminus*, *Lactobacillus rhamnosus* et des combinaisons de celles-ci, et où le sujet est la volaille.

3. Utilisation d'un produit microbien administré directement (DFM) en combinaison avec une phytase pouvant être dérivée de *Citrobacter* spp, pour améliorer la rétention d'azote ou pour améliorer le rapport de conversion alimentaire (FCR) ou pour améliorer le gain de poids chez un sujet ou pour améliorer l'efficacité alimentaire chez un sujet ou pour réduire l'excrétion de nutriments dans les excréments, où la phytase comprend un polypeptide comprenant une séquence d'acides aminés qui a au moins 99,1 % d'identité avec les acides aminés 23-433 de la SEQ ID NO: 1 ou 2, et où le produit microbien administré directement comprend une bactérie de l'une ou de plusieurs des espèces suivantes: *Bacillus subtilis*, *Lactobacillus farciminus*, *Lactobacillus rhamnosus* et des combinaisons de celles-ci, et où le sujet est la volaille.

4. Kit comprenant un produit microbien administré directement (DFM), une phytase pouvant être dérivée de *Citrobacter* spp., et des instructions pour l'administration, où la phytase comprend un polypeptide comprenant une séquence d'acides aminés qui a au moins 99,1 % d'identité avec les acides aminés 23-433 de la SEQ ID NO: 1 ou 2, où le produit microbien administré directement comprend une bactérie de l'une ou de plusieurs des espèces suivantes: *Bacillus subtils, Lactobacillus farciminus*, *Lactobacillus rhamnosus* et des combinaisons de celles-ci.

5. Composition d'additif alimentaire selon la revendication 1 ou procédé selon la revendication 2 ou utilisation selon la revendication 3 ou kit selon la revendication 4, où la phytase peut être dérivée d'une bactérie *Citrobacter* sélectionnée parmi le groupe consistant en: *Citrobacter braakii, Citrobacter freundii, Citrobacter amalonaticus*, *Citrobacter gillenii, Citrobacter intermedius, Citrobacter koseri, Citrobacter marrliniae, Citrobacter rodentium*, *Citrobacter sedlakii, Citrobacter werkmanii* et *Citrobacter youngae*, où la phytase est dérivée de préférence de *Citrobacter braakii.*

6. Composition d'additif alimentaire selon l'une quelconque des revendications 1 ou 5 ou procédé selon l'une des revendications 2 ou 5 ou utilisation selon la revendication 3 ou 5 ou kit selon les revendications 4-5, où la phytase est dérivée de *Citrobacter braakii* ATCC 51113.

7. Composition d'additif alimentaire selon la revendication 1 ou procédé selon la revendication 2 ou 5-6 ou utilisation selon les revendications 3 ou 5-6 ou kit selon les revendications 4-6, où la phytase est une 6-phytase (E.C. 3.1.3.26).

8. Composition d'additif alimentaire selon la revendication 1 ou procédé selon les revendications 2 ou 5-7 ou utilisation selon les revendications 3 ou 5-7 ou kit selon les revendications 4-7, où la phytase comprend au moins une modification et pas plus de 4 modifications telle que comparée à la SEQ ID NO:1 ou à la SEQ ID NO: 2, où au moins l'une desdites une à quatre modifications est sélectionnée parmi les éléments suivants: 4P, 46E, 107G, 111P, 119K, 162C, 223E, 241Q, 273L, 276K, 379K, 385D, 91C/46C, 52C/99C, 31C/176C, 31C/177C, 59C/100C, 141C/199C, 162C/247C, 111P/241Q, 31C, 119K, 202N, 286Q et 362K,R.

9. Composition d'additif alimentaire, procédé, utilisation ou kit selon la revendication 8, où la phytase comprend au moins une modification et pas plus de 4 modifications telle que comparée à la SEQ ID NO:1 ou à la SEQ ID NO: 2, où au moins l'une desdites une à quatre modifications est sélectionnée parmi les éléments suivants: 91C/46C, 52C/99C, 31C/176C, 31C/177C, 59C/100C, 141C/199C, 162C/247C, 111P/241Q.

10. Composition d'additif alimentaire selon l'une quelconque des revendications 1 ou 5-9 ou procédé selon les revendications 2 ou 5-9 ou utilisation selon les revendications 3 ou 5-9 ou kit selon les revendications 4-9, où la phytase a un pH optimum dans la plage de 3-4,5.

11. Composition d'additif alimentaire, procédé, utilisation ou kit selon la revendication 10, où la phytase a un pH optimum d'entre 3 et 3,5.

12. Composition d'additif alimentaire selon l'une quelconque des revendications 1 ou 5-11 ou procédé selon les revendications 2 ou 5-11 ou utilisation selon le revendications 3 ou 5-11 ou kit selon les revendications 4-11, où le produit microbien administré directement est un produit microbien antipathogène administré directement.

13. Composition d'additif alimentaire selon l'une quelconque des revendications 1 ou 5-12 ou procédé selon les revendications 2 ou 5-12 ou utilisation selon les revendications 3 ou 5-12 ou kit selon les revendications 4-12, où le produit microbien administré directement est une bactérie viable.

14. Composition d'additif alimentaire selon l'une quelconque des revendications 1 ou 8-13 ou procédé selon les revendications 2 ou 5-13 ou utilisation selon les revendications 4 ou 5-13 ou kit selon les revendications 4-13, où le produit microbien administré directement est l'un ou plusieurs des suivants: souches de *Bacillus subtilis* 3A-P4 (PTA-6506); 15A-P4 (PTA-6507); 22C-P1 (PTA-6508); 2084 (NRRL B-500130); LSSA01 (NRRL B-50104); BS27 (NRRL B-50105); BS 18 (NRRL B-50633); et BS 278 (NRRL B-50634).

15. Composition d'additif alimentaire selon l'une quelconque des revendications 1 ou 5-14 ou procédé selon les revendications 2 ou 5-14 ou utilisation selon les revendications 3 ou 5-14 ou kit selon les revendications 4-14, où le produit microbien administré directement est sous la forme d'une endospore.

16. Composition d'additif alimentaire selon l'une quelconque des revendications 1 ou 5-15, où la phytase est présente à un dosage d'entre 200 FTU/g de composition d'additif alimentaire et 10000 FTU/g de composition d'additif alimentaire.

17. Composition d'additif alimentaire selon l'une quelconque des revendications 1 ou 5-16, où le DFM est présent à un dosage d'entre 3,75x10⁷ CFU/g de composition d'additif alimentaire et 1x10¹¹ CFU/g de composition d'additif alimentaire.

18. Procédé selon la revendication 2, comprenant le fait d'administrer la composition d'additif alimentaire selon l'une quelconque des revendications 1 ou 5-17.

19. Utilisation selon la revendication 3, dans laquelle la composition d'additif alimentaire selon l'une quelconque des revendications 1 ou 5-17 est utilisée.

20. Kit selon la revendication 4, où ledit kit comprend la composition d'additif alimentaire selon l'une quelconque des revendications 1 ou 5-17.

21. Kit selon l'une quelconque des revendications 4-15 ou 20, lequel comprend au moins une vitamine et/ou au moins un minéral.

22. Procédé de préparation d'une composition d'additif alimentaire, comprenant mélanger un produit microbien administré directement (DFM) avec une phytase pouvant être dérivée de *Citrobacter* spp., où la phytase comprend un polypeptide comprenant une séquence d'acides aminés qui a au moins 99,1 % d'identité avec les acides aminés 23-433 de la SEQ ID NO: 1 ou 2, et où le produit microbien administré directement comprend une bactérie de l'une ou de plusieurs des espèces suivantes: *Bacillus subtils, Lactobacillus farciminus, Lactobacillus rhamnosus* et des combinaisons de celles-ci.

23. Procédé selon la revendication 22, qui comprend en outre un conditionnement.

24. Aliment pour animaux comprenant une composition d'additif alimentaire selon l'une quelconque des revendications 1 ou 5-17.

25. Aliment pour animaux selon la revendication 24, dans lequel la phytase est présente à un dosage d'entre 400 FTU/kg d'aliment pour animaux à 1000 FTU/kg d'aliment pour animaux.

26. Aliment pour animaux selon l'une quelconque des revendications 24-25, dans lequel le DFM est présent à un dosage d'entre 7,5x10⁴ CFU/kg d'aliment pour animaux à 1x10⁷ CFU/kg d'aliment pour animaux.

27. Procédé de préparation d'un aliment pour animaux comprenant mélanger un composant alimentaire avec une composition d'additif alimentaire selon l'une quelconque des revendications 1 ou 5-17.

28. Prémix comprenant une composition d'additif alimentaire comprenant un produit microbien administré directement en combinaison avec une phytase de *Citrobacter,* et au moins un minéral et/ou au moins une vitamine, où la phytase comprend un polypeptide comprenant une séquence d'acides aminés qui a au moins 99,1 % d'identité avec les acides aminés 23-433 de la SEQ ID NO: 1 ou 2, et où le produit microbien administré directement comprend une bactérie de l'une ou de plusieurs des espèces suivantes: *Bacillus subtilis*, *Lactobacillus farciminus*, *Lactobacillus rhamnosus* et des combinaisons de celles-ci.

29. Prémix comprenant une composition d'additif alimentaire selon l'une quelconque des revendications 1 ou 5-17 en combinaison avec au moins un minéral et/ou au moins une vitamine.

30. Composition d'additif alimentaire selon l'une quelconque des revendications 1 ou 5-17, à utiliser dans la prévention et/ou le traitement de l'entérite nécrotique et/ou de la coccidiose chez la volaille.
